(19)

Europäisches
Patentamt
European
Patent Office
Office européen
des brevets

(11) **EP 4 127 246 B1**

(12) **EUROPEAN PATENT SPECIFICATION**

(45) Date of publication and mention
of the grant of the patent:
**29.04.2026 Bulletin 2026/18**

(21) Application number: **21714963.2**

(22) Date of filing: **23.03.2021**

(51) International Patent Classification (IPC):
***C12Q 1/70*** *(2006.01)*

(52) Cooperative Patent Classification (CPC):
**C12Q 1/701;** C12Q 2600/118; Y02A 50/30

(86) International application number:
**PCT/IB2021/052400**

(87) International publication number:
**WO 2021/191795 (30.09.2021 Gazette 2021/39)**

(54) **CORONAVIRUS DETECTION**

CORONAVIRUS DETEKTION

DETECTION DE CORONAVIRUS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.03.2020 GB 202004203
06.04.2020 GB 202005062**

(43) Date of publication of application:
**08.02.2023 Bulletin 2023/06**

(73) Proprietor: **Diagnostics for the Real World, Ltd
San Jose, CA 95138 (US)**

(72) Inventors:
• **ASSENNATO, Sonny Michael
Little Chesterford Essex CB10 1XL (GB)**
• **RITCHIE, Allyson Victoria
Little Chesterford Essex CB10 1XL (GB)**

(74) Representative: **Avidity IP
Future Business Centre Cambridge Campus
Kings Hedges Road
Cambridge CB4 2HY (GB)**

(56) References cited:
**CN-A- 111 254 228**

• EL-THOLOTH MOHAMED ET AL: "A Single and Two-Stage, Closed-Tube, Molecular Test for the 2019 Novel Coronavirus (COVID-19) at Home, Clinic, and Points of Entry", 19 February 2020 (2020-02-19), XP093328503, Retrieved from the Internet <URL:https://chemrxiv.org/engage/ chemrxiv/article-details/ 60c74829f96a0042bd28702a> DOI: 10.26434/ chemrxiv.11860137.v1
• LAURA ELAINE LAMB ET AL: "The Lancet Rapid Detection of Novel Coronavirus (COVID-19) by Reverse Transcription-Loop- Mediated Isothermal Amplification –Manuscript Draft", THE LANCET, 24 February 2020 (2020-02-24), pages 1 - 19, XP055733471, Retrieved from the Internet <URL:https://www.medrxiv.org/content/ 10.1101/2020.02.19.20025155v1> [retrieved on 20200923], DOI: 10.1101/2020.02.19.20025155
• YINHUA ZHANG ET AL: "Rapid Molecular Detection of SARS-CoV-2 (COVID-19) Virus RNA Using Colorimetric LAMP", MEDRXIV, 29 February 2020 (2020-02-29), XP055730127, Retrieved from the Internet <URL:https://www. medrxiv.org/content/10.1101/ 2020.02.26.20028373v1> [retrieved on 20210610], DOI: 10.1101/2020.02.26.20028373

EP 4 127 246 B1

(Cont. next page)

- **WEIHUA YANG ET AL: "Rapid Detection of SARS-CoV-2 Using Reverse transcription RT-LAMP method", MEDRXIV, 3 March 2020 (2020-03-03), XP055733463, Retrieved from the Internet <URL:https://www.medrxiv.org/content/10.1101/2020.03.02.20030130v2> [retrieved on 20210610], DOI: 10.1101/2020.03.02.20030130**
- **BREITBACH ANDRÉ: "Loop Mediated Isothermal Amplification (LAMP) -Lateral Flow Loop mediated isothermal Amplification and Lateral Flow Tips and Tricks for a successful Assay Devel opment", 11 March 2020 (2020-03-11), XP055772550, Retrieved from the Internet <URL:https://www.milenia-biotec.com/en/isothermal-amplification-lateral-flow/> [retrieved on 20210204]**

**EP 4 127 246 B1**

**Description**

This invention relates to methods for detecting nucleic acid of severe acute respiratory syndrome coronavirus 2 (SARS-CoV-2), responsible for coronavirus disease 2019 (COVID-19), particularly for point-of-care (POC) testing, and to kits, sets of primers, sets of oligonucleotides, and oligonucleotides, and their use in the methods. Primers and probes are also described.

The 2019-20 coronavirus pandemic is an ongoing pandemic of COVID-19 caused by SARS-CoV-2. First identified in Wuhan, Hubei, China, in December 2019, the outbreak was recognised as a pandemic by the World Health Organization (WHO) on 11 March 2020. As of 18 March 2020, more than 203,000 cases of COVID-19 have been reported in over 160 countries and territories, with major outbreaks in mainland China, Europe, Iran, and South Korea. More than 8,200 people have died and over 82,000 have recovered.

SARS-CoV-2 belongs to the broad family of viruses known as coronaviruses. Coronaviruses are named for the crown-like spikes on their surface. There are four main sub-groupings of coronaviruses, known as alpha, beta, gamma, and delta. The seven coronaviruses that can infect people are: 229E (alpha coronavirus); NL63 (alpha coronavirus); OC43 (beta coronavirus); HKU1 (beta coronavirus); MERS-CoV (beta coronavirus that causes Middle East Respiratory Syndrome, or MERS); SARS-CoV (beta coronavirus that causes severe acute respiratory syndrome, or SARS); and SARS-CoV-2.

Like other coronaviruses, SARS-CoV-2 has four structural proteins, known as the S (spike), E (envelope), M (membrane), and N (nucleocapsid) proteins. The N protein holds the RNA genome, and the S, E, and M proteins together create the viral envelope. The spike protein, which has been imaged at the atomic level using cryogenic electron microscopy, is the protein responsible for allowing the virus to attach to the membrane of a host cell.

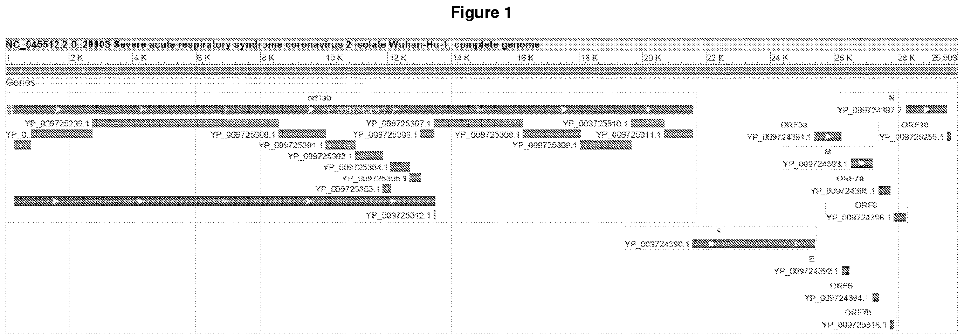

SARS-CoV-2 is a positive-sense single-stranded RNA (+ssRNA) virus. Like the SARS-related coronavirus strain implicated in the 2003 SARS outbreak, SARS-CoV-2 is a member of the subgenus Sarbecovirus (beta-CoV lineage B). Its RNA sequence is approximately 30,000 bases in length (see the SARS-CoV genome organisation shown in Figure 1).

Being able to test, identify and treat individuals infected with SARS-CoV-2 without delay is critical. Nucleic acid testing (NAT) is state of the art for diagnosis of infectious disease, but requires highly-trained staff and specialized facilities available only in sophisticated, centralized laboratories. Current NATs also require samples to be sent frozen or refrigerated; with turnaround times of 2-3 days for results leading to 'loss-to-follow-up,' a common problem in developing countries. Despite draconian efforts by central and local authorities, Wuhan experiences long delays in testing results, especially in hospitals without NAT testing capacity. This limitation is also seen in other settings such as cruise ships. China is also experiencing amplicon contamination, causing false positives, a common problem for NATs. China infrastructure is stretched and experiencing some of the same problems for diagnostics seen in developing countries. Lamb et al, The Lancet 2020, pages 1-19, describes reverse transcription loop-mediated isothermal amplification (RT-LAMP) detection of SARS-CoV-2 in SARS-CoV-2 oligonucleotide spiked patient samples, to develop a rapid screening diagnostic test.

There is clearly an urgent need for a decentralized, point-of-care (POC) diagnostic test for COVID-19 for use in the community.

We have designed primers and probes specific to SARS-CoV-2, and a test has been developed with a sensitivity of at least 10 copies/reaction for 2 specific regions of the SARS-CoV-2 genome.

Primers and probes specific to SARS-CoV-2 (COVID-19) genome have been designed in the ORF1 and nucleocapsid (N) genes. Alignment of all 7 human coronaviruses was used during primer and probe selection, ensuring high specificity.

According to the invention there is provided a method for determining whether a sample includes severe acute respiratory syndrome coronavirus 2 (SARS-CoV-2) nucleic acid, which comprises amplifying nucleic acid of the sample, or amplifying nucleic acid derived from nucleic acid of the sample, by an isothermal amplification reaction using a forward nucleic acid amplification primer and a reverse nucleic acid amplification primer, wherein each nucleic acid amplification primer hybridises specifically to nucleic acid sequence that is conserved in SARS-CoV-2 nucleic acid, but not in other human coronavirus nucleic acid, or the complement thereof, wherein the nucleic acid sequence that is conserved in SARS-CoV-2 nucleic acid is nucleic acid sequence that is conserved in the ORF1ab gene or the nucleocapsid gene of SARS-CoV-2, wherein:

the forward nucleic acid primer hybridizes specifically to nucleic acid sequence that is conserved in the ORF1ab gene of SARS-CoV-2, or the complement thereof, wherein the forward nucleic acid primer comprises a nucleic acid sequence of CTGTTGGTCAACAAGACGGCA (SEQ ID NO:1), or the complement thereof, or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, or the complement thereof, and the reverse nucleic acid primer hybridizes specifically to nucleic acid sequence that is conserved in the ORF1ab gene of SARS-CoV-2, or the complement thereof, wherein the reverse nucleic acid primer comprises a nucleic acid sequence CAATAGTCTGAACAACTGGTGT (SEQ ID NO:5), or the complement thereof, or a nucleic acid sequence that has at

3

least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, or the complement thereof; and/or

the forward nucleic acid primer hybridizes specifically to nucleic acid sequence that is conserved in the nucleocapsid gene of SARS-CoV-2, or the complement thereof, wherein the forward nucleic acid primer comprises a nucleic acid sequence of: TAGTTGATGACCCGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14, or the complement thereof and the reverse nucleic acid primer hybridizes specifically to nucleic acid sequence that is conserved in the nucleocapsid gene of SARS-CoV-2, or the complement thereof, wherein the reverse nucleic acid primer comprises a nucleic acid sequence CAACACGAACGTCATGATAC (SEQ ID NO:16), or the complement thereof, or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:16, or the complement thereof.

Optionally the other human coronavirus nucleic acid is human coronavirus 229E, SARS, HKU1, MERS, OC43, and NL63 nucleic acid.

Optionally the nucleic acid sequence that is conserved in SARS-CoV-2 nucleic acid, but not in other human coronavirus nucleic acid, comprises a contiguous nucleic acid sequence that is at least 19 nucleotides long.

Conserved sequences may be identified by homology search, using tools such as BLAST, HMMER and Infernal. Homology search tools may take an individual nucleic acid sequence as input, or use statistical models generated from multiple sequence alignments of known related sequences. Statistical models such as profile-HMMs, and RNA covariance models which also incorporate structural information, can be helpful when searching for more distantly related sequences. Input sequences are then aligned against a database of sequences from related individuals or other species. The resulting alignments are then scored based on the number of matching bases, and the number of gaps or deletions generated by the alignment. Acceptable conservative substitutions may be identified using substitution matrices such as PAM and BLOSUM. Highly scoring alignments are assumed to be from homologous sequences. The conservation of a sequence may then be inferred by detection of highly similar homologs over a broad phylogenetic range.

Optionally conserved SARS-CoV-2 nucleic acid is nucleic acid comprising nucleic acid sequence that includes up to 2 mismatches per 20 nucleotides compared with a reference SARS-CoV-2 nucleic acid sequence.

Optionally conserved SARS-CoV-2 nucleic acid is nucleic acid comprising nucleic acid sequence that includes up to 1 mismatch per 20 nucleotides compared to a reference SARS-CoV-2 nucleic acid sequence.

Optionally conserved SARS-CoV-2 nucleic acid is nucleic acid comprising nucleic acid sequence that is identical to a reference SARS-CoV-2 nucleic acid sequence.

Multiple sequence alignments can be used to visualise conserved sequences. The CLUSTAL format includes a plain-text key to annotate conserved columns of the alignment, denoting conserved sequence (*), conservative mutations (:), semi-conservative mutations (.), and non-conservative mutations ( ). Software such as MacVector can be used to perform multiple sequence alignments.

Optionally a nucleic acid amplification primer hybridises specifically to nucleic acid sequence that is conserved in SARS-CoV-2 nucleic acid, or the complement thereof, if it hybridises under stringent conditions to the conserved SARS-CoV-2 nucleic acid sequence, or the complement thereof, but not other human coronavirus nucleic acid, or the complement thereof.

The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C. below $T_m$, and high stringency conditions are when the temperature is 10°C below $T_m$. The $T_m$ is the temperature under defined ionic strength and pH, at which 50% of the target sequence hybridises to a perfectly matched primer or probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the $T_m$ decreases about 1°C per % base mismatch. The $T_m$ may be calculated using the following equations, depending on the types of hybrids:

1) DNA-DNA hybrids (Meinkoth and Wahl, Anal. Biochem., 138: 267-284, 1984):

$$T_m=81.5° \text{ C.}+16.6 \times \log_{10}[Na^+]^a+0.41 \times \%[G/C^b]-500 \times [L^c]^{-1}-0.61 \times \% \text{ formamide;}$$

2) DNA-RNA or RNA-RNA hybrids:

$$T_m=79.8°C+18.5(\log_{10}[Na^+]^a)+0.58(\%G/C^b)+11.8(\%G/C^b)^2-820/L^c;$$

3) oligo-DNA or oligo-RNAs hybrids:

For <20 nucleotides: $T_m=2(I_n)$;

For 20-35 nucleotides: $T_m=22+1.46(I_n)$;

[a] or for other monovalent cation, but only accurate in the 0.01-0.4 M range.

[b] only accurate for % GC in the 30% to 75% range.

[c] L = length of duplex in base pairs.

[d] oligo, oligonucleotide; $1_n$, = effective length of primer=2x (no. of G/C)+(no. of NT).

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical stringent conditions (also referred to as high stringency hybridisation conditions) for DNA hybrids longer than 50 nucleotides encompass hybridisation at 65°C in 1xSSC or at 42°C in 1xSSC and 50% formamide, followed by washing at 65°C in 0.3xSSC. The length of the hybrid is the anticipated length for the hybridising nucleic acid. When nucleic acids of known sequence are hybridised, the hybrid length may be determined by aligning the sequences and identifying the conserved regions described herein. 1xSSC is 0.15M NaCl and 15 mM sodium citrate; the hybridisation solution and wash solutions may additionally include 5xDenhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Optionally the forward nucleic acid primer hybridizes specifically to nucleic acid sequence that is conserved in the ORF1ab gene of SARS-CoV-2, or the complement thereof.

Optionally the forward nucleic acid primer hybridizes specifically to nucleic acid sequence that is the complement of nucleic acid sequence that is conserved in the ORF1ab gene of SARS-CoV-2.

Optionally the forward nucleic acid primer comprises a nucleic acid sequence of: CTGTTGGTCAACAAGAC GGCA (SEQ ID NO:1), GTCAACAAACTGTTGGTCAA (SEQ ID NO:2), GTCAACAAACTGTTGGTCAACA (SEQ ID NO:3), CATTACAGGTGGTGTTGTTCAGTT (SEQ ID NO:4), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, 2, 3, or 4.

Optionally the reverse nucleic acid primer hybridizes specifically to nucleic acid sequence that is conserved in the ORF1ab gene of SARS-CoV-2, or the complement thereof.

Optionally the reverse nucleic acid primer hybridizes specifically to nucleic acid sequence that is conserved in the ORF1ab gene of SARS-CoV-2.

Optionally the reverse nucleic acid primer comprises a nucleic acid sequence: CAATAGTCTGAACAACTGG TGT (SEQ ID NO:5), CTGGTGTAAGTTCCATCTCT (SEQ ID NO:6), AGGTGACAATTTGTCCACCGAC (SEQ ID NO:7), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, 6, or 7.

Optionally the forward and reverse nucleic acid amplification primers comprise respective nucleic acid sequences according to any of the combinations of forward and reverse primer sequences shown in Table 1 below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

Table1

| Combination | Forward | Reverse |
|---|---|---|
| 1 | SEQ ID NO:1 (nCoV-ARP1.1F) | SEQ ID NO:5 (nCoV-ARP1.1R) |
| 2 | SEQ ID NO:2 (nCoV-ARP1.2F) | SEQ ID NO:5 (nCoV-ARP1.1R) |
| 3 | SEQ ID NO:3 (nCoV-ARP1.3F) | SEQ ID NO:5 (nCoV-ARP1.1R) |
| 4 | SEQ ID NO:2 (nCoV-ARP1.2F) | SEQ ID NO:6 (nCoV-ARP1.2R) |
| 5 | SEQ ID NO:3 (nCoV-ARP1.3F) | SEQ ID NO:6 (nCoV-ARP1.2R) |
| 6 | SEQ ID NO:4 (SA_nCoV_F2) | SEQ ID NO:7 (SA_nCoV_R2) |

Optionally the forward nucleic acid amplification primer comprises a nucleic acid sequence of SEQ ID NO:1, and the reverse nucleic acid amplification primer comprises a nucleic acid sequence of SEQ ID NO:5.

Optionally the forward nucleic acid primer hybridizes specifically to nucleic acid sequence that is conserved in the Nucleocapsid gene of SARS-CoV-2, or the complement thereof.

Optionally the forward nucleic acid primer hybridizes specifically to nucleic acid sequence that is the complement of nucleic acid sequence that is conserved in the Nucleocapsid gene of SARS-CoV-2.

Optionally the forward nucleic acid primer comprises a nucleic acid sequence of: TAGTTGATGACCCGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14.

Optionally the forward nucleic acid primer comprises a nucleic acid sequence of SEQ ID NO:14.

Optionally the reverse nucleic acid primer hybridizes specifically to nucleic acid sequence that is conserved in the Nucleocapsid gene of SARS-CoV-2, or the complement thereof.

Optionally the reverse nucleic acid primer hybridizes specifically to nucleic acid sequence that is conserved in the Nucleocapsid gene of SARS-CoV-2.

Optionally the reverse nucleic acid primer comprises a nucleic acid sequence: TGGGGTCCATTATCAGACAT (SEQ ID NO:15), CAACACGAACGTCATGATAC (SEQ ID NO:16), CATAGAACGAACAACGCAC (SEQ ID NO:17), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:15, 16, or 17.

Optionally the forward nucleic acid amplification primer comprises a nucleic acid sequence of SEQ ID NO:14, and the reverse nucleic acid amplification primer comprises a nucleic acid sequence of SEQ ID NO:16.

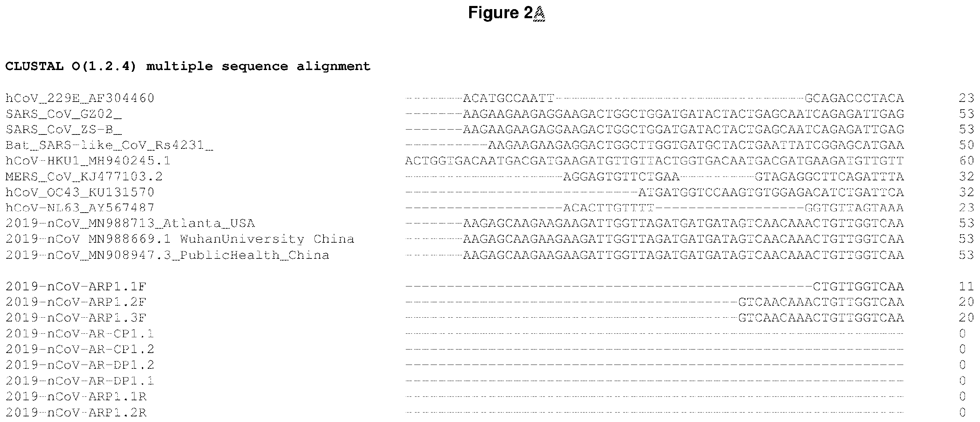

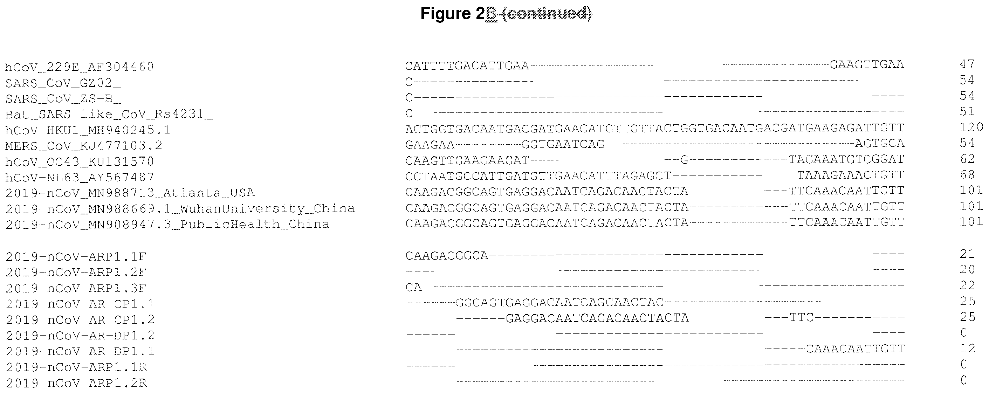

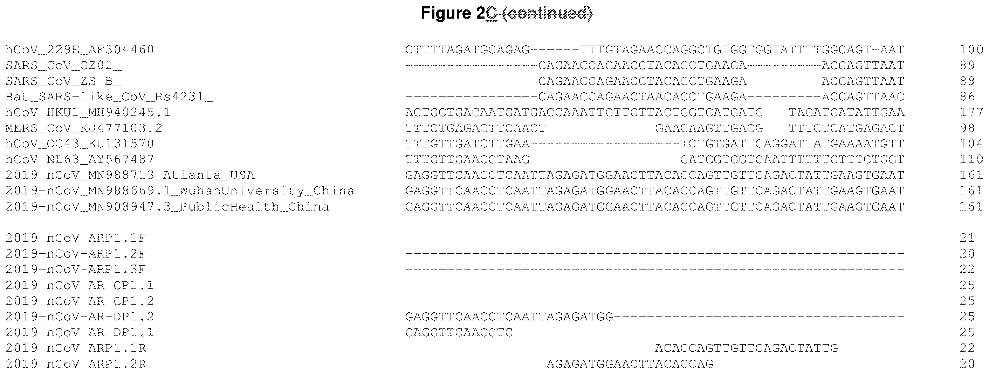

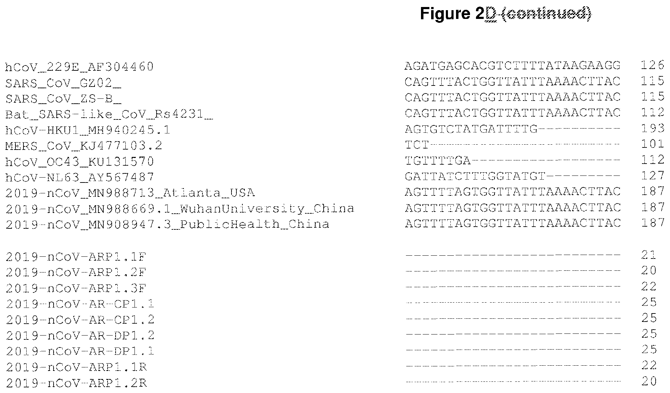

Figure 2A-D shows a multiple sequence alignment of the ORF1ab region for all 7 human coronaviruses (229E, SARS, HKU1, MERS, OC43, and NL63, and SARS-CoV-2 sequences of several different isolates), and a bat SARS-like coronavirus sequence. In the figure, SARS-CoV-2 is referred to by its previous provisional name "2019-nCoV" (2019 novel coronavirus). The locations in the SARS-CoV-2 ORF1ab gene sequence corresponding to the sequence of the forward primers of SEQ ID NOs:1-3 (2019-nCoV-ARP1.1F, 2019-nCoV-ARP1.2F, 2019-nCoV-ARP1.3F, respectively), and to the reverse complement of sequence of the reverse primers of SEQ ID NOs:5,6 (2019-nCoV-ARP1.1R, 2019-nCoV-ARP1.2R, respectively), are shown in Figure 2.

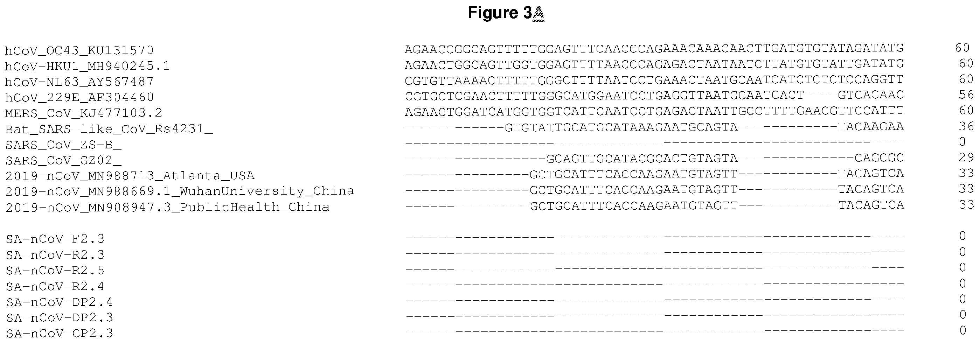

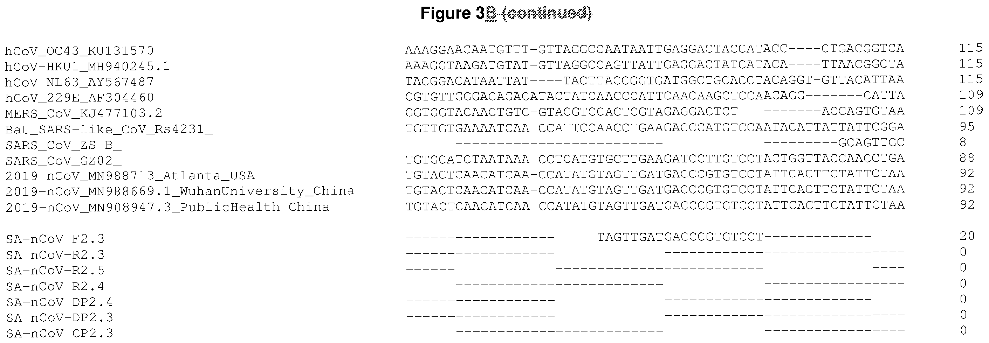

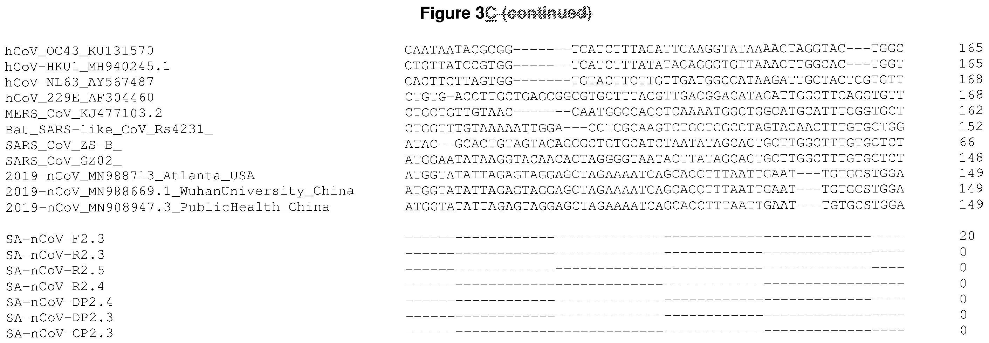

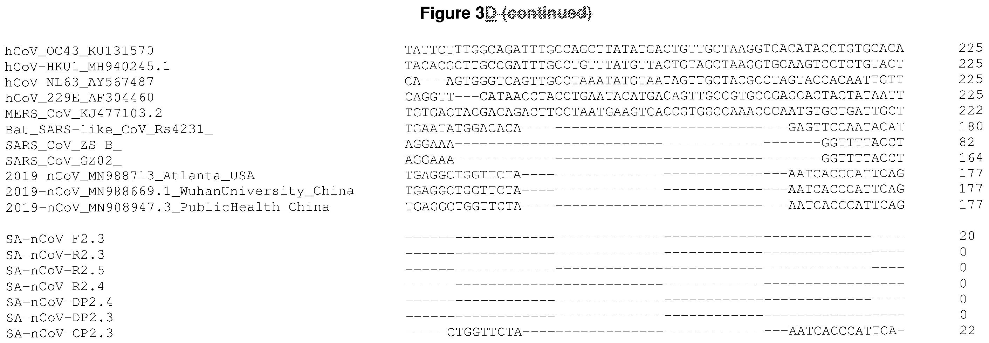

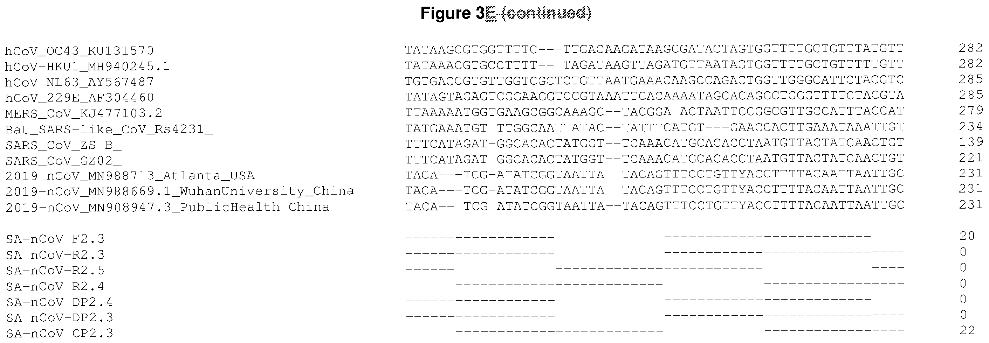

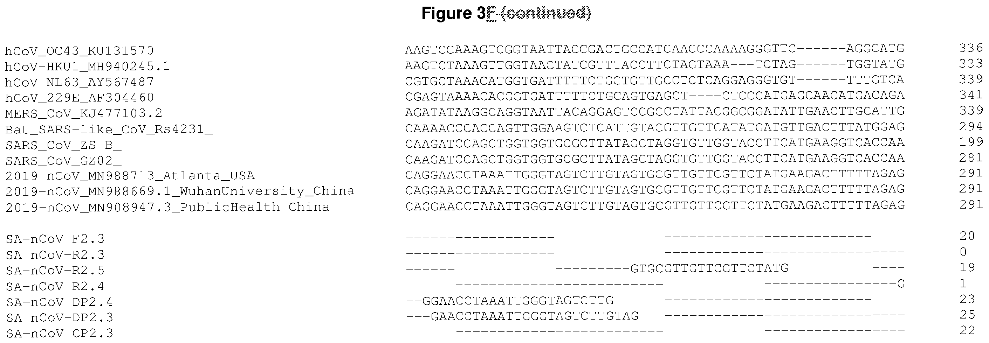

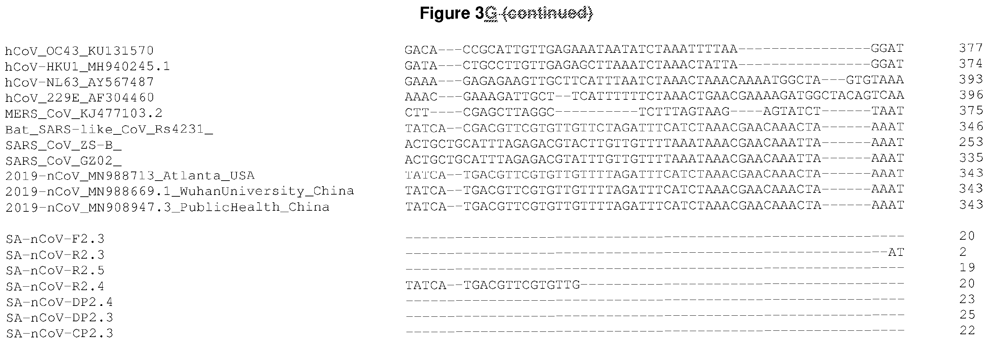

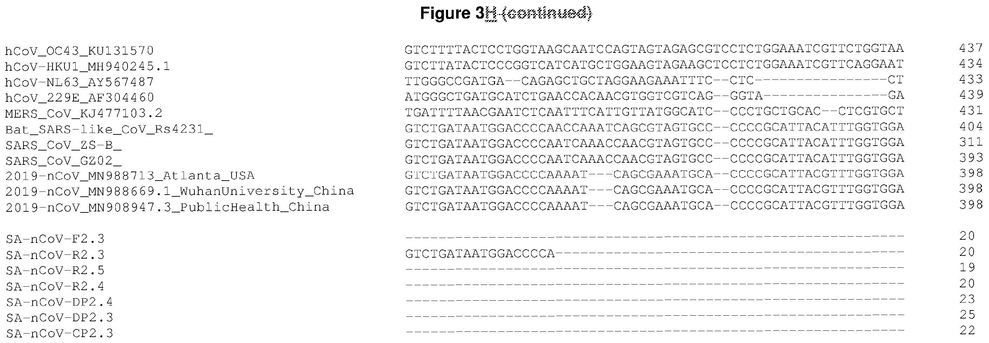

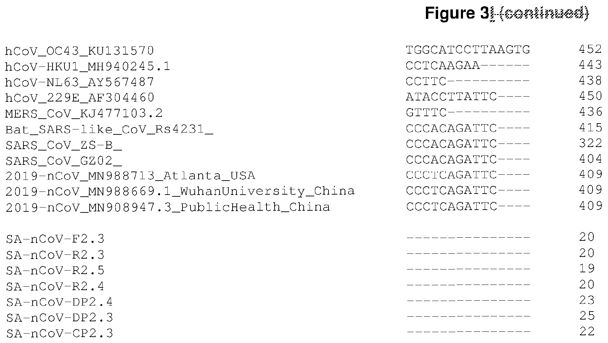

Figure 3A-I shows a multiple sequence alignment of the nucleocapsid region for all 7 human coronaviruses (229E, SARS, HKU1, MERS, OC43, and NL63, and SARS-CoV-2 sequences of several different isolates), and a bat SARS-like coronavirus sequence. In the figure, SARS-CoV-2 is referred to by its previous provisional name "2019-nCoV" (2019 novel coronavirus). The locations in the SARS-CoV-2 nucleocapsid gene sequence corresponding to the sequence of the forward primer of SEQ ID NO:14 (SA-nCoV-F2.3), and to the reverse complement of sequence of the reverse primers of SEQ ID NOs:15-17 (SA-nCoV-R2.3, SA-nCoV-R2.4, SA-nCoV-R2.5, respectively), are shown in Figure 3.

Nucleic acid may be derived from nucleic acid of the sample, for example by reverse transcribing SARS-CoV-2 nucleic acid of the sample, and amplifying a product of the reverse transcription by an isothermal nucleic acid amplification reaction using the forward and reverse nucleic acid amplification primers.

Optionally a method of the invention further comprises reverse transcribing SARS-CoV-2 RNA of the sample, and amplifying a product of the reverse transcription by an isothermal amplification reaction using the forward and reverse nucleic acid amplification primers.

Optionally the reverse nucleic acid primer further comprises a promoter sequence for a DNA-dependent RNA polymerase at its 5'-end, and reverse transcription is carried out using the reverse nucleic acid primer.

Any suitable method of isothermal nucleic acid amplification may be used in methods of the invention. Several suitable methods of isothermal nucleic acid amplification are known to the skilled person. Optionally the isothermal nucleic acid amplification is a transcription-based amplification. Such methods involve amplification of an RNA template using reverse transcriptase (RT), RNase H, and RNA polymerase activities, and include nucleic acid sequence-based amplification (NASBA), transcription-mediated amplification (TMA), and self-sustained sequence replication (3SR) (Chan and Fox, Rev. Med. Microbiol. 10: 185-196 (1999); Guatelli et al., Proc. Natl. Acad. Sci. 87: 1874-1878 (1990); Compton, Nature 350:91-92 (1991)). NASBA and 3SR use RT from Avian Myeloblastosis Virus (AMV) (which also has RNaseH activity), RNase H from *E.coli,* and T7 RNA polymerase. TMA uses Moloney Murine Leukemia Virus (MMLV) RT (which also has RNase H activity), and T7 RNA polymerase.

Isothermal amplification methods, such as transcription-based amplification methods, have several advantages over amplification using a Polymerase Chain Reaction (PCR). The reactions occur simultaneously in a single tube, and are carried out under isothermal conditions so a thermocycler is not required. The amplification reaction is faster than PCR ($1\times10^9$-fold amplification can be seen after five cycles, compared with $1\times10^6$-fold amplification after 20 cycles for PCR). DNA background does not interfere with transcription-based amplification, and so these methods are not affected by double stranded DNA contamination. The amplification product is single stranded and can be detected without any requirement for strand separation.

Optionally the reverse nucleic acid primer further comprises a promoter sequence for a DNA-dependent RNA polymerase at its 5'-end. Such a primer can be used for the reverse transcription and for a transcription-based isothermal amplification reaction, thereby minimising the number of primers required to carry out reverse transcription and isothermal nucleic acid amplification.

For example, the promoter sequence may be a T7 promoter sequence comprising: 5' taatacgactcactatag 3' (SEQ ID NO:23). T7 RNA polymerase starts transcription at the underlined G in the promoter sequence. The polymerase then transcribes using the opposite strand as a template from 5'->3'. The first base in the transcript will be a G. Other examples of T7 promoter sequence for inclusion at the 5'-end of the reverse nucleic acid primer include a nucleic acid sequence of:

- aattctaatacgactcactagggagaagg (SEQ ID NO:21) - for use with 2019-CoV-ARP1.1R (SEQ ID NO:5);
- aattctaatacgactcactaggggaaaag (SEQ ID NO:22) - for use with SA_nCoV_R2.4 (SEQ ID NO:16);
- aattctaatacgactcactaggggaaagga (SEQ ID NO:24) - for use with 2019-CoV-ARP1.2R (SEQ ID NO:6);
- aattctaatacgactcactaggggaaagg (SEQ ID NO:25) - for use with SA_nCoV_R2 (SEQ ID NO:7);
- aattctaatacgactcactaggggaaaga (SEQ ID NO:26) - for use with SA_nCoV_R2.3 (SEQ ID NO:15) or SA_nCoV_R2.3 R2.5 (SEQ ID NO:17)

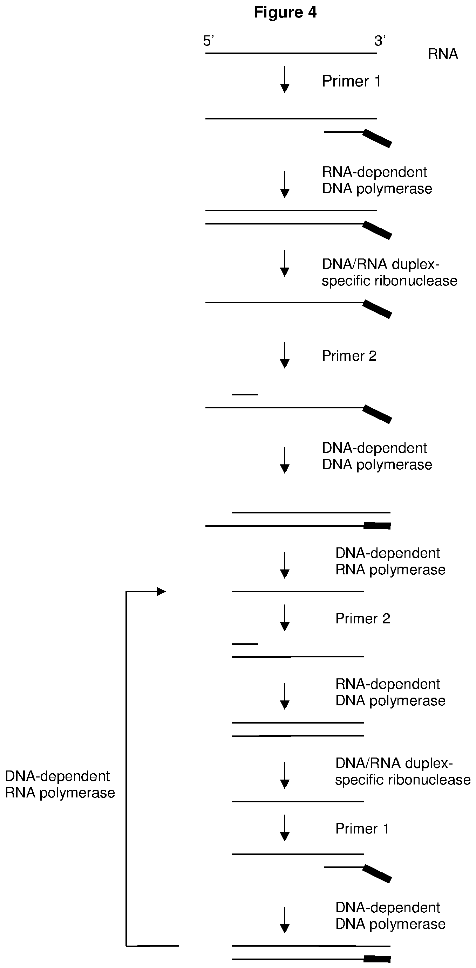

A transcription-based isothermal amplification reaction suitable for use in methods of the invention is described below, with reference to Figure 4.

An antisense Primer 1 comprises nucleic acid sequence complementary to a portion of a target RNA so that the primer can hybridise specifically to the target RNA (for example, SEQ ID NO:5, 2019-CoV-ARP1.1R, reverse primer), and a single stranded-version of a promoter sequence for a DNA-dependent RNA polymerase at its 5'-end (for example, a T7 promoter sequence comprising SEQ ID NO:21). Primer 1 is annealed to the RNA target. An RNA-dependent DNA polymerase extends Primer 1 to synthesise a complementary DNA (cDNA) copy of the RNA target. A DNA/RNA duplex-specific ribonuclease digests the RNA of the RNA-cDNA hybrid. A sense Primer 2 comprises nucleic acid sequence complementary to a portion of the cDNA. Primer 2 is annealed to the cDNA downstream of the part of the cDNA formed by Primer 1. Primer 2 is extended by a DNA-dependent DNA polymerase to produce a second DNA strand which extends through the DNA-dependent RNA polymerase promoter sequence at one end (thereby forming a double stranded promoter). This promoter is used by a DNA-dependent RNA polymerase to synthesise a large number of RNAs complementary to the original target sequence. These RNA products then function as templates for a cyclic phase of the reaction, but with the primer annealing steps reversed, i.e., Primer 2 followed by Primer 1.

In a variation of this method, Primer 2 may also include a single stranded version of a promoter sequence for the DNA-dependent RNA polymerase. This results in production of RNAs with the same sense as the original target sequence (as well as RNAs complementary to the original target sequence).

In some conventional isothermal transcription-based amplification reactions it is known to cleave the target RNA at the 5'-end before it serves as the template for cDNA synthesis. An enzyme with RNase H activity is used to cleave the RNA portion of an RNA-DNA hybrid formed by adding an oligonucleotide (a cleavage oligonucleotide) having a sequence complementary to the region overlapping and adjacent to the 5'-end of the target RNA. The cleavage oligonucleotide may have its 3'-terminal-OH appropriately modified to prevent extension reaction. Whilst in some embodiments of the invention a cleavage oligonucleotide could be used, it is preferred that a method of the invention is carried out in the absence of a cleavage oligonucleotide thereby simplifying the amplification reaction and the components required.

Isothermal nucleic acid amplification is advantageous because it can readily be used in resource-limited settings. Such methods do not require the use of thermal cyclers which may not be available in resource-limited settings. Examples of suitable methods are described in WO 2008/090340 and Lee et al., Journal of Infectious Diseases 2010;201(S1):S65-S71.

Examples of suitable reagents for carrying out reverse transcription of RNA, and for isothermal amplification of a product of the reverse transcription, are given in WO 2008/090340, and include, for example, the following enzyme activities: an RNA-dependent DNA polymerase, a DNA-dependent DNA polymerase, a DNA/RNA duplex-specific ribonuclease, and a DNA-dependent RNA polymerase.

It will be appreciated that in addition to the required enzyme activities, it will also be necessary to provide appropriate nucleotide triphosphates (for transcription-based amplifications, ribonucleotide triphosphates (rNTPs, i.e. rATP, rGTP, rCTP, and rUTP), and deoxyribonucleotide triphosphates (dNTPs, i.e. dATP, dGTP, dCTP, and dTTP) are required), appropriate primers for specific amplification of the target nucleic acid, a suitable buffer for carrying out the amplification reaction, and any necessary cofactors (for example magnesium ions) required by the enzyme activities. Examples of suitable buffers include Tris-HCl, HEPES, or acetate buffer. A suitable salt may be provided, such as potassium chloride or sodium chloride. Suitable concentrations of these components may readily be determined by the skilled person. Suitable rNTP concentrations are typically in the range 0.25-5mM, or 0.5-2.5mM. Suitable dNTP concentrations are typically in the range 0.25-5mM dNTP, or 0.5-2.5mM. Suitable magnesium ion concentrations are typically in the range 5-15mM.

Some conventional transcription-based amplification methods use very high amounts of T7 RNA polymerase (for example 142 or more units, where one unit incorporates 1nmole of labelled nucleotide into acid insoluble material in 1 hour at 37°C under standard assay conditions, such as: 40mM Tris-HCl (pH8.0), 50mM NaCl, 8mM $MgCl_2$, 5mM DTT, 400$\mu$M rNTPs, 400$\mu$M [$^3$H]-UTP(30cpm/pmoles), 20$\mu$g/ml T7 DNA, 50$\mu$g/ml BSA, 100$\mu$l reaction volume, 37°C, 10min.). Methods of the invention can be carried out using significantly less T7 RNA polymerase than such conventional methods, thereby reducing cost. For example, methods of the invention can be carried out using less than 142 units of a DNA-dependent RNA polymerase (for example T7 RNA polymerase), suitably less than 100 units or less than 50 units, such as 30-40 units.

Optionally nucleic acid of the sample is isolated before reverse transcribing SARS-CoV-2 RNA of the sample present in the isolated nucleic acid.

Many suitable methods for isolation of nucleic acid are known to the skilled person. Some methods use chaotropic agents, such as guanidinium thiocyanate, and organic solvents to lyse cells, and denature proteins. For example, Boom et al. (Journal of Clinical Microbiology, 1990, Vol. 28(3): 495-503) describes methods in which a sample is contacted with silica particles in the presence of a lysis/binding buffer containing guanidinium thiocyanate.

Released nucleic acid binds to the silica particles, which are then washed with a wash buffer containing guanidinium thiocyanate, then with ethanol, and then acetone. The bound nucleic acid is subsequently eluted in an aqueous low salt buffer (Tris-HCl, EDTA, pH 8.0).

Some methods avoid the requirement for chaotropic salts and organic solvents. For example, Hourfar et al. (Clinical Chemistry, 2005, 51(7): 1217-1222) describes methods in which a sample is mixed with magnetic silica particles in the presence of a lysis/binding buffer containing a kosmotropic salt (ammonium sulphate) before addition of proteinase K. Following separation, the magnetic particles are washed with wash buffer containing proteinase K, and eluted in elution buffer (Tris-HCl, pH 8.5) at 80$^0$C. Other suitable methods are described in WO 2010/015835.

Isolation of nucleic acid may be carried out using conventional binding buffers and/or elution buffers for use with a solid phase that is able to bind the nucleic acid in the presence of binding buffer at a first pH, and from which the nucleic acid can be eluted at a second pH.

Optionally the solid phase comprises an ionisable group, which changes charge according to the ambient conditions. The pKa of the ionisable group is appropriate to the conditions at which it is desired to bind nucleic acid to and release nucleic acid from the solid phase. Generally, nucleic acid will bind to the solid phase at a pH below or roughly equal to the pKa, and will be released at a higher pH (usually above the pKa). Suitable solid phases for binding a nucleic acid at a first pH, and elution of bound nucleic acid at a second pH that is higher than the first pH, are well known to those of ordinary skill in the art. For example, at the first pH the solid phase may comprise a positive charge, and at the second pH the solid phase may have a less positive, neutral, or negative charge. Alternatively or additionally, at the first pH the solid phase may comprise a neutral or less negative charge, and at the second pH the solid phase may have a negative or more negative charge. Such changes in charge allow the nucleic acid to be adsorbed to the solid phase at the first pH, and released at the second pH.

For example, the solid phase may comprise a negatively ionisable group with a pKa between the first and second pH. Nucleic acid will bind to the solid phase when the solid phase is neutral or less negatively charged, and will be released when the solid phase is negatively or more negatively charged. Alternatively, or additionally, the solid phase may comprise a positively ionisable group with a pKa between the first and second pH. Nucleic acid will bind to the solid phase when the solid phase is positively charged, and will be released when the solid phase is neutral or less positively charged.

Examples of solid phases that may be used for extraction of nucleic acid include solid phases that comprise inorganic oxides, such as silica or glass (for example, as described in Boom *et al,* or Hourfar *et al*), or aluminium oxide, sugar polymers, or charge-switch materials (for example, as described in WO 02/48164).

The solid phase may be in any suitable form, for example comprising a membrane, gel, or particles, for example magnetic particles. Silica membrane or gel, and magnetic silica particles are preferred examples. Silica membrane is particularly preferred. This is less expensive than magnetic silica particles (used for example by Hourfar, *et al*.) and does not require refrigerated storage, unlike magnetic silica particles.

The solid phase may be a solid phase to which binding of nucleic acid is enhanced by the presence of a kosmotropic agent. Optionally binding of the nucleic acid to the solid phase is carried out in the presence of a kosmotropic agent. Such agents are known to enhance binding of nucleic acid to solid phases such as silica-based solid phases.

The terms "chaotropic" and "kosmotropic" agent originate from the Hofmeister series (Cacace et al., Q Rev Biophys 1997;30:241-77), which divides these agents depending on their influence on the structure of macromolecules and water. A chaotrope may be defined as a substance that breaks solvent structure, and a kosmotrope as a substance that enhances solvent structure. Figure 1 of Cacace *et al* shows the Hofmeister series and commonly occurring organic solutes with effects on protein structure/function. Examples of chaotropic agents are known to those in the art, and include sodium iodide, sodium perchlorate, guanidinium thiocyanate and guanidinium hydrochloride. Examples of kosmotropic agents are known to those in the art, and include ammonium sulphate and lithium chloride.

Optionally lysis is carried out using the binding buffer. Binding buffers that may be used for cell lysis are known to those of ordinary skill in the art. The lysis buffer used by Boom *et al.* comprises guanidinium thiocyanate, Tris hydrochloride, pH 6.4, EDTA (adjusted to pH 8), and Triton X-100. Optionally, the lysis buffer does not include a chaotropic agent. For example, a lysis/binding buffer that comprises a kosmotropic agent may be used. Optionally the buffer is an acidic buffer, suitably a strong acidic buffer with a pKa (25°C) in the range 3-5.

Optionally a method of the invention further comprises capturing a product of the isothermal amplification reaction by hybridising nucleic acid of the product to a nucleic acid capture probe, wherein the capture probe hybridises specifically to nucleic acid sequence that is conserved in SARS-CoV-2 nucleic acid, but not in other human coronavirus nucleic acid, or the complement thereof.

Optionally the other human coronavirus nucleic acid is human coronavirus 229E, SARS, HKU1, MERS, OC43, and NL63 nucleic acid.

Optionally the nucleic acid sequence that is conserved in SARS-CoV-2 nucleic acid, but not in other human coronavirus nucleic acid, comprises a contiguous nucleic acid sequence that is at least 19 nucleotides long.

Optionally a capture probe hybridises specifically to nucleic acid sequence that is conserved in SARS-CoV-2 nucleic acid, or the complement thereof, if it hybridises under stringent conditions to nucleic acid sequence that is conserved in SARS-CoV-2 nucleic acid, or the complement thereof, but not to other human coronavirus nucleic acid, or the complement thereof.

Optionally the forward and reverse nucleic acid primers hybridise specifically to nucleic acid sequence that is conserved in the ORF1ab gene of SARS-CoV-2, or the complement thereof, and the capture probe hybridizes specifically to nucleic acid sequence that is conserved in the ORF1ab gene of SARS-CoV-2, or the complement thereof.

Optionally the capture probe comprises a nucleic acid of sequence: GGCAGTGAGGACAATCAGCAACTAC (SEQ ID NO:8), GAGGACAATCAGACAACTACTATTC (SEQ ID NO:9), ACCCGTCCTTGATTGGCTTG (SEQ ID NO:10), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:8, 9, or 10, or the complement thereof.

Optionally the capture probe comprises a nucleic acid sequence of SEQ ID NO:9.

Optionally the forward and reverse nucleic acid primers hybridise specifically to nucleic acid sequence that is conserved in the nucleocapsid gene of SARS-CoV-2, or the complement thereof, and the capture probe hybridizes specifically to nucleic acid sequence that is conserved in the nucleocapsid gene of SARS-CoV-2, or the complement thereof.

Optionally the capture probe comprises a nucleic acid of sequence: CTGGTTCTAAATCACCCATTCA (SEQ ID NO:18), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:18, or the complement thereof.

Optionally the capture probe comprises a nucleic acid of SEQ ID NO:18.

Optionally a method of the invention further comprises detecting a product of the isothermal amplification reaction by hybridising the product to a nucleic acid detector probe, wherein the detector probe hybridises specifically to nucleic acid sequence that is conserved in SARS-CoV-2 nucleic acid, but not in other human coronavirus nucleic acid, or the complement thereof.

Optionally the other human coronavirus nucleic acid is human coronavirus 229E, SARS, HKU1, MERS, OC43, and NL63 nucleic acid.

Optionally the nucleic acid sequence that is conserved in SARS-CoV-2 nucleic acid, but not in other human coronavirus nucleic acid, comprises a contiguous nucleic acid sequence that is at least 19 nucleotides long.

Optionally a detector probe hybridises specifically to nucleic acid sequence that is conserved in SARS-CoV-2 nucleic acid, or the complement thereof, if it hybridises under stringent conditions to nucleic acid sequence that is conserved in SARS-CoV-2 nucleic acid, or the complement thereof, but not to other human coronavirus nucleic acid, or the complement thereof.

Optionally the forward and reverse nucleic acid primers hybridise specifically to nucleic acid sequence that is conserved in the ORF1ab gene of SARS-CoV-2, or the complement thereof, and the detector probe hybridizes specifically to nucleic acid sequence that is conserved in the ORF1ab gene of SARS-CoV-2, or the complement thereof.

Optionally the detector probe comprises a nucleic acid sequence of: CAAACAATTGTTGAGGTTCAACCTC (SEQ ID NO:11), GAGGTTCAACCTCAATTAGAGATGG (SEQ ID NO:12), GGAAGGTGTAGAGTTTCTTAGAGAC (SEQ ID NO:13), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:11, 12, or 13, or the complement thereof.

Optionally a method of the disclosure comprises amplifying with forward and reverse nucleic acid amplification primers, capture of amplification product with capture probe, and detection of amplification product with detector probe, wherein the amplification primers and capture and detector probes comprise respective nucleic acid sequences according to any of the combinations of forward and reverse primer sequences, and capture probe (CP) and detector probe (DP) shown in Table 2 below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

Table 2

| Combination | Forward | Reverse | CP | DP |
|---|---|---|---|---|
| 1 | SEQ ID NO:1 (nCoV-ARP1.1F) | SEQ ID NO:5 (nCoV-ARP1.1R) | SEQ ID NO:9 (nCoV-ARCP1.2) | SEQ ID NO:12 (nCoV-ARDP1.2) |
| 2 | SEQ ID NO:2 (nCoV-ARP1.2F) | SEQ ID NO:5 (nCoV-ARP1.1R) | SEQ ID NO:8 (nCoV-ARCP1.1) | SEQ ID NO:11 (nCoV-ARDP1.1) |
| 3 | SEQ ID NO:2 (nCoV-ARP1.2F) | SEQ ID NO:5 (nCoV-ARP1.1R) | SEQ ID NO:8 (nCoV-ARCP1.1) | SEQ ID NO:12 (nCoV-ARDP1.2) |
| 4 | SEQ ID NO:2 (nCoV-ARP1.2F) | SEQ ID NO:5 (nCoV-ARP1.1R) | SEQ ID NO:9 (nCoV-ARCP1.2) | SEQ ID NO:12 (nCoV-ARDP1.2) |
| 5 | SEQ ID NO:3 (nCoV-ARP1.3F) | SEQ ID NO:5 (nCoV-ARP1.1R) | SEQ ID NO:8 (nCoV-ARCP1.1) | SEQ ID NO:11 (nCoV-ARDP1.1) |
| 6 | SEQ ID NO:3 (nCoV-ARP1.3F) | SEQ ID NO:5 (nCoV-ARP1.1R) | SEQ ID NO:8 (nCoV-ARCP1.1) | SEQ ID NO:12 (nCoV-ARDP1.2) |
| 7 | SEQ ID NO:3 (nCoV-ARP1.3F) | SEQ ID NO:5 (nCoV-ARP1.1R) | SEQ ID NO:9 (nCoV-ARCP1.2) | SEQ ID NO:12 (nCoV-ARDP1.2) |
| 8 | SEQ ID NO:2 (nCoV-ARP1.2F) | SEQ ID NO:6 (nCoV-ARP1.2R) | SEQ ID NO:8 (nCoV-ARCP1.1) | SEQ ID NO:11 (nCoV-ARDP1.1) |
| 9 | SEQ ID NO:3 (nCoV-ARP1.3F) | SEQ ID NO:6 (nCoV-ARP1.2R) | SEQ ID NO:8 (nCoV-ARCP1.1) | SEQ ID NO:11 (nCoV-ARDP1.1) |
| 10 | SEQ ID NO:4 (SA_nCoV_F2) | SEQ ID NO:7 (SA_nCoV_R2) | SEQ ID NO:10 (SA_n-CoV_CP2) | SEQ ID NO:13 (SA_n-CoV_DP2) |

Optionally the detector probe comprises a nucleic acid sequence of SEQ ID NO:12.

Optionally the forward and reverse nucleic acid primers hybridise specifically to nucleic acid sequence that is conserved in the nucleocapsid gene of SARS-CoV-2, or the complement thereof, and the detector probe hybridizes specifically to nucleic acid sequence that is conserved in the nucleocapsid gene of SARS-CoV-2, or the complement thereof.

Optionally the detector probe comprises a nucleic acid sequence of: GAACCTAAATTGGGTAGTCTTGTAG (SEQ ID NO:19), GGAACCTAAATTGGGTAGTCTTG (SEQ ID NO:20), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO: 19 or 20, or the complement thereof.

Optionally the detector probe comprises a nucleic acid sequence of SEQ ID NO:19.

The locations in the SARS-CoV-2 ORF1ab gene sequence corresponding to the sequence of the capture probes of SEQ ID NOs:8,9 (2019-nCoV-AR-CP1.1, 2019-nCoV-AR-CP1.2, respectively), and the detector probes of SEQ ID NOs:11, 12 (2019-nCoV-AR-DP1.1, 2019-nCoV-AR-DP1.2, respectively), are shown in Figure 2.

The locations in the SARS-CoV-2 nucleocapsid gene sequence corresponding to the sequence of the capture probe of SEQ ID NO:18 (SA-nCoV-CP2.3), and the detector probe of SEQ ID NOs:19, 20 (SA-nCoV-DP2.3, SA-nCoV-DP2.4, respectively), are shown in Figure 3.

Sequence identity between nucleic acid sequences can be determined by comparing an alignment of the sequences. When an equivalent position in the compared sequences is occupied by the same nucleotide, then the molecules are identical at that position. Scoring an alignment as a percentage of identity is a function of the number of identical nucleotides at positions shared by the compared sequences. When comparing sequences, optimal alignments may require gaps to be introduced into one or more of the sequences to take into consideration possible insertions and deletions in the sequences. Sequence comparison methods may employ gap penalties so that, for the same number of identical molecules in sequences being compared, a sequence alignment with as few gaps as possible, reflecting higher relatedness between the two compared sequences, will achieve a higher score than one with many gaps. Calculation of maximum percent identity involves the production of an optimal alignment, taking into consideration gap penalties.

Suitable computer programs for carrying out sequence comparisons are widely available in the commercial and public sector. Examples include MatGat (Campanella et al., 2003, BMC Bioinformatics 4: 29; program available from http://bitincka.com/ledion/matgat), Gap (Needleman & Wunsch, 1970, J. Mol. Biol. 48: 443-453), FASTA (Altschul et al., 1990, J. Mol. Biol. 215: 403-410; program available from http://www.ebi.ac.uk/fasta), Clustal W 2.0 and X 2.0 (Larkin et al., 2007, Bioinformatics 23: 2947-2948; program available from http://www.ebi.ac.uk/tools/clustalw2) and EMBOSS Pairwise Alignment Algorithms (Needleman & Wunsch, 1970, supra; Kruskal, 1983, In: Time warps, string edits and macromolecules: the theory and practice of sequence comparison, Sankoff & Kruskal (eds), pp 1-44, Addison Wesley; programs available from http://www.ebi.ac.uk/tools/emboss/align). All programs may be run using default parameters.

For example, sequence comparisons may be undertaken using the "needle" method of the EMBOSS Pairwise Alignment Algorithms, which determines an optimum alignment (including gaps) of two sequences when considered over their entire length and provides a percentage identity score.

Optionally the detector probe is labelled with a detectable label. Optionally the label is a visually detectable label (i.e. a label that is detectable by eye, without the aid of instrumentation).

Examples of suitable visually detectable labels include colloidal metal sol particles, latex particles, or textile dye particles. An example of colloidal metal sol particles is colloidal gold particles.

Optionally capture and/or detection of the product of the isothermal amplification reaction is carried out by chromatographic dipstick assay.

A product of the isothermal nucleic acid amplification may be labelled with a visually detectable label, and captured and detected using a chromatographic test strip, for example as described in WO 2008/090340, and Lee et al., Journal of Infectious Diseases 2010;201(S1):S65-S71.

Optionally the sample is a biological sample, for example a biological sample obtained from a subject suspected of being infected with SARS-CoV-2. Optionally the sample is a swab sample obtained from a subject suspected of being infected with SARS-CoV-2. Optionally the sample is a nasopharyngeal or a throat swab sample obtained from a subject suspected of being infected with SARS-CoV-2.

Optionally a method of the invention is an *in vitro* method.

Optionally a method of the invention comprises amplifying nucleic acid of the sample, or amplifying nucleic acid derived from nucleic acid of the sample, by an isothermal amplification reaction using a first forward nucleic acid amplification primer and a first reverse nucleic acid amplification primer, and a second forward nucleic acid amplification primer and a second reverse nucleic acid amplification primer, wherein each nucleic acid amplification primer hybridises specifically to nucleic acid sequence that is conserved in SARS-CoV-2 nucleic acid, but not in other human coronavirus nucleic acid, or the complement thereof, and wherein the first forward nucleic acid amplification primer and the first reverse nucleic acid amplification primer hybridise specifically to nucleic acid sequence that is conserved in the ORF1ab gene of SARS-CoV-2 nucleic acid, or the complement thereof, and the second forward nucleic acid amplification primer and the second reverse nucleic acid amplification primer hybridise specifically to nucleic acid sequence that is conserved in the nucleocapsid gene of SARS-CoV-2 nucleic acid, or the complement thereof.

Optionally a method of the invention further comprises capturing a product of the isothermal amplification reaction using the first forward nucleic acid amplification primer and the first reverse nucleic acid amplification primer by hybridising nucleic acid of the product to a first nucleic acid capture probe, wherein the first capture probe hybridises specifically to nucleic acid sequence that is conserved in SARS-CoV-2 nucleic acid, but not in other human coronavirus nucleic acid, or the complement thereof, and capturing a product of the isothermal amplification reaction using the second forward nucleic acid amplification primer and the second reverse nucleic acid amplification primer by hybridising nucleic acid of the product to a second nucleic acid capture probe, wherein the second capture probe hybridises specifically to nucleic acid sequence that is conserved in SARS-CoV-2 nucleic acid, but not in other human coronavirus nucleic acid, or the complement thereof, and wherein the first capture probe hybridises specifically to nucleic acid sequence that is conserved in the ORF1ab gene of SARS-CoV-2 nucleic acid, or the complement thereof, and the second capture probe hybridises specifically to nucleic acid sequence that is conserved in the nucleocapsid gene of SARS-CoV-2 nucleic acid, or the complement thereof.

Optionally a method of the invention further comprises detecting a product of the isothermal amplification reaction using the first forward nucleic acid amplification primer and the first reverse nucleic acid amplification primer by hybridising nucleic acid of the product to a first nucleic acid detector probe, wherein the first detector probe hybridises specifically to nucleic acid sequence that is conserved in SARS-CoV-2 nucleic acid, but not in other human coronavirus nucleic acid, or the complement thereof, and detecting a product of the isothermal amplification reaction using the second forward nucleic acid amplification primer and the second reverse nucleic acid amplification primer by hybridising nucleic acid of the product to a second nucleic acid detector probe, wherein the second detector probe hybridises specifically to nucleic acid sequence that is conserved in SARS-CoV-2 nucleic acid, but not in other human coronavirus nucleic acid, or the complement thereof, and wherein the first detector probe hybridises specifically to nucleic acid sequence that is conserved in the ORF1ab gene of SARS-CoV-2 nucleic acid, or the complement thereof, and the second detector probe hybridises specifically to nucleic acid sequence that is conserved in the nucleocapsid gene of SARS-CoV-2 nucleic acid, or the complement thereof.

Optionally the first forward nucleic acid primer comprises a nucleic acid sequence of: CTGTTGGTCAACAA GACGGCA (SEQ ID NO:1), GTCAACAAACTGTTGGTCAA (SEQ ID NO:2), GTCAACAAACTGTTGGTCAACA (SEQ ID NO:3), CATTACAGGTGGTGTTGTTCAGTT (SEQ ID NO:4), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, 2, 3, or 4, or the complement thereof.

Optionally the first reverse nucleic acid primer comprises a nucleic acid sequence: CAATAGTCTGAACAAC TGGTGT (SEQ ID NO:5), CTGGTGTAAGTTCCATCTCT (SEQ ID NO:6), AGGTGACAATTTGTCCACCGAC (SEQ ID NO:7), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, 6, or 7, or the complement thereof.

Optionally the first forward and reverse nucleic acid amplification primers comprise respective nucleic acid sequences according to any of the combinations of forward and reverse primer sequences shown in the table below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

| Combination | Forward | Reverse |
|---|---|---|
| 1 | SEQ ID NO:1 (nCoV-ARP1.1F) | SEQ ID NO:5 (nCoV-ARP1.1R) |
| 2 | SEQ ID NO:2 (nCoV-ARP1.2F) | SEQ ID NO:5 (nCoV-ARP1.1R) |
| 3 | SEQ ID NO:3 (nCoV-ARP1.3F) | SEQ ID NO:5 (nCoV-ARP1.1R) |
| 4 | SEQ ID NO:2 (nCoV-ARP1.2F) | SEQ ID NO:6 (nCoV-ARP1.2R) |
| 5 | SEQ ID NO:3 (nCoV-ARP1.3F) | SEQ ID NO:6 (nCoV-ARP1.2R) |
| 6 | SEQ ID NO:4 (SA_nCoV_F2) | SEQ ID NO:7 (SA_nCoV_R2) |

Optionally the first forward nucleic acid amplification primer comprises a nucleic acid sequence of SEQ ID NO:1, and the first reverse nucleic acid amplification primer comprises a nucleic acid sequence of SEQ ID NO:5.

Optionally the second forward nucleic acid primer comprises a nucleic acid sequence of: TAGTTGATGACC CGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14, or the complement thereof.

Optionally the second reverse nucleic acid primer comprises a nucleic acid sequence: TGGGGTCCATTATC AGACAT (SEQ ID NO:15), CAACACGAACGTCATGATAC (SEQ ID NO:16), CATAGAACGAACAACGCAC (SEQ ID NO:17), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:15, 16, or 17, or the complement thereof.

Optionally the second forward nucleic acid amplification primer comprises a nucleic acid sequence of SEQ ID NO:14, and the second reverse nucleic acid amplification primer comprises a nucleic acid sequence of SEQ ID NO:16.

Optionally the first and/or second reverse nucleic acid primer further comprises a promoter sequence for a DNA-dependent RNA polymerase at its 5'-end for reverse transcription of SARS-CoV-2 RNA using the reverse nucleic acid primer.

Optionally the first capture probe comprises a nucleic acid of sequence: GGCAGTGAGGACAATCAGCAACTAC (SEQ ID NO:8), GAGGACAATCAGACAACTACTATTC (SEQ ID NO:9), ACCCGTCCTTGATTGGCTTG (SEQ ID NO:10), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:8, 9, or 10, or the complement thereof.

Optionally the first capture probe comprises a nucleic acid sequence of SEQ ID NO:9.

Optionally the second capture probe comprises a nucleic acid of sequence: CTGGTTCTAAATCACCCATTCA (SEQ ID NO:18), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:18, or the complement thereof.

Optionally the first detector probe comprises a nucleic acid sequence of: CAAACAATTGTTGAGGTTCAACCTC (SEQ ID NO:11), GAGGTTCAACCTCAATTAGAGATGG (SEQ ID NO:12), GGAAGGTGTAGAGTTTCTTAGAGAC (SEQ ID NO:13), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:11, 12, or 13, or the complement thereof.

Optionally the first forward and reverse amplification primers, and the first capture and detector probes comprise respective nucleic acid sequences according to any of the combinations of forward and reverse primer sequences, and capture probe (CP) and detector probe (DP) shown in the table below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

| Combination | Forward | Reverse | CP | DP |
|---|---|---|---|---|
| 1 | SEQ ID NO:1 (nCoV-ARP1.1F) | SEQ ID NO:5 (nCoV-ARP1.1R) | SEQ ID NO:9 (nCoV-ARCP1.2) | SEQ ID NO:12 (nCoV-ARDP1.2) |
| 2 | SEQ ID NO:2 (nCoV-ARP1.2F) | SEQ ID NO:5 (nCoV-ARP1.1R) | SEQ ID NO:8 (nCoV-ARCP1.1) | SEQ ID NO:11 (nCoV-ARDP1.1) |
| 3 | SEQ ID NO:2 (nCoV-ARP1.2F) | SEQ ID NO:5 (nCoV-ARP1.1R) | SEQ ID NO:8 (nCoV-ARCP1.1) | SEQ ID NO:12 (nCoV-ARDP1.2) |
| 4 | SEQ ID NO:2 (nCoV-ARP1.2F) | SEQ ID NO:5 (nCoV-ARP1.1R) | SEQ ID NO:9 (nCoV-ARCP1.2) | SEQ ID NO:12 (nCoV-ARDP1.2) |
| 5 | SEQ ID NO:3 (nCoV-ARP1.3F) | SEQ ID NO:5 (nCoV-ARP1.1R) | SEQ ID NO:8 (nCoV-ARCP1.1) | SEQ ID NO:11 (nCoV-ARDP1.1) |
| 6 | SEQ ID NO:3 (nCoV-ARP1.3F) | SEQ ID NO:5 (nCoV-ARP1.1R) | SEQ ID NO:8 (nCoV-ARCP1.1) | SEQ ID NO:12 (nCoV-ARDP1.2) |
| 7 | SEQ ID NO:3 (nCoV-ARP1.3F) | SEQ ID NO:5 (nCoV-ARP1.1R) | SEQ ID NO:9 (nCoV-ARCP1.2) | SEQ ID NO:12 (nCoV-ARDP1.2) |
| 8 | SEQ ID NO:2 (nCoV-ARP1.2F) | SEQ ID NO:6 (nCoV-ARP1.2R) | SEQ ID NO:8 (nCoV-ARCP1.1) | SEQ ID NO:11 (nCoV-ARDP1.1) |
| 9 | SEQ ID NO:3 (nCoV-ARP1.3F) | SEQ ID NO:6 (nCoV-ARP1.2R) | SEQ ID NO:8 (nCoV-ARCP1.1) | SEQ ID NO:11 (nCoV-ARDP1.1) |
| 10 | SEQ ID NO:4 (SA_nCoV_F2) | SEQ ID NO:7 (SA_nCoV_R2) | SEQ ID NO:10 (SA_nCoV_CP2) | SEQ ID NO:13 (SA_nCoV_DP2) |

Optionally the first detector probe comprises a nucleic acid sequence of SEQ ID NO:12.

Optionally the second detector probe comprises a nucleic acid sequence of: GAACCTAAATTGGGTAGTCTT GTAG (SEQ ID NO:19), GGAACCTAAATTGGGTAGTCTTG (SEQ ID NO:20), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO: 19 or 20, or the complement thereof.

Optionally the second detector probe comprises a nucleic acid sequence of SEQ ID NO:19.

Amplification using first forward and reverse amplification primers specific for the ORF1ab gene, and second forward and reverse amplification primers specific for the nucleocapsid gene (and optional capture and detection with probes specific to the ORF1ab and nucleocapsid genes), provides methods with exceptional sensitivity and specificity.

For embodiments of a method of the invention comprising an isothermal amplification reaction using a first forward nucleic acid amplification primer and a first reverse nucleic acid amplification primer, and a second forward nucleic acid amplification primer and a second reverse nucleic acid amplification primer, capture and/or detection of the product of the isothermal amplification reactions may be carried out by chromatographic dipstick assay using a chromatographic test strip. The test strip may comprise a first capture zone for capturing a product of the amplification reaction using the first forward and reverse nucleic acid amplification primers, and a second, separate capture zone for capturing a product of the amplification reaction using the second forward and reverse nucleic acid amplification primers.

Methods of the invention are particularly useful as POC tests for testing or screening for SARS-CoV-2 infection. In particular, methods of the invention can be carried out rapidly, without use of laboratory facilities or thermal cyclers. SARS-CoV-2 infection can be potentially be detected using a method of the invention before the subject is showing symptoms of COVID-19. Once a subject has been identified as being infected with SARS-CoV-2, they can be isolated, administered appropriate treatment, and the infection can be monitored.

The invention also provides a kit for carrying out a method of the invention.

There is also provided according to the invention a kit for determining whether a sample includes SARS-CoV-2 nucleic acid, which comprises:

a forward nucleic acid amplification primer and a reverse nucleic acid amplification primer, for amplifying a template nucleic acid by an isothermal amplification reaction, wherein each nucleic acid amplification primer hybridises specifically to nucleic acid sequence that is conserved in SARS-CoV-2 nucleic acid, but not in other human coronavirus nucleic acid, or the complement thereof;

a nucleic acid capture probe, wherein the capture probe hybridises specifically to nucleic acid sequence that is conserved in SARS-CoV-2 nucleic acid, but not in other human coronavirus nucleic acid, or the complement thereof; and/or

a nucleic acid detector probe, wherein the detector probe hybridises specifically to nucleic acid sequence that is conserved in SARS-CoV-2 nucleic acid, but not in other human coronavirus nucleic acid, or the complement thereof, optionally wherein the detector probe comprises a detectable label for labelling a product of the isothermal nucleic acid amplification, wherein:

the forward nucleic acid primer hybridizes specifically to nucleic acid sequence that is conserved in the ORF1ab gene of SARS-CoV-2, or the complement thereof, wherein the forward and reverse nucleic acid amplification primers comprise respective nucleic acid sequences of SEQ ID NO:1 (forward) and SEQ ID NO:5 (reverse), or nucleic acid sequences which have at least 85%, 86%, 87%, 88%, 89%, 90%, 91%, 92%, 93%, 94%, 95%, 96%, 97%, 98%, or 99% identity along their entire length with such sequences, or the complement thereof; and/or

the forward nucleic acid primer hybridizes specifically to nucleic acid sequence that is conserved in the nucleocapsid gene of SARS-CoV-2, or the complement thereof, wherein the forward nucleic acid primer comprises a nucleic acid sequence of: TAGTTGATGACCCGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14, or the complement thereof, and wherein the reverse nucleic acid primer hybridizes specifically to nucleic acid sequence that is conserved in the nucleocapsid gene of SARS-CoV-2, or the complement thereof, wherein the reverse nucleic acid primer comprises a nucleic acid sequence CAACACGAACGTCATGATAC (SEQ ID NO:16), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:16, or the complement thereof, optionally wherein the forward nucleic acid amplification primer comprises a nucleic acid sequence of SEQ ID NO:14, and the reverse nucleic acid amplification primer comprises a nucleic acid sequence of SEQ ID NO:16.

Optionally the other human coronavirus nucleic acid is human coronavirus 229E, SARS, HKU1, MERS, OC43, and NL63 nucleic acid.

The forward and reverse nucleic acid amplification primers, or the capture and/or detector probes, of a kit of the disclosure may be any of the forward and reverse nucleic acid amplification primers, or any of the capture and/or detector probes, or any of the combinations of primers and/or probes as recited herein for methods of the disclosure.

Optionally the nucleic acid sequence that is conserved in SARS-CoV-2 nucleic acid, but not in other human coronavirus nucleic acid, comprises a contiguous nucleic acid sequence that is at least 19 nucleotides long.

Optionally the forward nucleic acid primer hybridizes specifically to nucleic acid sequence that is conserved in the ORF1ab gene of SARS-CoV-2, or the complement thereof.

Optionally the forward nucleic acid primer comprises a nucleic acid sequence of: CTGTTGGTCAACAAGAC GGCA (SEQ ID NO:1), GTCAACAAACTGTTGGTCAA (SEQ ID NO:2), GTCAACAAACTGTTGGTCAACA (SEQ ID NO:3), CATTACAGGTGGTGTTGTTCAGTT (SEQ ID NO:4), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, 2, 3, or 4.

Optionally the reverse nucleic acid primer hybridizes specifically to nucleic acid sequence that is conserved in the ORF1ab gene of SARS-CoV-2, or the complement thereof.

Optionally the reverse nucleic acid primer comprises a nucleic acid sequence: CAATAGTCTGAACAACTGG TGT (SEQ ID NO:5), CTGGTGTAAGTTCCATCTCT (SEQ ID NO:6), AGGTGACAATTTGTCCACCGAC (SEQ ID NO:7), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, 6, or 7.

Optionally the forward and reverse nucleic acid amplification primers comprise respective nucleic acid sequences according to any of the combinations of forward and reverse primer sequences shown in the table below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

| Combination | Forward | Reverse |
|---|---|---|
| 1 | SEQ ID NO:1 (nCoV-ARP1.1F) | SEQ ID NO:5 (nCoV-ARP1.1R) |
| 2 | SEQ ID NO:2 (nCoV-ARP1.2F) | SEQ ID NO:5 (nCoV-ARP1.1R) |
| 3 | SEQ ID NO:3 (nCoV-ARP1.3F) | SEQ ID NO:5 (nCoV-ARP1.1R) |
| 4 | SEQ ID NO:2 (nCoV-ARP1.2F) | SEQ ID NO:6 (nCoV-ARP1.2R) |
| 5 | SEQ ID NO:3 (nCoV-ARP1.3F) | SEQ ID NO:6 (nCoV-ARP1.2R) |
| 6 | SEQ ID NO:4 (SA_nCoV_F2) | SEQ ID NO:7 (SA_nCoV_R2) |

Optionally the forward nucleic acid amplification primer comprises a nucleic acid sequence of SEQ ID NO:1, and the reverse nucleic acid amplification primer comprises a nucleic acid sequence of SEQ ID NO:5.

Optionally the forward nucleic acid primer hybridizes specifically to nucleic acid sequence that is conserved in the nucleocapsid gene of SARS-CoV-2, or the complement thereof.

Optionally the forward nucleic acid primer comprises a nucleic acid sequence of: TAGTTGATGACCCGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14.

Optionally the reverse nucleic acid primer hybridizes specifically to nucleic acid sequence that is conserved in the nucleocapsid gene of SARS-CoV-2, or the complement thereof.

Optionally the reverse nucleic acid primer comprises a nucleic acid sequence: TGGGGTCCATTATCAGACAT (SEQ ID NO:15), CAACACGAACGTCATGATAC (SEQ ID NO:16), CATAGAACGAACAACGCAC (SEQ ID NO:17), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:15, 16, or 17.

Optionally the forward nucleic acid amplification primer comprises a nucleic acid sequence of SEQ ID NO:14, and the reverse nucleic acid amplification primer comprises a nucleic acid sequence of SEQ ID NO:16.

Optionally the reverse nucleic acid primer further comprises a promoter sequence for a DNA-dependent RNA polymerase at its 5'-end for reverse transcription of SARS-CoV-2 RNA using the reverse nucleic acid primer.

Optionally the forward and reverse nucleic acid primers hybridise specifically to nucleic acid sequence that is conserved in the ORF1ab gene of SARS-CoV-2, or the complement thereof, and the capture probe hybridizes specifically to nucleic acid sequence that is conserved in the ORF1ab gene of SARS-CoV-2, or the complement thereof.

Optionally the capture probe comprises a nucleic acid of sequence: GGCAGTGAGGACAATCAGCAACTAC (SEQ ID NO:8), GAGGACAATCAGACAACTACTATTC (SEQ ID NO:9), ACCCGTCCTTGATTGGCTTG (SEQ ID NO:10), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:8, 9, or 10, or the complement thereof.

Optionally the capture probe comprises a nucleic acid sequence of SEQ ID NO:9.

Optionally the forward and reverse nucleic acid primers hybridise specifically to nucleic acid sequence that is conserved in the nucleocapsid gene of SARS-CoV-2, or the complement thereof, and the capture probe hybridizes specifically to nucleic acid sequence that is conserved in the nucleocapsid gene of SARS-CoV-2, or the complement thereof.

Optionally the capture probe comprises a nucleic acid of sequence: CTGGTTCTAAATCACCCATTCA (SEQ ID NO:18), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:18, or the complement thereof.

Optionally the forward and reverse nucleic acid primers hybridise specifically to nucleic acid sequence that is conserved in the ORF1ab gene of SARS-CoV-2, or the complement thereof, and the detector probe hybridizes specifically to nucleic acid sequence that is conserved in the ORF1ab gene of SARS-CoV-2, or the complement thereof.

Optionally the detector probe comprises a nucleic acid sequence of: CAAACAATTGTTGAGGTTCAACCTC (SEQ ID NO:11), GAGGTTCAACCTCAATTAGAGATGG (SEQ ID NO:12), GGAAGGTGTAGAGTTTCTTAGAGAC (SEQ ID NO:13), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:11, 12, or 13, or the complement thereof.

Optionally a kit of the disclosure comprises forward and reverse amplification primers, and capture and detector probes comprising respective nucleic acid sequences according to any of the combinations of forward and reverse primer sequences, and capture probe (CP) and detector probe (DP) shown in the table below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

| Combination | Forward | Reverse | CP | DP |
|---|---|---|---|---|
| 1 | SEQ ID NO:1 (nCoV-ARP1.1F) | SEQ ID NO:5 (nCoV-ARP1.1R) | SEQ ID NO:9 (nCoV-ARCP1.2) | SEQ ID NO:12 (nCoV-ARDP1.2) |
| 2 | SEQ ID NO:2 (nCoV-ARP1.2F) | SEQ ID NO:5 (nCoV-ARP1.1R) | SEQ ID NO:8 (nCoV-ARCP1.1) | SEQ ID NO:11 (nCoV-ARDP1.1) |
| 3 | SEQ ID NO:2 (nCoV-ARP1.2F) | SEQ ID NO:5 (nCoV-ARP1.1R) | SEQ ID NO:8 (nCoV-ARCP1.1) | SEQ ID NO:12 (nCoV-ARDP1.2) |
| 4 | SEQ ID NO:2 (nCoV-ARP1.2F) | SEQ ID NO:5 (nCoV-ARP1.1R) | SEQ ID NO:9 (nCoV-ARCP1.2) | SEQ ID NO:12 (nCoV-ARDP1.2) |
| 5 | SEQ ID NO:3 (nCoV-ARP1.3F) | SEQ ID NO:5 (nCoV-ARP1.1R) | SEQ ID NO:8 (nCoV-ARCP1.1) | SEQ ID NO:11 (nCoV-ARDP1.1) |
| 6 | SEQ ID NO:3 (nCoV-ARP1.3F) | SEQ ID NO:5 (nCoV-ARP1.1R) | SEQ ID NO:8 (nCoV-ARCP1.1) | SEQ ID NO:12 (nCoV-ARDP1.2) |
| 7 | SEQ ID NO:3 (nCoV-ARP1.3F) | SEQ ID NO:5 (nCoV-ARP1.1R) | SEQ ID NO:9 (nCoV-ARCP1.2) | SEQ ID NO:12 (nCoV-ARDP1.2) |
| 8 | SEQ ID NO:2 (nCoV-ARP1.2F) | SEQ ID NO:6 (nCoV-ARP1.2R) | SEQ ID NO:8 (nCoV-ARCP1.1) | SEQ ID NO:11 (nCoV-ARDP1.1) |
| 9 | SEQ ID NO:3 (nCoV-ARP1.3F) | SEQ ID NO:6 (nCoV-ARP1.2R) | SEQ ID NO:8 (nCoV-ARCP1.1) | SEQ ID NO:11 (nCoV-ARDP1.1) |
| 10 | SEQ ID NO:4 (SA_nCoV_F2) | SEQ ID NO:7 (SA_nCoV_R2) | SEQ ID NO:10 (SA_n-CoV_CP2) | SEQ ID NO:13 (SA_n-CoV_DP2) |

Optionally the detector probe comprises a nucleic acid sequence of SEQ ID NO:12.

Optionally the forward and reverse nucleic acid primers hybridise specifically to nucleic acid sequence that is conserved in the nucleocapsid gene of SARS-CoV-2, or the complement thereof, and the detector probe hybridizes specifically to nucleic acid sequence that is conserved in the nucleocapsid gene of SARS-CoV-2, or the complement thereof.

Optionally the detector probe comprises a nucleic acid sequence of: GAACCTAAATTGGGTAGTCTTGTAG (SEQ ID NO:19), GGAACCTAAATTGGGTAGTCTTG (SEQ ID NO:20), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO: 19 or 20, or the complement thereof.

Optionally the detector probe comprises a nucleic acid sequence of SEQ ID NO:19.

There is also provided a kit for determining whether a sample includes SARS-CoV-2 nucleic acid, which comprises:

a first forward nucleic acid amplification primer and a first reverse nucleic acid amplification primer; and

a second forward nucleic acid amplification primer and a second reverse nucleic acid amplification primer; wherein each nucleic acid amplification primer hybridises specifically to nucleic acid sequence that is conserved in SARS-CoV-2 nucleic acid, but not in other human coronavirus nucleic acid, or the complement thereof, and wherein the first forward nucleic acid amplification primer and the first reverse nucleic acid amplification primer hybridise specifically to nucleic acid sequence that is conserved in the ORF1ab gene of SARS-CoV-2 nucleic acid, or the complement thereof, and the second forward nucleic acid amplification primer and the second reverse nucleic acid amplification primer hybridise specifically to nucleic acid sequence that is conserved in the nucleocapsid gene of SARS-CoV-2 nucleic acid, or the complement thereof;

a first nucleic acid capture probe, and a second nucleic acid capture probe, wherein the first and second nucleic acid capture probe each hybridise specifically to nucleic acid sequence that is conserved in SARS-CoV-2 nucleic acid, but not in other human coronavirus nucleic acid, or the complement thereof, and wherein the first nucleic acid capture probe hybridises specifically to nucleic acid sequence that is conserved in the ORF1ab gene of SARS-CoV-2 nucleic acid, or the complement thereof, and the second nucleic acid capture probe hybridises specifically to nucleic acid sequence that is conserved in the nucleocapsid gene of SARS-CoV-2 nucleic acid, or the complement thereof; and/or

a first nucleic acid detector probe, and a second nucleic acid detector probe, wherein the first and second nucleic acid detector probe each hybridise specifically to nucleic acid sequence that is conserved in SARS-CoV-2 nucleic acid, but not in other human coronavirus nucleic acid, or the complement thereof, and wherein the first nucleic acid detector probe hybridises specifically to nucleic acid sequence that is conserved in the ORF1ab gene of SARS-CoV-2 nucleic acid, or the complement thereof, and the second nucleic acid detector probe hybridises specifically to nucleic acid sequence that is conserved in the nucleocapsid gene of SARS-CoV-2 nucleic acid, or the complement thereof.

Optionally the first forward nucleic acid primer comprises a nucleic acid sequence of: CTGTTGGTCAACAA GACGGCA (SEQ ID NO:1), GTCAACAAACTGTTGGTCAA (SEQ ID NO:2), GTCAACAAACTGTTGGTCAACA (SEQ ID NO:3), CATTACAGGTGGTGTTGTTCAGTT (SEQ ID NO:4), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, 2, 3, or 4.

Optionally the first reverse nucleic acid primer comprises a nucleic acid sequence: CAATAGTCTGAACAAC TGGTGT (SEQ ID NO:5), CTGGTGTAAGTTCCATCTCT (SEQ ID NO:6), AGGTGACAATTTGTCCACCGAC (SEQ ID NO:7), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, 6, or 7.

Optionally the first forward and reverse nucleic acid amplification primers comprise respective nucleic acid sequences according to any of the combinations of forward and reverse primer sequences shown in the table below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

| Combination | Forward | Reverse |
|---|---|---|
| 1 | SEQ ID NO:1 (nCoV-ARP1.1F) | SEQ ID NO:5 (nCoV-ARP1.1R) |
| 2 | SEQ ID NO:2 (nCoV-ARP1.2F) | SEQ ID NO:5 (nCoV-ARP1.1R) |
| 3 | SEQ ID NO:3 (nCoV-ARP1.3F) | SEQ ID NO:5 (nCoV-ARP1.1R) |
| 4 | SEQ ID NO:2 (nCoV-ARP1.2F) | SEQ ID NO:6 (nCoV-ARP1.2R) |
| 5 | SEQ ID NO:3 (nCoV-ARP1.3F) | SEQ ID NO:6 (nCoV-ARP1.2R) |
| 6 | SEQ ID NO:4 (SA_nCoV_F2) | SEQ ID NO:7 (SA_nCoV_R2) |

Optionally the first forward nucleic acid amplification primer comprises a nucleic acid sequence of SEQ ID NO:1, and the first reverse nucleic acid amplification primer comprises a nucleic acid sequence of SEQ ID NO:5.

Optionally the second forward nucleic acid primer comprises a nucleic acid sequence of: TAGTTGATGACC CGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14.

Optionally the second reverse nucleic acid primer comprises a nucleic acid sequence: TGGGGTCCATTATC AGACAT (SEQ ID NO:15), CAACACGAACGTCATGATAC (SEQ ID NO:16), CATAGAACGAACAACGCAC (SEQ ID NO:17), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:15, 16, or 17.

Optionally the second forward nucleic acid amplification primer comprises a nucleic acid sequence of SEQ ID NO:14, and the second reverse nucleic acid amplification primer comprises a nucleic acid sequence of SEQ ID NO:16.

Optionally the first and/or second reverse nucleic acid primer further comprises a promoter sequence for a DNA-dependent RNA polymerase at its 5'-end for reverse transcription of SARS-CoV-2 RNA using the reverse nucleic acid primer.

Optionally the first capture probe comprises a nucleic acid of sequence: GGCAGTGAGGACAATCAGCAACTAC (SEQ ID NO:8), GAGGACAATCAGACAACTACTATTC (SEQ ID NO:9), ACCCGTCCTTGATTGGCTTG (SEQ ID NO:10), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:8, 9, or 10, or the complement thereof.

Optionally the first capture probe comprises a nucleic acid sequence of SEQ ID NO:9.

Optionally the second capture probe comprises a nucleic acid of sequence: CTGGTTCTAAATCACCCATTCA (SEQ ID NO:18), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:18, or the complement thereof.

Optionally the first detector probe comprises a nucleic acid sequence of: CAAACAATTGTTGAGGTTCAACCTC (SEQ ID NO:11), GAGGTTCAACCTCAATTAGAGATGG (SEQ ID NO:12), GGAAGGTGTAGAGTTTCTTAGAGAC (SEQ ID NO:13), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:11, 12, or 13, or the complement thereof.

Optionally the first forward and reverse amplification primers, and the first capture and detector probes comprise respective nucleic acid sequences according to any of the combinations of forward and reverse primer sequences, and capture probe (CP) and detector probe (DP) shown in the table below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

| Combination | Forward | Reverse | CP | DP |
|---|---|---|---|---|
| 1 | SEQ ID NO:1 (nCoV-ARP1.1F) | SEQ ID NO:5 (nCoV-ARP1.1R) | SEQ ID NO:9 (nCoV-ARCP1.2) | SEQ ID NO:12 (nCoV-ARDP1.2) |
| 2 | SEQ ID NO:2 (nCoV-ARP1.2F) | SEQ ID NO:5 (nCoV-ARP1.1R) | SEQ ID NO:8 (nCoV-ARCP1.1) | SEQ ID NO:11 (nCoV-ARDP1.1) |
| 3 | SEQ ID NO:2 (nCoV-ARP1.2F) | SEQ ID NO:5 (nCoV-ARP1.1R) | SEQ ID NO:8 (nCoV-ARCP1.1) | SEQ ID NO:12 (nCoV-ARDP1.2) |
| 4 | SEQ ID NO:2 (nCoV-ARP1.2F) | SEQ ID NO:5 (nCoV-ARP1.1R) | SEQ ID NO:9 (nCoV-ARCP1.2) | SEQ ID NO:12 (nCoV-ARDP1.2) |
| 5 | SEQ ID NO:3 (nCoV-ARP1.3F) | SEQ ID NO:5 (nCoV-ARP1.1R) | SEQ ID NO:8 (nCoV-ARCP1.1) | SEQ ID NO:11 (nCoV-ARDP1.1) |
| 6 | SEQ ID NO:3 (nCoV-ARP1.3F) | SEQ ID NO:5 (nCoV-ARP1.1R) | SEQ ID NO:8 (nCoV-ARCP1.1) | SEQ ID NO:12 (nCoV-ARDP1.2) |
| 7 | SEQ ID NO:3 (nCoV-ARP1.3F) | SEQ ID NO:5 (nCoV-ARP1.1R) | SEQ ID NO:9 (nCoV-ARCP1.2) | SEQ ID NO:12 (nCoV-ARDP1.2) |
| 8 | SEQ ID NO:2 (nCoV-ARP1.2F) | SEQ ID NO:6 (nCoV-ARP1.2R) | SEQ ID NO:8 (nCoV-ARCP1.1) | SEQ ID NO:11 (nCoV-ARDP1.1) |
| 9 | SEQ ID NO:3 (nCoV-ARP1.3F) | SEQ ID NO:6 (nCoV-ARP1.2R) | SEQ ID NO:8 (nCoV-ARCP1.1) | SEQ ID NO:11 (nCoV-ARDP1.1) |
| 10 | SEQ ID NO:4 (SA_nCoV_F2) | SEQ ID NO:7 (SA_nCoV_R2) | SEQ ID NO:10 (SA_n-CoV_CP2) | SEQ ID NO:13 (SA_n-CoV_DP2) |

Optionally the first detector probe comprises a nucleic acid sequence of SEQ ID NO:12.

Optionally the second detector probe comprises a nucleic acid sequence of: GAACCTAAATTGGGTAGTCTTGTAG (SEQ ID NO:19), GGAACCTAAATTGGGTAGTCTTG (SEQ ID NO:20), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO: 19 or 20, or the complement thereof.

Optionally the second detector probe comprises a nucleic acid sequence of SEQ ID NO:19.

Optionally a kit of the invention further comprises an RNA-dependent DNA polymerase, a DNA-dependent DNA polymerase, a DNA/RNA duplex-specific ribonuclease, and a DNA-dependent RNA polymerase.

Optionally a kit of the invention further comprises appropriate nucleotide triphosphates (for transcription-based amplifications ribonucleotide triphosphates (rNTPs, i.e. rATP, rGTP, rCTP, and rUTP), and deoxyribonucleotide triphosphates (dNTPs, i.e. dATP, dGTP, dCTP, and dTTP) are required), a suitable buffer for carrying out the amplification reaction, and any necessary cofactors (for example magnesium ions) required by the enzyme activities. Examples of suitable buffers include Tris-HCl, HEPES, or acetate buffer. A suitable salt may be provided, such as potassium chloride or sodium chloride. Suitable concentrations of these components may readily be determined by the skilled person. Suitable rNTP concentrations are typically in the range 0.25-5mM, or 0.5-2.5mM. Suitable dNTP concentrations are typically in the range 0.25-5mM dNTP, or 0.5-2.5mM. Suitable magnesium ion concentrations are typically in the range 5-15mM.

A kit of the invention may further comprise a detectable label (for example, a visually detectable label) for labelling a product of the isothermal nucleic acid amplification and/or a chromatographic test strip and reagents for capturing and detecting a product of the isothermal nucleic acid amplification. Examples of suitable labels, test strips, and reagents, and methods for capturing and detecting a product of the isothermal nucleic acid amplification by a simple amplification-based assay (SAMBA), are described in WO 2008/090340 and Lee et al., Journal of Infectious Diseases 2010;201(S1):S65-S71. An amplification device for carrying out SAMBA methods, and apparatus for carrying out automated SAMBA methods, are described in WO 2014/140640.

For embodiments of a kit of the invention comprising a first forward and reverse nucleic acid primer, a second forward and reverse nucleic acid primer, a first and second nucleic acid capture probe and/or a first and second nucleic acid detector probe, the chromatographic test strip may comprise a first capture zone for capturing a product of the amplification reaction using the first forward and reverse nucleic acid amplification primers, and a second, separate capture zone for capturing a product of the amplification reaction using the second forward and reverse nucleic acid amplification primers.

SAMBA enables complex NAT to be carried out in doctor's offices, primary and community healthcare settings and small hospitals where complex NAT technology cannot be used. The technology can be instrumental in public health emergencies such as the current Corona virus outbreak. SAMBA POC testing augments testing capacity, alleviating delays due to sample transport and locating infected patients. SAMBA allows accurate NAT at POC settings in ~1 hour, thus enabling healthcare workers to rapidly and correctly identify, isolate and treat patients infectious for SARS-CoV-2 -- keys to limiting the present outbreak. SAMBA will give more hospitals capability for SARS-CoV-2 RNA testing, allowing infected patients to be identified and treated without waiting for results from a centralized testing laboratory.

A kit of the invention may further comprise reagents for isolating nucleic acid from a sample, for example using a method of nucleic acid extraction as described above. Suitable reagents for extracting nucleic acid may include a lysis buffer for lysing cells present in the sample, a solid phase for binding nucleic acid, a binding buffer for binding nucleic acid to the solid phase (optionally, the lysis buffer is the same as the binding buffer) optionally a wash buffer for washing nucleic acid bound to the solid phase, and an elution buffer for eluting nucleic acid from the solid phase. Suitable lysis, wash, and elution buffers are described above, as well as suitable solid phases for use with the buffers.

Optionally a kit of the invention further comprises a lysis/binding buffer, an elution buffer, and optionally a wash buffer, for extracting nucleic acid from a biological sample obtained from the subject.

A kit of the invention may further comprise a swab stick for obtaining a nasopharyngeal or throat swab sample from a subject.

Optionally a kit of the invention comprises a first swab stick for obtaining a nasopharyngeal swab sample from a subject, and a second swab stick for obtaining a throat swab sample from a subject.

Suitable swab sticks are commercially available: Copan Diagnostics Nylon Flocked Dry Swabs in Peel Pouches (Copan Diagnostics 503CS01).

Optionally a kit of the invention further comprises a chromatographic test strip for capturing and detecting a product of the isothermal nucleic acid amplification.

Optionally a kit of the invention further comprises positive and/or negative controls.

Optionally a kit of the invention further comprises instructions for carrying out a method of the invention using the kit.

According to the invention there is also provided a set of primers for amplifying SARS-CoV-2 nucleic acid by an isothermal nucleic acid amplification reaction, which comprises a forward nucleic acid amplification primer and a reverse nucleic acid amplification primer, wherein each nucleic acid amplification primer hybridises specifically to nucleic acid sequence that is conserved in SARS-CoV-2 nucleic acid, but not in other human coronavirus nucleic acid, or the complement thereof, wherein the nucleic acid sequence that is conserved in SARS-CoV-2 nucleic acid is nucleic acid sequence that is conserved in the ORF1ab gene or the Nucleocapsid gene of SARS-CoV-2, or the complement thereof, wherein:

the forward nucleic acid primer hybridizes specifically to nucleic acid sequence that is conserved in the ORF1ab gene of SARS-CoV-2, or the complement thereof, wherein the forward nucleic acid primer comprises a nucleic acid sequence of: CTGTTGGTCAACAAGACGGCA (SEQ ID NO:1), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, or the complement thereof, and the reverse nucleic acid primer hybridizes specifically to nucleic acid sequence that is conserved in the ORF1ab gene of SARS-CoV-2, or the complement thereof, wherein the reverse nucleic acid primer comprises a nucleic acid sequence: CAATAGTCTGAACAACTG GTGT (SEQ ID NO:5), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, or the complement thereof; and/or

the forward nucleic acid primer hybridizes specifically to nucleic acid sequence that is conserved in the nucleocapsid gene of SARS-CoV-2, or the complement thereof, wherein the forward nucleic acid primer comprises a nucleic acid sequence of: TAGTTGATGACCCGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14, or the complement thereof, and wherein the reverse nucleic acid primer hybridizes specifically to nucleic acid sequence that is conserved in the nucleocapsid gene of SARS-CoV-2, or the complement thereof, wherein the reverse nucleic acid primer comprises a nucleic acid sequence: CAACACGAA CGTCATGATAC (SEQ ID NO:16), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:16, or the complement thereof;

optionally wherein the forward and/or the reverse nucleic acid primer is up to 50 nucleotides long.

Optionally the other human coronavirus nucleic acid is human coronavirus 229E, SARS, HKU1, MERS, OC43, and NL63 nucleic acid.

The forward and reverse nucleic acid amplification primers of a set of primers of the invention may be any of the forward and reverse nucleic acid amplification primers, or any of the combinations of primers as recited herein for methods of the invention.

Optionally the nucleic acid sequence that is conserved in SARS-CoV-2 nucleic acid, but not in other human coronavirus nucleic acid, comprises a contiguous nucleic acid sequence that is at least 19 nucleotides long.

Optionally the forward nucleic acid primer hybridizes specifically to nucleic acid sequence that is conserved in the ORF1ab gene of SARS-CoV-2, or the complement thereof.

Optionally the forward nucleic acid primer comprises a nucleic acid sequence of: CTGTTGGTCAACAAGAC GGCA (SEQ ID NO:1), GTCAACAAACTGTTGGTCAA (SEQ ID NO:2), GTCAACAAACTGTTGGTCAACA (SEQ ID NO:3), CATTACAGGTGGTGTTGTTCAGTT (SEQ ID NO:4), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, 2, 3, or 4.

Optionally the reverse nucleic acid primer hybridizes specifically to nucleic acid sequence that is conserved in the ORF1ab gene of SARS-CoV-2, or the complement thereof.

Optionally the reverse nucleic acid primer comprises a nucleic acid sequence: CAATAGTCTGAACAACTGG TGT (SEQ ID NO:5), CTGGTGTAAGTTCCATCTCT (SEQ ID NO:6), AGGTGACAATTTGTCCACCGAC (SEQ ID NO:7), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, 6, or 7.

Optionally the forward and reverse nucleic acid amplification primers comprise respective nucleic acid sequences according to any of the combinations of forward and reverse primer sequences shown in the table below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

| Combination | Forward | Reverse |
|---|---|---|
| 1 | SEQ ID NO:1 (nCoV-ARP1.1F) | SEQ ID NO:5 (nCoV-ARP1.1R) |
| 2 | SEQ ID NO:2 (nCoV-ARP1.2F) | SEQ ID NO:5 (nCoV-ARP1.1R) |
| 3 | SEQ ID NO:3 (nCoV-ARP1.3F) | SEQ ID NO:5 (nCoV-ARP1.1R) |
| 4 | SEQ ID NO:2 (nCoV-ARP1.2F) | SEQ ID NO:6 (nCoV-ARP1.2R) |
| 5 | SEQ ID NO:3 (nCoV-ARP1.3F) | SEQ ID NO:6 (nCoV-ARP1.2R) |
| 6 | SEQ ID NO:4 (SA_nCoV_F2) | SEQ ID NO:7 (SA_nCoV_R2) |

Optionally the forward nucleic acid amplification primer comprises a nucleic acid sequence of SEQ ID NO:1, and the reverse nucleic acid amplification primer comprises a nucleic acid sequence of SEQ ID NO:5.

Optionally the forward nucleic acid primer hybridizes specifically to nucleic acid sequence that is conserved in the nucleocapsid gene of SARS-CoV-2, or the complement thereof.

Optionally the forward nucleic acid primer comprises a nucleic acid sequence of: TAGTTGATGACCCGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14.

Optionally the reverse nucleic acid primer hybridizes specifically to nucleic acid sequence that is conserved in the nucleocapsid gene of SARS-CoV-2, or the complement thereof.

Optionally the reverse nucleic acid primer comprises a nucleic acid sequence: TGGGGTCCATTATCAGACAT (SEQ ID NO:15), CAACACGAACGTCATGATAC (SEQ ID NO:16), CATAGAACGAACAACGCAC (SEQ ID NO:17), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:15, 16, or 17.

Optionally the forward nucleic acid amplification primer comprises a nucleic acid sequence of SEQ ID NO:14, and the reverse nucleic acid amplification primer comprises a nucleic acid sequence of SEQ ID NO:16.

Optionally the reverse nucleic acid primer further comprises a promoter sequence for a DNA-dependent RNA polymerase at its 5'-end.

Optionally the forward and/or the reverse nucleic acid primer is up to 50 nucleotides long.

There is also provided a set of primers for amplifying SARS-CoV-2 nucleic acid by an isothermal nucleic acid amplification reaction, which comprises:

a first forward nucleic acid amplification primer and a first reverse nucleic acid amplification primer; and

a second forward nucleic acid amplification primer and a second reverse nucleic acid amplification primer;

wherein each nucleic acid amplification primer hybridises specifically to nucleic acid sequence that is conserved in SARS-CoV-2 nucleic acid, but not in other human coronavirus nucleic acid, or the complement thereof, and wherein the first forward nucleic acid amplification primer and the first reverse nucleic acid amplification primer hybridise specifically to nucleic acid sequence that is conserved in the ORF1ab gene of SARS-CoV-2 nucleic acid, or the complement thereof, and the second forward nucleic acid amplification primer and the second reverse nucleic acid amplification primer hybridise specifically to nucleic acid sequence that is conserved in the nucleocapsid gene of SARS-CoV-2 nucleic acid, or the complement thereof.

Optionally the first forward nucleic acid primer comprises a nucleic acid sequence of: CTGTTGGTCAACAA GACGGCA (SEQ ID NO:1), GTCAACAAACTGTTGGTCAA (SEQ ID NO:2), GTCAACAAACTGTTGGTCAACA (SEQ ID NO:3), CATTACAGGTGGTGTTGTTCAGTT (SEQ ID NO:4), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, 2, 3, or 4.

Optionally the first reverse nucleic acid primer comprises a nucleic acid sequence: CAATAGTCTGAACAAC TGGTGT (SEQ ID NO:5), CTGGTGTAAGTTCCATCTCT (SEQ ID NO:6), AGGTGACAATTTGTCCACCGAC (SEQ ID NO:7), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, 6, or 7.

Optionally the first forward and reverse nucleic acid amplification primers comprise respective nucleic acid sequences according to any of the combinations of forward and reverse primer sequences shown in the table below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

| Combination | Forward | Reverse |
|---|---|---|
| 1 | SEQ ID NO:1 (nCoV-ARP1.1F) | SEQ ID NO:5 (nCoV-ARP1.1R) |
| 2 | SEQ ID NO:2 (nCoV-ARP1.2F) | SEQ ID NO:5 (nCoV-ARP1.1R) |
| 3 | SEQ ID NO:3 (nCoV-ARP1.3F) | SEQ ID NO:5 (nCoV-ARP1.1R) |
| 4 | SEQ ID NO:2 (nCoV-ARP1.2F) | SEQ ID NO:6 (nCoV-ARP1.2R) |
| 5 | SEQ ID NO:3 (nCoV-ARP1.3F) | SEQ ID NO:6 (nCoV-ARP1.2R) |
| 6 | SEQ ID NO:4 (SA_nCoV_F2) | SEQ ID NO:7 (SA_nCoV_R2) |

Optionally the first forward nucleic acid amplification primer comprises a nucleic acid sequence of SEQ ID NO:1, and the first reverse nucleic acid amplification primer comprises a nucleic acid sequence of SEQ ID NO:5.

Optionally the second forward nucleic acid primer comprises a nucleic acid sequence of: TAGTTGATGACC CGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14.

Optionally the second reverse nucleic acid primer comprises a nucleic acid sequence: TGGGGTCCATTATC AGACAT (SEQ ID NO:15), CAACACGAACGTCATGATAC (SEQ ID NO:16), CATAGAACGAACAACGCAC (SEQ ID NO:17), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:15, 16, or 17.

Optionally the second forward nucleic acid amplification primer comprises a nucleic acid sequence of SEQ ID NO:14, and the second reverse nucleic acid amplification primer comprises a nucleic acid sequence of SEQ ID NO:16.

Optionally the first and/or second reverse nucleic acid primer further comprises a promoter sequence for a DNA-dependent RNA polymerase at its 5'-end for reverse transcription of SARS-CoV-2 RNA using the reverse nucleic acid primer.

There is also provided according to the invention a set of oligonucleotides for amplifying SARS-CoV-2 nucleic acid by an isothermal nucleic acid amplification reaction, and for capturing and/or detecting a product of the amplification reaction, which comprises:

a set of primers of the invention;

a nucleic acid capture probe, wherein the capture probe hybridises specifically to nucleic acid sequence that is conserved in SARS-CoV-2 nucleic acid, but not in other human coronavirus nucleic acid, or the complement thereof; and/or

a nucleic acid detector probe, wherein the detector probe hybridises specifically to nucleic acid sequence that is conserved in SARS-CoV-2 nucleic acid, but not in other human coronavirus nucleic acid, or the complement thereof, optionally wherein the detector probe comprises a detectable label for labelling a product of the isothermal nucleic acid amplification.

Optionally the other human coronavirus nucleic acid is human coronavirus 229E, SARS, HKU1, MERS, OC43, and NL63 nucleic acid.

The forward and reverse nucleic acid amplification primers, or the capture and/or detector probes, of a set of oligonucleotides of the invention may be any of the forward and reverse nucleic acid amplification primers, or any of the capture and/or detector probes, or any of the combinations of primers and/or probes as recited herein for methods of the invention.

Optionally the nucleic acid sequence that is conserved in SARS-CoV-2 nucleic acid, but not in other human coronavirus nucleic acid, comprises a contiguous nucleic acid sequence that is at least 19 nucleotides long.

Optionally the forward nucleic acid primer hybridizes specifically to nucleic acid sequence that is conserved in the ORF1ab gene of SARS-CoV-2, or the complement thereof.

Optionally the forward nucleic acid primer comprises a nucleic acid sequence of: CTGTTGGTCAACAAGAC GGCA (SEQ ID NO:1), GTCAACAAACTGTTGGTCAA (SEQ ID NO:2), GTCAACAAACTGTTGGTCAACA (SEQ ID NO:3), CATTACAGGTGGTGTTGTTCAGTT (SEQ ID NO:4), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, 2, 3, or 4.

Optionally the reverse nucleic acid primer hybridizes specifically to nucleic acid sequence that is conserved in the ORF1ab gene of SARS-CoV-2, or the complement thereof.

Optionally the reverse nucleic acid primer comprises a nucleic acid sequence: CAATAGTCTGAACAACTGG TGT (SEQ ID NO:5), CTGGTGTAAGTTCCATCTCT (SEQ ID NO:6), AGGTGACAATTTGTCCACCGAC (SEQ ID NO:7), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, 6, or 7.

Optionally the forward and reverse nucleic acid amplification primers comprise respective nucleic acid sequences according to any of the combinations of forward and reverse primer sequences shown in the table below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

| Combination | Forward | Reverse |
|---|---|---|
| 1 | SEQ ID NO:1 (nCoV-ARP1.1F) | SEQ ID NO:5 (nCoV-ARP1.1R) |

(continued)

| Combination | Forward | Reverse |
|---|---|---|
| 2 | SEQ ID NO:2 (nCoV-ARP1.2F) | SEQ ID NO:5 (nCoV-ARP1.1R) |
| 3 | SEQ ID NO:3 (nCoV-ARP1.3F) | SEQ ID NO:5 (nCoV-ARP1.1R) |
| 4 | SEQ ID NO:2 (nCoV-ARP1.2F) | SEQ ID NO:6 (nCoV-ARP1.2R) |
| 5 | SEQ ID NO:3 (nCoV-ARP1.3F) | SEQ ID NO:6 (nCoV-ARP1.2R) |
| 6 | SEQ ID NO:4 (SA_nCoV_F2) | SEQ ID NO:7 (SA_nCoV_R2) |

Optionally the forward nucleic acid amplification primer comprises a nucleic acid sequence of SEQ ID NO:1, and the reverse nucleic acid amplification primer comprises a nucleic acid sequence of SEQ ID NO:5.

Optionally the forward nucleic acid primer hybridizes specifically to nucleic acid sequence that is conserved in the nucleocapsid gene of SARS-CoV-2, or the complement thereof.

Optionally the forward nucleic acid primer comprises a nucleic acid sequence of: TAGTTGATGACCCGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14.

Optionally the reverse nucleic acid primer hybridizes specifically to nucleic acid sequence that is conserved in the nucleocapsid gene of SARS-CoV-2, or the complement thereof.

Optionally the reverse nucleic acid primer comprises a nucleic acid sequence: TGGGGTCCATTATCAGACAT (SEQ ID NO:15), CAACACGAACGTCATGATAC (SEQ ID NO:16), CATAGAACGAACAACGCAC (SEQ ID NO:17), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:15, 16, or 17.

Optionally the forward nucleic acid amplification primer comprises a nucleic acid sequence of SEQ ID NO:14, and the reverse nucleic acid amplification primer comprises a nucleic acid sequence of SEQ ID NO:16.

Optionally the reverse nucleic acid primer further comprises a promoter sequence for a DNA-dependent RNA polymerase at its 5'-end for reverse transcription of SARS-CoV-2 RNA using the reverse nucleic acid primer.

Optionally the forward and reverse nucleic acid primers hybridise specifically to nucleic acid sequence that is conserved in the ORF1ab gene of SARS-CoV-2, or the complement thereof, and the capture probe hybridizes specifically to nucleic acid sequence that is conserved in the ORF1ab gene of SARS-CoV-2, or the complement thereof.

Optionally the capture probe comprises a nucleic acid of sequence: GGCAGTGAGGACAATCAGCAACTAC (SEQ ID NO:8), GAGGACAATCAGACAACTACTATTC (SEQ ID NO:9), ACCCGTCCTTGATTGGCTTG (SEQ ID NO:10), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:8, 9, or 10, or the complement thereof.

Optionally the capture probe comprises a nucleic acid sequence of SEQ ID NO:9.

Optionally the forward and reverse nucleic acid primers hybridise specifically to nucleic acid sequence that is conserved in the nucleocapsid gene of SARS-CoV-2, or the complement thereof, and the capture probe hybridizes specifically to nucleic acid sequence that is conserved in the nucleocapsid gene of SARS-CoV-2, or the complement thereof.

Optionally the capture probe comprises a nucleic acid of sequence: CTGGTTCTAAATCACCCATTCA (SEQ ID NO:18), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:18, or the complement thereof.

Optionally the forward and reverse nucleic acid primers hybridise specifically to nucleic acid sequence that is conserved in the ORF1ab gene of SARS-CoV-2, or the complement thereof, and the detector probe hybridizes specifically to nucleic acid sequence that is conserved in the ORF1ab gene of SARS-CoV-2, or the complement thereof.

Optionally the detector probe comprises a nucleic acid sequence of: CAAACAATTGTTGAGGTTCAACCTC (SEQ ID NO:11), GAGGTTCAACCTCAATTAGAGATGG (SEQ ID NO:12), GGAAGGTGTAGAGTTTCTTAGAGAC (SEQ ID NO:13), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:11, 12, or 13, or the complement thereof.

Optionally a set of oligonucleotides of the disclosure comprises forward and reverse amplification primers, and capture and detector probes comprising respective nucleic acid sequences according to any of the combinations of forward and reverse primer sequences, and capture probe (CP) and detector probe (DP) shown in the table below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

| Combination | Forward | Reverse | CP | DP |
|---|---|---|---|---|
| 1 | SEQ ID NO:1 (nCoV-ARP1.1F) | SEQ ID NO:5 (nCoV-ARP1.1R) | SEQ ID NO:9 (nCoV-ARCP1.2) | SEQ ID NO:12 (nCoV-ARDP1.2) |
| 2 | SEQ ID NO:2 (nCoV-ARP1.2F) | SEQ ID NO:5 (nCoV-ARP1.1R) | SEQ ID NO:8 (nCoV-ARCP1.1) | SEQ ID NO:11 (nCoV-ARDP1.1) |
| 3 | SEQ ID NO:2 (nCoV-ARP1.2F) | SEQ ID NO:5 (nCoV-ARP1.1R) | SEQ ID NO:8 (nCoV-ARCP1.1) | SEQ ID NO:12 (nCoV-ARDP1.2) |
| 4 | SEQ ID NO:2 (nCoV-ARP1.2F) | SEQ ID NO:5 (nCoV-ARP1.1R) | SEQ ID NO:9 (nCoV-ARCP1.2) | SEQ ID NO:12 (nCoV-ARDP1.2) |
| 5 | SEQ ID NO:3 (nCoV-ARP1.3F) | SEQ ID NO:5 (nCoV-ARP1.1R) | SEQ ID NO:8 (nCoV-ARCP1.1) | SEQ ID NO:11 (nCoV-ARDP1.1) |
| 6 | SEQ ID NO:3 (nCoV-ARP1.3F) | SEQ ID NO:5 (nCoV-ARP1.1R) | SEQ ID NO:8 (nCoV-ARCP1.1) | SEQ ID NO:12 (nCoV-ARDP1.2) |
| 7 | SEQ ID NO:3 (nCoV-ARP1.3F) | SEQ ID NO:5 (nCoV-ARP1.1R) | SEQ ID NO:9 (nCoV-ARCP1.2) | SEQ ID NO:12 (nCoV-ARDP1.2) |
| 8 | SEQ ID NO:2 (nCoV-ARP1.2F) | SEQ ID NO:6 (nCoV-ARP1.2R) | SEQ ID NO:8 (nCoV-ARCP1.1) | SEQ ID NO:11 (nCoV-ARDP1.1) |
| 9 | SEQ ID NO:3 (nCoV-ARP1.3F) | SEQ ID NO:6 (nCoV-ARP1.2R) | SEQ ID NO:8 (nCoV-ARCP1.1) | SEQ ID NO:11 (nCoV-ARDP1.1) |
| 10 | SEQ ID NO:4 (SA_nCoV_F2) | SEQ ID NO:7 (SA_nCoV_R2) | SEQ ID NO:10 (SA_n-CoV_CP2) | SEQ ID NO:13 (SA_n-CoV_DP2) |

Optionally the detector probe comprises a nucleic acid sequence of SEQ ID NO:12.

Optionally the forward and reverse nucleic acid primers hybridise specifically to nucleic acid sequence that is conserved in the nucleocapsid gene of SARS-CoV-2, or the complement thereof, and the detector probe hybridizes specifically to nucleic acid sequence that is conserved in the nucleocapsid gene of SARS-CoV-2, or the complement thereof.

Optionally the detector probe comprises a nucleic acid sequence of: GAACCTAAATTGGGTAGTCTTGTAG (SEQ ID NO:19), GGAACCTAAATTGGGTAGTCTTG (SEQ ID NO:20), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO: 19 or 20, or the complement thereof.

Optionally the detector probe comprises a nucleic acid sequence of SEQ ID NO:19.

Optionally the capture and/or detector probe is up to 20, 25, 30, 35, 40, 45, 50, 55, or 60 nucleotides long.

There is also provided a set of oligonucleotides for amplifying SARS-CoV-2 nucleic acid by an isothermal nucleic acid amplification reaction, and for capturing and/or detecting a product of the amplification reaction, which comprises:

a first forward nucleic acid amplification primer and a first reverse nucleic acid amplification primer; and

a second forward nucleic acid amplification primer and a second reverse nucleic acid amplification primer;
wherein each nucleic acid amplification primer hybridises specifically to nucleic acid sequence that is conserved in SARS-CoV-2 nucleic acid, but not in other human coronavirus nucleic acid, or the complement thereof, and wherein the first forward nucleic acid amplification primer and the first reverse nucleic acid amplification primer hybridise specifically to nucleic acid sequence that is conserved in the ORF1ab gene of SARS-CoV-2 nucleic acid, or the complement thereof, and the second forward nucleic acid amplification primer and the second reverse nucleic acid amplification primer hybridise specifically to nucleic acid sequence that is conserved in the nucleocapsid gene of SARS-CoV-2 nucleic acid, or the complement thereof;

a first nucleic acid capture probe, and a second nucleic acid capture probe, wherein the first and second nucleic acid capture probe each hybridise specifically to nucleic acid sequence that is conserved in SARS-CoV-2 nucleic acid, but not in other human coronavirus nucleic acid, or the complement thereof, and wherein the first nucleic acid capture probe hybridises specifically to nucleic acid sequence that is conserved in the ORF1ab gene of SARS-CoV-2 nucleic acid, or the complement thereof, and the second nucleic acid capture probe hybridises specifically to nucleic acid sequence that is conserved in the nucleocapsid gene of SARS-CoV-2 nucleic acid, or the complement thereof; and/or

a first nucleic acid detector probe, and a second nucleic acid detector probe, wherein the first and second nucleic acid detector probe each hybridise specifically to nucleic acid sequence that is conserved in SARS-CoV-2 nucleic acid, but not in other human coronavirus nucleic acid, or the complement thereof, and wherein the first nucleic acid detector probe hybridises specifically to nucleic acid sequence that is conserved in the ORF1ab gene of SARS-CoV-2 nucleic acid, or the complement thereof, and the second nucleic acid detector probe hybridises specifically to nucleic acid sequence that is conserved in the nucleocapsid gene of SARS-CoV-2 nucleic acid, or the complement thereof.

Optionally the first forward nucleic acid primer comprises a nucleic acid sequence of: CTGTTGGTCAACAA GACGGCA (SEQ ID NO:1), GTCAACAAACTGTTGGTCAA (SEQ ID NO:2), GTCAACAAACTGTTGGTCAACA (SEQ ID NO:3), CATTACAGGTGGTGTTGTTCAGTT (SEQ ID NO:4), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, 2, 3, or 4.

Optionally the first reverse nucleic acid primer comprises a nucleic acid sequence: CAATAGTCTGAACAAC TGGTGT (SEQ ID NO:5), CTGGTGTAAGTTCCATCTCT (SEQ ID NO:6), AGGTGACAATTTGTCCACCGAC (SEQ ID NO:7), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, 6, or 7.

Optionally the first forward and reverse nucleic acid amplification primers comprise respective nucleic acid sequences according to any of the combinations of forward and reverse primer sequences shown in the table below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

| Combination | Forward | Reverse |
|---|---|---|
| 1 | SEQ ID NO:1 (nCoV-ARP1.1F) | SEQ ID NO:5 (nCoV-ARP1.1R) |
| 2 | SEQ ID NO:2 (nCoV-ARP1.2F) | SEQ ID NO:5 (nCoV-ARP1.1R) |
| 3 | SEQ ID NO:3 (nCoV-ARP1.3F) | SEQ ID NO:5 (nCoV-ARP1.1R) |
| 4 | SEQ ID NO:2 (nCoV-ARP1.2F) | SEQ ID NO:6 (nCoV-ARP1.2R) |
| 5 | SEQ ID NO:3 (nCoV-ARP1.3F) | SEQ ID NO:6 (nCoV-ARP1.2R) |
| 6 | SEQ ID NO:4 (SA_nCoV_F2) | SEQ ID NO:7 (SA_nCoV_R2) |

Optionally the first forward nucleic acid amplification primer comprises a nucleic acid sequence of SEQ ID NO:1, and the first reverse nucleic acid amplification primer comprises a nucleic acid sequence of SEQ ID NO:5.

Optionally the second forward nucleic acid primer comprises a nucleic acid sequence of: TAGTTGATGACC CGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14.

Optionally the second reverse nucleic acid primer comprises a nucleic acid sequence: TGGGGTCCATTATC AGACAT (SEQ ID NO:15), CAACACGAACGTCATGATAC (SEQ ID NO:16), CATAGAACGAACAACGCAC (SEQ ID NO:17), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:15, 16, or 17.

Optionally the second forward nucleic acid amplification primer comprises a nucleic acid sequence of SEQ ID NO:14, and the second reverse nucleic acid amplification primer comprises a nucleic acid sequence of SEQ ID NO:16.

Optionally the first and/or second reverse nucleic acid primer further comprises a promoter sequence for a DNA-dependent RNA polymerase at its 5'-end for reverse transcription of SARS-CoV-2 RNA using the reverse nucleic acid primer.

Optionally the first capture probe comprises a nucleic acid of sequence: GGCAGTGAGGACAATCAGCAACTAC (SEQ ID NO:8), GAGGACAATCAGACAACTACTATTC (SEQ ID NO:9), ACCCGTCCTTGATTGGCTTG (SEQ ID NO:10), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:8, 9, or 10, or the complement thereof.

Optionally the first capture probe comprises a nucleic acid sequence of SEQ ID NO:9.

Optionally the second capture probe comprises a nucleic acid of sequence: CTGGTTCTAAATCACCCATTCA (SEQ ID NO:18), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:18, or the complement thereof.

Optionally the first detector probe comprises a nucleic acid sequence of: CAAACAATTGTTGAGGTTCAACCTC (SEQ ID NO:11), GAGGTTCAACCTCAATTAGAGATGG (SEQ ID NO:12), GGAAGGTGTAGAGTTTCTTAGAGAC (SEQ ID NO:13), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:11, 12, or 13, or the complement thereof.

Optionally the first forward and reverse amplification primers, and the first capture and detector probes comprise respective nucleic acid sequences according to any of the combinations of forward and reverse primer sequences, and capture probe (CP) and detector probe (DP) shown in the table below, or nucleic acid sequences which have at least 85%, 86%, 87%, 88%, 89%, 90%, 91%, 92%, 93%, 94%, 95%, 96%, 97%, 98%, or 99% identity along their entire length with such sequences:

| Combination | Forward | Reverse | CP | DP |
|---|---|---|---|---|
| 1 | SEQ ID NO:1 (nCoV-ARP1.1F) | SEQ ID NO:5 (nCoV-ARP1.1R) | SEQ ID NO:9 (nCoV-ARCP1.2) | SEQ ID NO:12 (nCoV-ARDP1.2) |
| 2 | SEQ ID NO:2 (nCoV-ARP1.2F) | SEQ ID NO:5 (nCoV-ARP1.1R) | SEQ ID NO:8 (nCoV-ARCP1.1) | SEQ ID NO:11 (nCoV-ARDP1.1) |
| 3 | SEQ ID NO:2 (nCoV-ARP1.2F) | SEQ ID NO:5 (nCoV-ARP1.1R) | SEQ ID NO:8 (nCoV-ARCP1.1) | SEQ ID NO:12 (nCoV-ARDP1.2) |
| 4 | SEQ ID NO:2 (nCoV-ARP1.2F) | SEQ ID NO:5 (nCoV-ARP1.1R) | SEQ ID NO:9 (nCoV-ARCP1.2) | SEQ ID NO:12 (nCoV-ARDP1.2) |
| 5 | SEQ ID NO:3 (nCoV-ARP1.3F) | SEQ ID NO:5 (nCoV-ARP1.1R) | SEQ ID NO:8 (nCoV-ARCP1.1) | SEQ ID NO:11 (nCoV-ARDP1.1) |
| 6 | SEQ ID NO:3 (nCoV-ARP1.3F) | SEQ ID NO:5 (nCoV-ARP1.1R) | SEQ ID NO:8 (nCoV-ARCP1.1) | SEQ ID NO:12 (nCoV-ARDP1.2) |
| 7 | SEQ ID NO:3 (nCoV-ARP1.3F) | SEQ ID NO:5 (nCoV-ARP1.1R) | SEQ ID NO:9 (nCoV-ARCP1.2) | SEQ ID NO:12 (nCoV-ARDP1.2) |
| 8 | SEQ ID NO:2 (nCoV-ARP1.2F) | SEQ ID NO:6 (nCoV-ARP1.2R) | SEQ ID NO:8 (nCoV-ARCP1.1) | SEQ ID NO:11 (nCoV-ARDP1.1) |
| 9 | SEQ ID NO:3 (nCoV-ARP1.3F) | SEQ ID NO:6 (nCoV-ARP1.2R) | SEQ ID NO:8 (nCoV-ARCP1.1) | SEQ ID NO:11 (nCoV-ARDP1.1) |
| 10 | SEQ ID NO:4 (SA_nCoV_F2) | SEQ ID NO:7 (SA_nCoV_R2) | SEQ ID NO:10 (SA_n-CoV_CP2) | SEQ ID NO:13 (SA_n-CoV_DP2) |

Optionally the first detector probe comprises a nucleic acid sequence of SEQ ID NO:12.

Optionally the second detector probe comprises a nucleic acid sequence of: GAACCTAAATTGGGTAGTCTT GTAG (SEQ ID NO:19), GGAACCTAAATTGGGTAGTCTTG (SEQ ID NO:20), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO: 19 or 20, or the complement thereof.

Optionally the second detector probe comprises a nucleic acid sequence of SEQ ID NO:19.

There is also provided an oligonucleotide, which comprises:

a nucleic acid sequence of: CTGTTGGTCAACAAGACGGCA (SEQ ID NO:1), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, or the complement thereof;

a nucleic acid sequence of: GTCAACAAACTGTTGGTCAA (SEQ ID NO:2), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:2, or the complement thereof;

a nucleic acid sequence of: GTCAACAAACTGTTGGTCAACA (SEQ ID NO:3), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:3, or the complement thereof;

a nucleic acid sequence of: CATTACAGGTGGTGTTGTTCAGTT (SEQ ID NO:4), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:4, or the complement thereof;

a nucleic acid sequence of: CAATAGTCTGAACAACTGGTGT (SEQ ID NO:5), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, or the complement thereof;

a nucleic acid sequence of: CTGGTGTAAGTTCCATCTCT (SEQ ID NO:6), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:6, or the complement thereof;

a nucleic acid sequence of: AGGTGACAATTTGTCCACCGAC (SEQ ID NO:7), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:7, or the complement thereof;

a nucleic acid sequence of: GGCAGTGAGGACAATCAGCAACTAC (SEQ ID NO:8), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:8, or the complement thereof;

a nucleic acid sequence of: GAGGACAATCAGACAACTACTATTC (SEQ ID NO:9), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:9, or the complement thereof;

a nucleic acid sequence of: ACCCGTCCTTGATTGGCTTG (SEQ ID NO:10), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:10, or the complement thereof;

a nucleic acid sequence of: CAAACAATTGTTGAGGTTCAACCTC (SEQ ID NO:11), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:11, or the complement thereof;

a nucleic acid sequence of: GAGGTTCAACCTCAATTAGAGATGG (SEQ ID NO:12), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:12, or the complement thereof;

a nucleic acid sequence of: GGAAGGTGTAGAGTTTCTTAGAGAC (SEQ ID NO:13), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:13, or the complement thereof;

a nucleic acid sequence of: TAGTTGATGACCCGTGTCCT (SEQ ID NO:14), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14, or the complement thereof;

a nucleic acid sequence of: TGGGGTCCATTATCAGACAT (SEQ ID NO:15), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:15, or the complement thereof;

a nucleic acid sequence of: CAACACGAACGTCATGATAC (SEQ ID NO:16), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:16, or the complement thereof;

a nucleic acid sequence of: CATAGAACGAACAACGCAC (SEQ ID NO:17), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:17, or the complement thereof;

a nucleic acid sequence of: CTGGTTCTAAATCACCCATTCA (SEQ ID NO:18), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:18, or the complement thereof;

a nucleic acid sequence of: GAACCTAAATTGGGTAGTCTTGTAG (SEQ ID NO:19), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:19, or the complement thereof; or

a nucleic acid sequence of: GGAACCTAAATTGGGTAGTCTTG (SEQ ID NO:20), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:20, or the complement thereof.

There is also provided according to the invention an oligonucleotide, which comprises:

a nucleic acid sequence of: CTGTTGGTCAACAAGACGGCA (SEQ ID NO:1), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, or the complement thereof;

a nucleic acid sequence of: CAATAGTCTGAACAACTGGTGT (SEQ ID NO:5), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, or the complement thereof;

a nucleic acid sequence of: GAGGACAATCAGACAACTACTATTC (SEQ ID NO:9), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:9, or the complement thereof;

a nucleic acid sequence of: GAGGTTCAACCTCAATTAGAGATGG (SEQ ID NO:12), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:12, or the complement thereof;

a nucleic acid sequence of: TAGTTGATGACCCGTGTCCT (SEQ ID NO:14), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14, or the complement thereof;

a nucleic acid sequence of: CAACACGAACGTCATGATAC (SEQ ID NO:16), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:16, or the complement thereof;

a nucleic acid sequence of: CTGGTTCTAAATCACCCATTCA (SEQ ID NO:18), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:18, or the complement thereof; or

a nucleic acid sequence of: GAACCTAAATTGGGTAGTCTTGTAG (SEQ ID NO:19), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:19, or the complement thereof.

A set of primers, a set of oligonucleotides, or an oligonucleotide, of the invention may be used in a kit of the invention, or in a method of the invention.

A primer, probe, or oligonucleotide of the invention, or of a set of primers or oligonucleotides of the invention, or of a kit of the invention, or for use in a method of the invention, may be at least 15, 20, 25, 30, 35, 40, 45, 50, or over 50 nucleotides in length.

A primer, probe, or oligonucleotide of the invention, or of a set of primers or oligonucleotides of the invention, or of a kit of the invention, or for use in a method of the invention, may be up to 20, 25, 30, 35, 40, 45, 50, 55, 60, or 100 nucleotides in length.

A primer or oligonucleotide of the invention, or of a set of primers or oligonucleotides of the invention, or of a kit of the invention, or for use in a method of the invention, which comprises a nucleic acid sequence of SEQ ID NO:1 may be up to 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the invention, or of a set of primers or oligonucleotides of the invention, or of a kit of the invention, or for use in a method of the invention, which comprises the complement of a nucleic acid sequence of SEQ ID NO:1 may be up to 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the disclosure, or of a set of primers or oligonucleotides of the disclosure, or of a kit of the disclosure, or for use in a method of the disclosure, which comprises a nucleic acid sequence of SEQ ID NO:2 may be up to 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the disclosure, or of a set of primers or oligonucleotides of the disclosure, or of a kit of the disclosure, or for use in a method of the disclosure, which comprises the complement of a nucleic acid sequence of SEQ ID NO:2 may be up to 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the disclosure, or of a set of primers or oligonucleotides of the disclosure, or of a kit of the disclosure, or for use in a method of the disclosure, which comprises a nucleic acid sequence of SEQ ID NO:3 may be up to 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the disclosure, or of a set of primers or oligonucleotides of the disclosure, or of a kit of the disclosure, or for use in a method of the disclosure, which comprises the complement of a nucleic acid sequence of SEQ ID NO:3 may be up to 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the disclosure, or of a set of primers or oligonucleotides of the disclosure, or of a kit of the disclosure, or for use in a method of the disclosure, which comprises a nucleic acid sequence of SEQ ID NO:4 may be up to 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the disclosure, or of a set of primers or oligonucleotides of the disclosure, or of a kit of the disclosure, or for use in a method of the disclosure, which comprises the complement of a nucleic acid sequence of SEQ ID NO:4 may be up to 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the invention, or of a set of primers or oligonucleotides of the invention, or of a kit of the invention, or for use in a method of the invention, which comprises a nucleic acid sequence of SEQ ID NO:5 may be up to 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the invention, or of a set of primers or oligonucleotides of the invention, or of a kit of the invention, or for use in a method of the invention, which comprises the complement of a nucleic acid sequence of SEQ ID NO:5 may be up to 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the disclosure, or of a set of primers or oligonucleotides of the disclosure, or of a kit of the invention, or for use in a method of the disclosure, which comprises a nucleic acid sequence of SEQ ID NO:6 may be up to 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the disclosure, or of a set of primers or oligonucleotides of the disclosure, or of a kit of the disclosure, or for use in a method of the disclosure, which comprises the complement of a nucleic acid sequence of SEQ ID NO:6 may be up to 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the disclosure, or of a set of primers or oligonucleotides of the disclosure, or of a kit of the disclosure, or for use in a method of the disclosure, which comprises a nucleic acid sequence of SEQ ID NO:7 may be up to 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the disclosure, or of a set of primers or oligonucleotides of the disclosure, or of a kit of the disclosure, or for use in a method of the disclosure, which comprises the complement of a nucleic acid sequence of SEQ ID NO:7 may be up to 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the disclosure, or of a set of primers or oligonucleotides of the disclosure, or of a kit of the disclosure, or for use in a method of the disclosure, which comprises a nucleic acid sequence of SEQ ID NO:8 may be up to 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the disclosure, or of a set of primers or oligonucleotides of the disclosure, or of a kit of the disclosure, or for use in a method of the disclosure, which comprises the complement of a nucleic acid sequence of SEQ ID NO:8 may be up to 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the invention, or of a set of primers or oligonucleotides of the invention, or of a kit of the invention, or for use in a method of the invention, which comprises a nucleic acid sequence of SEQ ID NO:9 may be up to 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the invention, or of a set of primers or oligonucleotides of the invention, or of a kit of the invention, or for use in a method of the invention, which comprises the complement of a nucleic acid sequence of SEQ ID NO:9 may be up to 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the disclosure, or of a set of primers or oligonucleotides of the disclosure, or of a kit of the disclosure, or for use in a method of the disclosure, which comprises a nucleic acid sequence of SEQ ID NO:10 may be up to 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the disclosure, or of a set of primers or oligonucleotides of the disclosure, or of a kit of the disclosure, or for use in a method of the disclosure, which comprises the complement of a nucleic acid sequence of SEQ ID NO:10 may be up to 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the disclosure, or of a set of primers or oligonucleotides of the disclosure, or of a kit of the disclosure, or for use in a method of the disclosure, which comprises a nucleic acid sequence of SEQ ID NO:11 may be up to 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the disclosure, or of a set of primers or oligonucleotides of the disclosure, or of a kit of the disclosure, or for use in a method of the disclosure, which comprises the complement of a nucleic acid sequence of SEQ ID NO:11 may be up to 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the invention, or of a set of primers or oligonucleotides of the invention, or of a kit of the invention, or for use in a method of the invention, which comprises a nucleic acid sequence of SEQ ID NO:12 may be up to 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the invention, or of a set of primers or oligonucleotides of the invention, or of a kit of the invention, or for use in a method of the invention, which comprises the complement of a nucleic acid sequence of SEQ ID NO:12 may be up to 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the disclosure, or of a set of primers or oligonucleotides of the disclosure, or of a kit of the disclosure, or for use in a method of the disclosure, which comprises a nucleic acid sequence of SEQ ID NO:13 may be up to 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the disclosure, or of a set of primers or oligonucleotides of the disclosure, or of a kit of the disclosure, or for use in a method of the disclosure, which comprises the complement of a nucleic acid sequence of SEQ ID NO:13 may be up to 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the invention, or of a set of primers or oligonucleotides of the invention, or of a kit of the invention, or for use in a method of the invention, which comprises a nucleic acid sequence of SEQ ID NO:14 may be up to 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the invention, or of a set of primers or oligonucleotides of the invention, or of a kit of the invention, or for use in a method of the invention, which comprises the complement of a nucleic acid sequence of SEQ ID NO:14 may be up to 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the disclosure, or of a set of primers or oligonucleotides of the disclosure, or of a kit of the disclosure, or for use in a method of the disclosure, which comprises a nucleic acid sequence of SEQ ID NO:15 may be up to 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the disclosure, or of a set of primers or oligonucleotides of the disclosure, or of a kit of the disclosure, or for use in a method of the disclosure, which comprises the complement of a nucleic acid sequence of SEQ ID NO:15 may be up to 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the invention, or of a set of primers or oligonucleotides of the invention, or of a kit of the invention, or for use in a method of the invention, which comprises a nucleic acid sequence of SEQ ID NO:16 may be up to 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the invention, or of a set of primers or oligonucleotides of the invention, or of a kit of the invention, or for use in a method of the invention, which comprises the complement of a nucleic acid sequence of SEQ ID NO:16 may be up to 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the disclosure, or of a set of primers or oligonucleotides of the disclosure, or of a kit of the disclosure, or for use in a method of the disclosure, which comprises a nucleic acid sequence of SEQ ID NO:17 may be up to 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the disclosure, or of a set of primers or oligonucleotides of the disclosure, or of a kit of the disclosure, or for use in a method of the disclosure, which comprises the complement of a nucleic acid sequence of SEQ ID NO:17 may be up to 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the invention, or of a set of primers or oligonucleotides of the invention, or of a kit of the invention, or for use in a method of the invention, which comprises a nucleic acid sequence of SEQ ID NO:18 may be up to 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the invention, or of a set of primers or oligonucleotides of the invention, or of a kit of the invention, or for use in a method of the invention, which comprises the complement of a nucleic acid sequence of SEQ ID NO:18 may be up to 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the invention, or of a set of primers or oligonucleotides of the invention, or of a kit of the invention, or for use in a method of the invention, which comprises a nucleic acid sequence of SEQ ID NO:19 may be up to 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the invention, or of a set of primers or oligonucleotides of the invention, or of a kit of the invention, or for use in a method of the invention, which comprises the complement of a nucleic acid sequence of SEQ ID NO:19 may be up to 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

A primer or oligonucleotide of the disclosure, or of a set of primers or oligonucleotides of the disclosure, or of a kit of the disclosure, or for use in a method of the disclosure, which comprises a nucleic acid sequence of SEQ ID NO:20 may be up to 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the disclosure, or of a set of primers or oligonucleotides of the disclosure, or of a kit of the disclosure, or for use in a method of the disclosure, which comprises the complement of a nucleic acid sequence of SEQ ID NO:20 may be up to 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, or 100 nucleotides in length.

An oligonucleotide of the disclosure may comprise a nucleotide sequence that comprises or consists of a sequence that is at least 85%, 86%, 87%, 88%, 89%, 90%, 91%, 92%, 93%, 94%, 95%, 96%, 97%, 98%, or 99% identical, or that is 100% identical, over its entire length to the nucleotide sequence of any of SEQ ID NOs: 1-20, or the complement thereof.

The oligonucleotide may be labelled with a detectable label, for example with a visually detectable label. In particular, an oligonucleotide that comprises or consists of a nucleic acid sequence of SEQ ID NO:11, 12, 13, 19, or 20, or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO: 11, 12, 13, 19, or 20, or the complement thereof, may be labelled with a visually detectable label. Examples of visually detectable labels include colloidal metal sol particles, latex particles, or textile dye particles. An example of colloidal metal sol particles is colloidal gold particles.

A set of primers or oligonucleotides of the invention may comprise an oligonucleotide of the invention.

A kit of the invention may comprise a set of primers, a set of oligonucleotides, or an oligonucleotide, of the invention.

There is also provided according to the invention use of a set of primers, a set of oligonucleotides, or an oligonucleotide, of the invention in a method of the invention.

Embodiments of the invention are described below, by way of example only, with reference to the accompanying drawings in which:

Figure 1 shows the genome organisation of SARS-CoV-2 (isolate Wuhan-Hu-1, GenBank Acc MN908947);

Figure 2A-D shows a multiple sequence alignment of the ORF1ab region for all 7 human coronaviruses (229E, SARS, HKU1, MERS, OC43, and NL63, and SARS-CoV-2 sequences of several different isolates), and a bat SARS-like coronavirus sequence. In the figure, SARS-CoV-2 is referred to by its previous provisional name "2019-nCoV" (2019 novel coronavirus). The locations in the SARS-CoV-2 ORF1ab gene sequence corresponding to the sequence of the forward primers of SEQ ID NOs:1-3 (2019-nCoV-ARP1.1F, 2019-nCoV-ARP1.2F, 2019-nCoV-ARP1.3F, respectively), and to the reverse complement of sequence of the reverse primers of SEQ ID NOs:5,6 (2019-nCoV-ARP1.1R, 2019-nCoV-ARP1.2R, respectively), are also shown;

Figure 3A-I shows a multiple sequence alignment of the nucleocapsid region for all 7 human coronaviruses (229E, SARS, HKU1, MERS, OC43, and NL63, and SARS-CoV-2 sequences of several different isolates), and a bat SARS-like coronavirus sequence. In the figure, SARS-CoV-2 is referred to by its previous provisional name "2019-nCoV" (2019 novel coronavirus). The locations in the SARS-CoV-2 nucleocapsid gene sequence corresponding to the sequence of the forward primer of SEQ ID NO:14 (SA-nCoV-F2.3), and to the reverse complement of sequence of the reverse primers of SEQ ID NOs:15-17 (SA-nCoV-R2.3, SA-nCoV-R2.4, SA-nCoV-R2.5, respectively), are also shown;

Figure 4 shows schematically the steps for transcription-based amplification of a target RNA; and

Figure 5 shows the results of a Point-of-care (POC) nucleic acid test for detecting SARS-CoV-2 using a method according to an embodiment of the invention.

## Example 1

### Design of primers and probes from coronavirus Orf1ab sequence alignments

Nucleic acid sequences of the Orf1ab gene for all seven human coronaviruses (229E, SARS, HKU1, MERS, OC43, NL63, and SARS-CoV-2) were obtained, including three different SARS-CoV-2 sequences, and a bat SARS-like coronavirus sequence. These sequences were aligned using the CLUSTAL 0(1.2.4) multiple sequence alignment software. The multiple sequence alignment is shown in Figure 2. In Figure 2, SARS-CoV-2 is referred to by its previous provisional name, 2019-nCoV (2019 novel coronavirus).
The sequences obtained are shown below:

**MERS** CoV KJ477103.2 (SEQ ID NO:27)

AGGAGTGTTCTGAAGTAGAGGCTTCAGATTTAGAAGAAGGTGAATCAGAGTGCATTTC
TGAGACTTCAACTGAACAAGTTGACGTTTCTCATGAGACTTCT

hCoV **OC43** KU131570 (SEQ ID NO:28)

ATGATGGTCCAAGTGTGGAGACATCTGATTCACAAGTTGAAGAAGATGTAGAAATGTC
GGATTTTGTTGATCTTGAATCTGTGATTCAGGATTATGAAAATGTTTGTTTTGA

hCoV-**NL63** AY567487 (SEQ ID NO:29)

ACACTTGTTTTGGTGTTAGTAAACCTAATGCCATTGATGTTGAACATTTAGAGCTTAAA
GAAACTGTTTTTGTTGAACCTAAGGATGGTGGTCAATTTTTTGTTTCTGGTGATTATCTT
TGGTATGT

hCoV **229E** AF304460 (SEQ ID NO:30)

ACATGCCAATTGCAGACCCTACACATTTTGACATTGAAGAAGTTGAACTTTTAGATGCA
GAGTTTGTAGAACCAGGCTGTGGTGGTATTTTGGCAGTAATAGATGAGCACGTCTTTT
ATAAGAAGG

hCoV-**HKU1** MH940245.1 (SEQ ID NO:31)

ACTGGTGACAATGACGATGAAGATGTTGTTACTGGTGACAATGACGATGAAGATGTTG
TTACTGGTGACAATGACGATGAAGATGTTGTTACTGGTGACAATGACGATGAAGAGAT
TGTTACTGGTGACAATGATGACCAAATTGTTGTTACTGGTGATGATGTAGATGATATTG
AAAGTGTCTATGATTTTG

**SARS** CoV GZ02 AY390556.1 China (SEQ ID NO:32)

AAGAAGAAGAGGAAGACTGGCTGGATGATACTACTGAGCAATCAGAGATTGAGCCAG
AACCAGAACCTACACCTGAAGAACCAGTTAATCAGTTTACTGGTTATTTAAAACTTAC

**SARS** CoV ZS-B AY394996.1 China (SEQ ID NO:33)

AAGAAGAAGAGGAAGACTGGCTGGATGATACTACTGAGCAATCAGAGATTGAGCCAG
AACCAGAACCTACACCTGAAGAACCAGTTAATCAGTTTACTGGTTATTTAAAACTTAC

**Bat SARS-like** CoV Rs4231 KY417146.1 China (SEQ ID NO:34)

AAGAAGAAGAGGACTGGCTTGGTGATGCTACTGAATTATCGGAGCATGAACCAGAAC
CAGAACTAACACCTGAAGAACCAGTTAACCAGTTTACTGGTTATTTAAAACTTAC

**2019-nCoV** MN988713 Atlanta USA (SEQ ID NO:35)

AAGAGCAAGAAGAAGATTGGTTAGATGATGATAGTCAACAAACTGTTGGTCAACAAGA
CGGCAGTGAGGACAATCAGACAACTACTATTCAAACAATTGTTGAGGTTCAACCTCAA
TTAGAGATGGAACTTACACCAGTTGTTCAGACTATTGAAGTGAATAGTTTTAGTGGTTA
TTTAAAACTTAC

**2019-nCoV** MN988669.1 WuhanUniversity China (SEQ ID NO:36)

AAGAGCAAGAAGAAGATTGGTTAGATGATGATAGTCAACAAACTGTTGGTCAACAAGA
CGGCAGTGAGGACAATCAGACAACTACTATTCAAACAATTGTTGAGGTTCAACCTCAA
TTAGAGATGGAACTTACACCAGTTGTTCAGACTATTGAAGTGAATAGTTTTAGTGGTTA
TTTAAAACTTAC

**2019-nCoV** MN908947.3 PublicHealth China (SEQ ID NO:37)

AAGAGCAAGAAGAAGATTGGTTAGATGATGATAGTCAACAAACTGTTGGTCAACAAGA
CGGCAGTGAGGACAATCAGACAACTACTATTCAAACAATTGTTGAGGTTCAACCTCAA
TTAGAGATGGAACTTACACCAGTTGTTCAGACTATTGAAGTGAATAGTTTTAGTGGTTA
TTTAAAACTTAC

**Primer and Probe Sequences**

Primer and probe sequences for specific amplification and detection of SARS-CoV-2 Orf1ab nucleic acid sequence were designed based on the multiple sequence alignment, and are set out below.

Forward primers

- 2019-nCoV-ARP1.1F
  CTGTTGGTCAACAAGACGGCA (SEQ ID NO:1)
- 2019-nCoV-ARP1.2F
  GTCAACAAACTGTTGGTCAA (SEQ ID NO:2)
- 2019-nCoV-ARP1.3F
  GTCAACAAACTGTTGGTCAACA (SEQ ID NO:3)

- SA nCoV F2
  CATTACAGGTGGTGTTGTTCAGTT (SEQ ID NO:4)

Reverse primers

- 2019-nCoV-ARP1.1R
  CAATAGTCTGAACAACTGGTGT (SEQ ID NO:5)
- 2019-nCoV-ARP1.2R
  CTGGTGTAAGTTCCATCTCT (SEQ ID NO:6)
- SA nCoV R2
  AGGTGACAATTTGTCCACCGAC (SEQ ID NO:7)

Capture probes

- 2019-nCoV-AR-CP1.1
  GGCAGTGAGGACAATCAGCAACTAC (SEQ ID NO:8)
- 2019-nCoV-AR-CP1.2
  GAGGACAATCAGACAACTACTATTC (SEQ ID NO:9)
- SA nCoV CP2
  ACCCGTCCTTGATTGGCTTG (SEQ ID NO:10)

Detector probes

- 2019-nCoV-AR-DP1.1
  CAAACAATTGTTGAGGTTCAACCTC (SEQ ID NO:11)
- 2019-nCoV-AR-DP1.2
  GAGGTTCAACCTCAATTAGAGATGG (SEQ ID NO:12)
- SA nCoV DP2
  GGAAGGTGTAGAGTTTCTTAGAGAC (SEQ ID NO:13)

**Example 2**

**Design of primers and probes from coronavirus nucleocapsid gene sequence alignments**

Nucleic acid sequences of the gene encoding the nucleocapsid (N) protein for all seven human coronaviruses (229E, SARS, HKU1, MERS, OC43, NL63, and SARS-CoV-2) were obtained, including three different SARS-CoV-2 sequences, and a bat SARS-like coronavirus sequence. These sequences were aligned using the CLUSTAL 0(1.2.4) multiple sequence alignment software. The multiple sequence alignment is shown in Figure 3. In Figure 3, SARS-CoV-2 is referred to by its previous provisional name, 2019-nCoV (2019 novel coronavirus).

The sequences obtained are shown below:

MERS CoV KJ477103.2 (SEQ ID NO:38)

AGAACTGGATCATGGTGGTCATTCAATCCTGAGACTAATTGCCTTTTGAACGTTCCATT
TGGTGGTACAACTGTCGTACGTCCACTCGTAGAGGACTCTACCAGTGTAACTGCTGTT
GTAACCAATGGCCACCTCAAAATGGCTGGCATGCATTTCGGTGCTTGTGACTACGACA
GACTTCCTAATGAAGTCACCGTGGCCAAACCCAATGTGCTGATTGCTTTAAAAATGGT
GAAGCGGCAAAGCTACGGAACTAATTCCGGCGTTGCCATTTACCATAGATATAAGGCA
GGTAATTACAGGAGTCCGCCTATTACGGCGGATATTGAACTTGCATTGCTTCGAGCTT
AGGCTCTTTAGTAAGAGTATCTTAATTGATTTTAACGAATCTCAATTTCATTGTTATGGC
ATCCCCTGCTGCACCTCGTGCTGTTTC

hCoV OC43 KU131570 (SEQ ID NO:39)

```
AGAACCGGCAGTTTTTGGAGTTTCAACCCAGAAACAAACAACTTGATGTGTATAGATAT
GAAAGGAACAATGTTTGTTAGGCCAATAATTGAGGACTACCATACCCTGACGGTCACA
ATAATACGCGGTCATCTTTACATTCAAGGTATAAAACTAGGTACTGGCTATTCTTTGGC
AGATTTGCCAGCTTATATGACTGTTGCTAAGGTCACATACCTGTGCACATATAAGCGTG
GTTTTCTTGACAAGATAAGCGATACTAGTGGTTTTGCTGTTTATGTTAAGTCCAAAGTC
GGTAATTACCGACTGCCATCAACCCAAAAGGGTTCAGGCATGGACACCGCATTGTTGA
GAAATAATATCTAAATTTTAAGGATGTCTTTTACTCCTGGTAAGCAATCCAGTAGTAGA
GCGTCCTCTGGAAATCGTTCTGGTAATGGCATCCTTAAGTG
```

hCoV-NL63 AY567487 (SEQ ID NO:40)

```
CGTGTTAAAACTTTTTGGGCTTTTAATCCTGAAACTAATGCAATCATCTCTCTCCAGGTT
TACGGACATAATTATTACTTACCGGTGATGGCTGCACCTACAGGTGTTACATTAACACT
TCTTAGTGGTGTACTTCTTGTTGATGGCCATAAGATTGCTACTCGTGTTCAAGTGGGTC
AGTTGCCTAAATATGTAATAGTTGCTACGCCTAGTACCACAATTGTTTGTGACCGTGTT
GGTCGCTCTGTTAATGAAACAAGCCAGACTGGTTGGGCATTCTACGTCCGTGCTAAAC
ATGGTGATTTTTCTGGTGTTGCCTCTCAGGAGGGTGTTTTGTCAGAAAGAGAGAAGTT
GCTTCATTTAATCTAAACTAAACAAAATGGCTAGTGTAAATTGGGCCGATGA--
CAGAGCTGCTAGGAAGAAATTTCCTCCTCCTTC
```

hCoV 229E AF304460 (SEQ ID NO:41)

```
CGTGCTCGAACTTTTTGGGCATGGAATCCTGAGGTTAATGCAATCACTGTCACAACCG
TGTTGGGACAGACATACTATCAACCCATTCAACAAGCTCCAACAGGCATTACTGTGAC
CTTGCTGAGCGGCGTGCTTTACGTTGACGGACATAGATTGGCTTCAGGTGTTCAGGTT
CATAACCTACCTGAATACATGACAGTTGCCGTGCCGAGCACTACTATAATTTATAGTAG
AGTCGGAAGGTCCGTAAATTCACAAAATAGCACAGGCTGGGTTTTCTACGTACGAGTA
AAACACGGTGATTTTTCTGCAGTGAGCTCTCCCATGAGCAACATGACAGAAAACGAAA
GATTGCTTCATTTTTTCTAAACTGAACGAAAAGATGGCTACAGTCAAATGGGCTGATGC
ATCTGAACCACAACGTGGTCGTCAGGGTAGAATACCTTATTC
```

hCoV-HKU1 MH940245.1 (SEQ ID NO:42)

```
AGAACTGGCAGTTGGTGGAGTTTTAACCCAGAGACTAATAATCTTATGTGTATTGATAT
GAAAGGTAAGATGTATGTTAGGCCAGTTATTGAGGACTATCATACATTAACGGCTACT
GTTATCCGTGGTCATCTTTATATACAGGGTGTTAAACTTGGCACTGGTTACACGCTTGC
CGATTTGCCTGTTTATGTTACTGTAGCTAAGGTGCAAGTCCTCTGTACTTATAAACGTG
CCTTTTTAGATAAGTTAGATGTTAATAGTGGTTTTGCTGTTTTTGTTAAGTCTAAAGTTG
GTAACTATCGTTTACCTTCTAGTAAATCTAGTGGTATGGATACTGCCTTGTTGAGAGCT
TAAATCTAAACTATTAGGATGTCTTATACTCCCGGTCATCATGCTGGAAGTAGAAGCTC
CTCTGGAAATCGTTCAGGAATCCTCAAGAA
```

SARS CoV GZ02 AY390556.1 China (SEQ ID NO:43)

```
GCAGTTGCATACGCACTGTAGTACAGCGCTGTGCATCTAATAAACCTCATGTGCTTGA
AGATCCTTGTCCTACTGGTTACCAACCTGAATGGAATATAAGGTACAACACTAGGGGT
AATACTTATAGCACTGCTTGGCTTTGTGCTCTAGGAAAGGTTTTACCTTTTCATAGATG
GCACACTATGGTTCAAACATGCACACCTAATGTTACTATCAACTGTCAAGATCCAGCTG
GTGGTGCGCTTATAGCTAGGTGTTGGTACCTTCATGAAGGTCACCAAACTGCTGCATT
TAGAGACGTATTTGTTGTTTTAAATAAACGAACAAATTAAAATGTCTGATAATGGACCC
CAATCAAACCAACGTAGTGCCCCCCGCATTACATTTGGTGGACCCACAGATTC
```

SARS CoV ZS-B AY394996.1 China (SEQ ID NO:44)

GCAGTTGCATACGCACTGTAGTACAGCGCTGTGCATCTAATATAGCACTGCTTGGCTT
TGTGCTCTAGGAAAGGTTTTACCTTTTCATAGATGGCACACTATGGTTCAAACATGCAC
ACCTAATGTTACTATCAACTGTCAAGATCCAGCTGGTGGTGCGCTTATAGCTAGGTGT
TGGTACCTTCATGAAGGTCACCAAACTGCTGCATTTAGAGACGTACTTGTTGTTTTAAA

TAAACGAACAAATTAAAATGTCTGATAATGGACCCCAATCAAACCAACGTAGTGCCCC
CCGCATTACATTTGGTGGACCCACAGATTC

Bat SARS-like CoV Rs4231 KY417146.1 China (SEQ ID NO:45)

GTGTATTGCATGCATAAAGAATGCAGTATACAAGAATGTTGTGAAAATCAACCATTCCA
ACCTGAAGACCCATGTCCAATACATTATTATTCGGACTGGTTTGTAAAAATTGGACCTC
GCAAGTCTGCTCGCCTAGTACAACTTTGTGCTGGTGAATATGGACACAGAGTTCCAAT
ACATTATGAAATGTTTGGCAATTATACTATTTCATGTGAACCACTTGAAATAAATTGTCA
AAACCCACCAGTTGGAAGTCTCATTGTACGTTGTTCATATGATGTTGACTTTATGGAGT
ATCACGACGTTCGTGTTGTTCTAGATTTCATCTAAACGAACAAACTAAAATGTCTGATA
ATGGACCCCAACCAAATCAGCGTAGTGCCCCCCGCATTACATTTGGTGGACCCACAG
ATTC

2019-nCoV MN988713 Atlanta USA (SEQ ID NO:46)

GCTGCATTTCACCAAGAATGTAGTTTACAGTCATGTACTCAACATCAACCATATGTAGT
TGATGACCCGTGTCCTATTCACTTCTATTCTAAATGGTATATTAGAGTAGGAGCTAGAA
AATCAGCACCTTTAATTGAATTGTGCSTGGATGAGGCTGGTTCTAAATCACCCATTCAG
TACATCGATATCGGTAATTATACAGTTTCCTGTTYACCTTTTACAATTAATTGCCAGGAA
CCTAAATTGGGTAGTCTTGTAGTGCGTTGTTCGTTCTATGAAGACTTTTTAGAGTATCA
TGACGTTCGTGTTGTTTTAGATTTCATCTAAACGAACAAACTAAAATGTCTGATAATGG
ACCCCAAAATCAGCGAAATGCACCCCGCATTACGTTTGGTGGACCCTCAGATTC

2019-nCoV MN988669.1 WuhanUniversity China (SEQ ID NO:47)

GCTGCATTTCACCAAGAATGTAGTTTACAGTCATGTACTCAACATCAACCATATGTAGT
TGATGACCCGTGTCCTATTCACTTCTATTCTAAATGGTATATTAGAGTAGGAGCTAGAA
AATCAGCACCTTTAATTGAATTGTGCSTGGATGAGGCTGGTTCTAAATCACCCATTCAG
TACATCGATATCGGTAATTATACAGTTTCCTGTTYACCTTTTACAATTAATTGCCAGGAA
CCTAAATTGGGTAGTCTTGTAGTGCGTTGTTCGTTCTATGAAGACTTTTTAGAGTATCA
TGACGTTCGTGTTGTTTTAGATTTCATCTAAACGAACAAACTAAAATGTCTGATAATGG
ACCCCAAAATCAGCGAAATGCACCCCGCATTACGTTTGGTGGACCCTCAGATTC

2019-nCoV MN908947.3 PublicHealth China (SEQ ID NO:48)

GCTGCATTTCACCAAGAATGTAGTTTACAGTCATGTACTCAACATCAACCATATGTAGT
TGATGACCCGTGTCCTATTCACTTCTATTCTAAATGGTATATTAGAGTAGGAGCTAGAA
AATCAGCACCTTTAATTGAATTGTGCSTGGATGAGGCTGGTTCTAAATCACCCATTCAG
TACATCGATATCGGTAATTATACAGTTTCCTGTTYACCTTTTACAATTAATTGCCAGGAA
CCTAAATTGGGTAGTCTTGTAGTGCGTTGTTCGTTCTATGAAGACTTTTTAGAGTATCA
TGACGTTCGTGTTGTTTTAGATTTCATCTAAACGAACAAACTAAAATGTCTGATAATGG
ACCCCAAAATCAGCGAAATGCACCCCGCATTACGTTTGGTGGACCCTCAGATTC

**Primer and Probe Sequences**

Primer and probe sequences for specific amplification and detection of SARS-CoV-2 nucleocapsid nucleic acid sequence were designed based on the multiple sequence alignment, and are set out below.

Forward Primer

- SA-nCoV-F2.3
  TAGTTGATGACCCGTGTCCT (SEQ ID NO:14)

Reverse Primers

- SA-nCoV-R2.3
  TGGGGTCCATTATCAGACAT (SEQ ID NO:15)
- SA-nCoV-R2.4
  CAACACGAACGTCATGATAC (SEQ ID NO:16)
- SA-nCoV-R2.5
  CATAGAACGAACAACGCAC (SEQ ID NO:17)

Capture Probe

- SA-nCoV-CP2.3
  CTGGTTCTAAATCACCCATTCA (SEQ ID NO:18)

Detector Probe

- SA-nCoV-DP2.3
  GAACCTAAATTGGGTAGTCTTGTAG (SEQ ID NO:19)
- SA-nCoV-DP2.4
  GGAACCTAAATTGGGTAGTCTTG (SEQ ID NO:20)

## Example 3

### Point-of-care (POC) nucleic acid test for detecting SARS-CoV-2

SARS-CoV-2 target RNA was extracted, reverse transcribed, and amplified by isothermal nucleic acid amplification, and the amplification products were detected by rapid visual detection with a dipstick, using a simple amplification-based assay (SAMBA) method similar to the method described in Lee et al., Journal of Infectious Diseases 2010;201(S1):S65-S71.

Briefly, a reverse nucleic acid amplification primer comprises nucleic acid sequence complementary to a portion of SARS-CoV-2 target RNA so that the primer can hybridise specifically to the target RNA, and a single stranded-version of a promoter sequence for a DNA-dependent RNA polymerase at its 5'-end. The reverse primer hybridizes to the RNA target. An RNA-dependent DNA polymerase extends the reverse primer to synthesise a complementary DNA (cDNA) copy of the RNA target. A DNA/RNA duplex-specific ribonuclease digests the RNA of the RNA-cDNA hybrid. A forward nucleic acid amplification primer comprises nucleic acid sequence complementary to a portion of the cDNA. The forward primer hybridizes to the cDNA downstream of the part of the cDNA formed by the reverse primer. The forward primer is extended by a DNA-dependent DNA polymerase to produce a second DNA strand which extends through the DNA-dependent RNA polymerase promoter sequence at one end (thereby forming a double stranded promoter). This promoter is used by a DNA-dependent RNA polymerase to synthesise a large number of RNAs complementary to the original target sequence. These RNA products then function as templates for a cyclic phase of the reaction, but with the primer hybridising steps reversed, i.e., the forward primer followed by the reverse primer.

The following primer/probe sequences were used for isothermal amplification, capture and detection of SARS-CoV-2 nucleic acid:

ORF1ab

- **forward primer** 2019-CoV-ARP1.1F: CTGTTGGTCAACAAGACGGCA (SEQ ID NO:1);
- **reverse primer** 2019-CoV-ARP1.1R: CAATAGTCTGAACAACTGGTGT (SEQ ID NO:5), with T7 promoter sequence at its 5'-end: aattctaatacgactcactatagggagaagg (SEQ ID NO:21);
- **capture probe** 2019-CoV-AR-CP1.2: GAGGACAATCAGACAACTACTATTC (SEQ ID NO:9);
- **detector probe** 2019-CoV-AR-DP1.2: GAGGTTCAACCTCAATTAGAGATGG (SEQ ID NO:12).

Nucleocapsid Protein (NP)

- **forward primer** SA_nCoV_F2.3: TAGTTGATGACCCGTGTCCT (SEQ ID NO:14)
- **reverse primer** SA_nCoV_R2.4: CAACACGAACGTCATGATAC (SEQ ID NO:16), with T7 promoter sequence at its 5'-end: aattctaatacgactcactataggggaaaag (SEQ ID NO:22);
- **capture probe** SA_nCoV_CP2.3: CTGGTTCTAAATCACCCATTCA (SEQ ID NO:18)
- **detector probe** SA_nCoV_DP2.3: GAACCTAAATTGGGTAGTCTTGTAG (SEQ ID NO:19)

Isothermal amplification, capture and detection was carried out for 1000, 100, 10, and 0 SARS-CoV-2 RNA target copies per test to determine the sensitivity of the test.

The chromatographic strip comprises a capture zone with capture probe 2019-CoV-AR-CP1.2 (GAGGACAATCAGACAACTACTATTC; SEQ ID NO:9) (for capturing amplified nucleic acid of the ORF1ab region) immobilised in a first, upper line, and capture probe SA_nCoV_CP2.3 (CTGGTTCTAAATCACCCATTCA; SEQ ID NO:18) (for capturing amplified nucleic acid of Nucleocapsid region) immobilised in a separate second, lower line. A third, uppermost line comprises a capture probe for hybridising to amplified nucleic acid of an internal control.

Isothermal amplification, capture and detection was also carried out for SARS and MERS target RNA (at >100,000 copies per test).

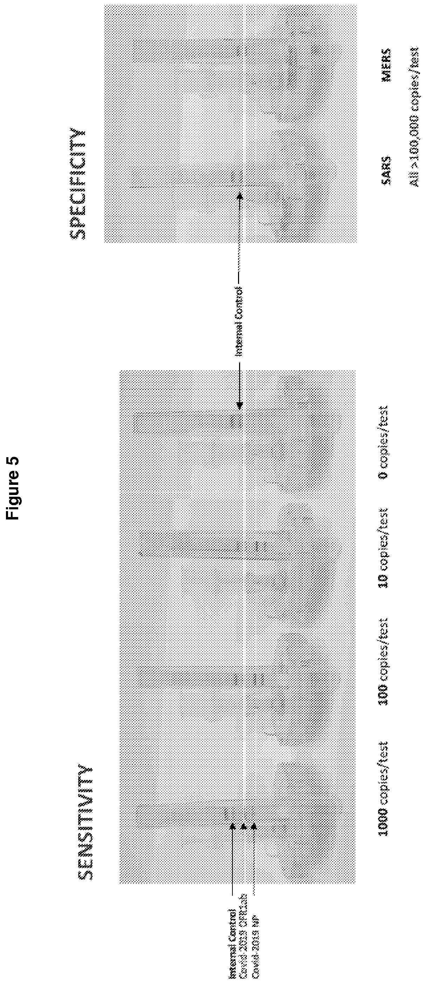

The results are recorded in Figure 5. The results show that SARS-CoV-2 target RNA was efficiently detected down to 10 copies/per test, and that the test results are highly specific for SARS-CoV-2 target RNA over SARS and MERS target RNA.

**Example 4**

**Sensitivity and Specificity of POC nucleic acid test for detecting SARS-CoV-2 in clinical samples**

This example describes an assessment of the clinical sensitivity and specificity of a SARS-CoV-2 Test according to an embodiment of the invention on 102 blinded frozen clinical samples collected from symptomatic individuals.

The samples were tested independently according to a real-time RT-PCR Reference method described in Corman et al., "Detection of 2019 novel coronavirus (2019-nCoV) by real-time RT-PCR", Euro Surveill. 2020;25(3):pii=2000045. The results obtained by this method were compared with the results obtained using a method according to an embodiment of the invention similar to the method described in Example 3, carried out using an automated sample processing system similar to that described in WO 2014/140640. The method is referred to herein as the SAMBA II SARS-CoV-2 Test. The test is a fully automated isothermal nucleic acid amplification test which is run using the CE-marked SAMBA II instrument system consisting of the SAMBA II Assay Module and Tablet Module.

102 nose/throat swab samples were collected from symptomatic individuals in Viral Transport Medium (VTM) and diluted 1:2 with buffer before supply for testing. The samples were provided blinded and coded with no patient information available to the tester.

a) Testing criteria

| Samples positive by Reference method | SAMBA IC signal ≥0.5, Orf >0 N ≥0 |
|---|---|
| Samples negative by Reference method | SAMBA IC signal ≥3, Orf =0 N =0 |

b) Clinical performance versus Reference Method (qPCR):

% Sensitivity: ≥ 98%
% Specificity: 100%

Results:

| Sample | Test date | IC | ORF | N | SAMBA result | Data (reference method) (Ct Value) |
|--------|-----------|-----|-----|-----|--------------|------------------------------------|
| 1 | 27/03/2020 | 4.5 | 5.0 | 4.5 | POSITIVE | 19.86 |
| 2 | 27/03/2020 | 5.0 | 4.5 | 5.0 | POSITIVE | 19.07 |
| 3 | 27/03/2020 | 4.5 | 4.5 | 0.5 | POSITIVE | 27.99 |
| 4 | 27/03/2020 | 5.0 | 4.0 | 0.0 | POSITIVE | 29.31 |
| 5 | 27/03/2020 | 5.0 | 1.0 | 0.0 | POSITIVE | 31.81 |
| 6 | 27/03/2020 | 4.5 | 5.0 | 0.0 | POSITIVE | 30.44 |
| 7 | 27/03/2020 | 4.0 | 5.0 | 3.0 | POSITIVE | 25.12 |
| 8 | 27/03/2020 | 5.0 | 0.0 | 0.0 | NEGATIVE | NEGATIVE |
| 9 | 27/03/2020 | 4.5 | 5.0 | 5.0 | POSITIVE | 20.4 |
| 10 | 27/03/2020 | 4.5 | 3.5 | 0.0 | POSITIVE | 25.19 |
| 11 | 27/03/2020 | 5.0 | 0.0 | 0.0 | NEGATIVE | NEGATIVE |
| 12 | 27/03/2020 | 4.5 | 5.0 | 5.0 | POSITIVE | 31 |
| 13 | 27/03/2020 | 4.0 | 5.0 | 0.0 | POSITIVE | 27.35 |
| 14 | 27/03/2020 | 5.0 | 3.5 | 0.0 | POSITIVE | 30.6 |
| 15 | 27/03/2020 | 4.0 | 5.0 | 3.0 | POSITIVE | 25.76 |
| 16 | 27/03/2020 | 5.0 | 2.5 | 0.5 | POSITIVE | 30.49 |
| 17 | 27/03/2020 | 4.0 | 5.0 | 4.5 | POSITIVE | 25.19 |
| 18 | 27/03/2020 | 4.0 | 5.0 | 4.5 | POSITIVE | 19.82 |
| 19 | 27/03/2020 | 4.5 | 5.0 | 4.5 | POSITIVE | 21.22 |
| 20 | 27/03/2020 | 4.5 | 5.0 | 0.5 | POSITIVE | 19.33 |
| 21 | 27/03/2020 | 4.5 | 5.0 | 4.5 | POSITIVE | 18.82 |
| 22 | 27/03/2020 | 4.5 | 5.0 | 0.5 | POSITIVE | 26.57 |
| 23 | 27/03/2020 | 5.0 | 0.0 | 0.0 | NEGATIVE | NEGATIVE |
| 24 | 27/03/2020 | 5.0 | 1.0 | 0.5 | POSITIVE | 26.53 |
| 25 | 27/03/2020 | 5.0 | 0.0 | 0.0 | NEGATIVE | 31.18 |

| Sample | Test date | IC | ORF | N | SAMBA result | Data (reference method) (Ct Value) |
|---|---|---|---|---|---|---|
| 26 | 27/03/2020 | 4.5 | 5.0 | 1.5 | POSITIVE | 22.81 |
| 27 | 27/03/2020 | 3.0 | 3.0 | 2.5 | POSITIVE | 20.38 |
| 28 | 27/03/2020 | 4.5 | 5.0 | 4.5 | POSITIVE | 20.43 |
| 29 | 27/03/2020 | 4.5 | 5.0 | 4.0 | POSITIVE | 20.62 |
| 30 | 27/03/2020 | 4.0 | 3.0 | 3.0 | POSITIVE | 14.07 |
| 31 | 27/03/2020 | 4.5 | 4.5 | 4.5 | POSITIVE | 22.58 |
| 32 | 27/03/2020 | 5.0 | 4.5 | 0.5 | POSITIVE | 26.86 |
| 33 | 27/03/2020 | 4.5 | 5.0 | 4.5 | POSITIVE | 21.64 |
| 34 | 27/03/2020 | 4.5 | 5.0 | 3.5 | POSITIVE | 18.56 |
| 35 | 27/03/2020 | 4.5 | 3.5 | 0 | POSITIVE | 24.92 |
| 36 | 27/03/2020 | 4.5 | 5 | 5 | POSITIVE | 19.12 |
| 37 | 27/03/2020 | 4.5 | 5 | 4.5 | POSITIVE | 19.74 |
| 38 | 27/03/2020 | 4.5 | 5 | 2 | POSITIVE | 24.52 |
| 39 | 27/03/2020 | 5 | 4 | 4.5 | POSITIVE | 16.75 |
| 40 | 27/03/2020 | 4.5 | 5 | 3 | POSITIVE | 22.73 |
| 41 | 27/03/2020 | 4.5 | 4.5 | 4.5 | POSITIVE | 17 |
| 42 | 27/03/2020 | 4 | 5 | 5 | POSITIVE | 21.36 |
| 43 | 27/03/2020 | 4 | 5 | 3.5 | POSITIVE | 15 |
| 44 | 27/03/2020 | 4 | 4.5 | 4 | POSITIVE | 22.54 |
| 45 | 27/03/2020 | 5 | 4.5 | 4.5 | POSITIVE | 23.4 |
| 46 | 27/03/2020 | 4.5 | 4.5 | 5 | POSITIVE | 19.44 |
| 47 | 27/03/2020 | 4.5 | 4 | 4.5 | POSITIVE | 19.05 |
| 48 | 27/03/2020 | 4 | 4.5 | 4.5 | POSITIVE | 20.83 |
| 49 | 27/03/2020 | 4.5 | 5 | 3.5 | POSITIVE | 18.9 |
| 50 | 27/03/2020 | 4.5 | 4.5 | 4.5 | POSITIVE | 20.34 |
| 51 | 27/03/2020 | 4.5 | 5 | 4.5 | POSITIVE | 20.57 |
| 52 | 27/03/2020 | 4.5 | 5 | 4 | POSITIVE | 21.65 |
| 53 | 29/03/2020 | 5 | 5 | 5 | POSITIVE | 20 |
| 54 | 29/03/2020 | 5 | 5 | 3 | POSITIVE | 28 |
| 55 | 29/03/2020 | 5 | 5 | 5 | POSITIVE | 27 |
| 56 | 29/03/2020 | 0 | 0 | 0 | INVALID | |
| 56 (1:4) | 29/03/2020 | 5 | 2 | 0 | POSITIVE | 22 |
| 57 | 29/03/2020 | 4.5 | 5 | 5 | POSITIVE | 24 |
| 58 | 29/03/2020 | 4.5 | 5 | 5 | POSITIVE | 24 |
| 59 | 29/03/2020 | 4.5 | 5 | 3 | POSITIVE | 30 |
| 60 | 29/03/2020 | 5 | 4.5 | 0 | POSITIVE | 26 |
| 61 | 29/03/2020 | 4.5 | 5 | 5 | POSITIVE | 19 |
| 62 | 29/03/2020 | 5 | 3 | 0 | POSITIVE | 27 |

| Sample | Test date | IC | ORF | N | SAMBA result | Data (reference method) (Ct Value) |
|---|---|---|---|---|---|---|
| 63 | 29/03/2020 | 4.5 | 5 | 3.5 | POSITIVE | 20 |
| 64 | 29/03/2020 | 4.5 | 4.5 | 3 | POSITIVE | 28 |
| 65 | 29/03/2020 | 5 | 4.5 | 5 | POSITIVE | 18 |
| 66 | 29/03/2020 | 4.5 | 5 | 3 | POSITIVE | 26 |
| 67 | 29/03/2020 | 5 | 4.5 | 5 | POSITIVE | 22 |
| 68 | 29/03/2020 | 4.5 | 5 | 0 | POSITIVE | 29 |
| 69 | 29/03/2020 | 5 | 1.5 | 0.5 | POSITIVE | 28 |
| 70 | 29/03/2020 | 4.5 | 5 | 5 | POSITIVE | 17 |
| 71 | 29/03/2020 | 5 | 5 | 5 | POSITIVE | 22 |
| 72 | 29/03/2020 | 5 | 5 | 5 | POSITIVE | 23 |
| 73 | 29/03/2020 | 5 | 4.5 | 5 | POSITIVE | 16 |
| 74 | 29/03/2020 | 5 | 4.5 | 5 | POSITIVE | 20 |
| 75 | 29/03/2020 | 4 | 5 | 3.5 | POSITIVE | 25 |
| 76 | 29/03/2020 | 4.5 | 5 | 5 | POSITIVE | 25 |
| 77 | 29/03/2020 | 4.5 | 5 | 4.5 | POSITIVE | 22 |
| 78 | 29/03/2020 | 5 | 4.5 | 5 | POSITIVE | 21 |
| 79 | 29/03/2020 | 5 | 0 | 0 | NEGATIVE | NEGATIVE |
| 80 | 29/03/2020 | 5 | 0 | 0 | NEGATIVE | NEGATIVE |
| 81 | 29/03/2020 | 4.5 | 0 | 0 | NEGATIVE | NEGATIVE |
| 82 | 29/03/2020 | 5 | 0 | 0 | NEGATIVE | NEGATIVE |
| 83 | 29/03/2020 | 4.5 | 0 | 0 | NEGATIVE | NEGATIVE |
| 84 | 29/03/2020 | 5 | 0 | 0 | NEGATIVE | NEGATIVE |
| 85 | 29/03/2020 | 5 | 0 | 0 | NEGATIVE | NEGATIVE |
| 86 | 29/03/2020 | 0 | 0 | 0 | INVALID | |
| 86 (1:10) | 29/03/2020 | 5 | 0 | 0 | NEGATIVE | NEGATIVE |
| 87 | 29/03/2020 | 5 | 0 | 0 | NEGATIVE | NEGATIVE |
| 88 | 29/03/2020 | 5 | 0 | 0 | NEGATIVE | NEGATIVE |
| 89 | 29/03/2020 | 5 | 0 | 0 | NEGATIVE | NEGATIVE |
| 90 | 29/03/2020 | 5 | 0 | 0 | NEGATIVE | NEGATIVE |
| 91 | 29/03/2020 | 5 | 0 | 0 | NEGATIVE | NEGATIVE |
| 92 | 29/03/2020 | 5 | 0 | 0 | NEGATIVE | NEGATIVE |
| 93 | 29/03/2020 | 5 | 0 | 0 | NEGATIVE | NEGATIVE |
| 94 | 29/03/2020 | 0 | 0 | 0 | INVALID | |
| 94 (1:4) | 29/03/2020 | 5 | 0 | 0 | NEGATIVE | NEGATIVE |
| 95 | 29/03/2020 | 4 | 0 | 0 | NEGATIVE | NEGATIVE |
| 96 | 29/03/2020 | 4.5 | 5 | 1 | POSITIVE | 25 |
| 97 | 29/03/2020 | 5 | 0 | 0 | NEGATIVE | NEGATIVE |
| 98 | 29/03/2020 | 5 | 0 | 0 | NEGATIVE | NEGATIVE |

| Sample | Test date | IC | ORF | N | SAMBA result | Data (reference method) (Ct Value) |
|---|---|---|---|---|---|---|
| 99 | 29/03/2020 | 5 | 0 | 0 | NEGATIVE | NEGATIVE |
| 100 | 29/03/2020 | 4 | 0 | 0 | NEGATIVE | NEGATIVE |
| 101 | 29/03/2020 | 4 | 0 | 0 | NEGATIVE | NEGATIVE |
| 102 | 29/03/2020 | 4.5 | 0 | 0 | NEGATIVE | NEGATIVE |

In total 102 samples were tested by SAMBA, of which 74 were positive, 25 were negative and 3 were invalid by SAMBA. The invalid samples were further diluted in Buffer 1:4 and 1:10 and retested, which resulted in 1 positive and 2 negatives.

Compared with the reference laboratory test, there were 75 concordant positives, 26 concordant negatives and one false-negative by SAMBA (see table below). Therefore, the SAMBA II SARS-CoV-2 Test has a sensitivity of 98.68% (95% CI 92.89-99.97%), specificity of 100% (95% CI 87.23-100%), PPV of 100% and NPV of 96.43% (79.39-99.47%) when compared to the reference method. The one discrepant sample gave a high Ct value on the reference method (>31) and it was further diluted (1:2) for SAMBA testing as the UTM was found to interfere with the assay, which may explain the false-negative result.

Clinical performance in 102 blinded surplus clinical samples compared to reference method:

| | | Reference method | |
|---|---|---|---|
| | | POSITIVE | NEGATIVE |
| SAMBA SARS-CoV-2 Test | POSITIVE | 75 | 0 |
| | NEGATIVE | 1 | 26 |

Conclusions:

The sensitivity and specificity of the SAMBA II SARS-CoV-2 Test was **98.68%** (95% CI 92.89-99.97%) and specificity of **100%** (95% CI 87.23-100%) respectively when tested 1:2 in buffer compared to the Reference method.

**Claims**

1. A method for determining whether a sample includes severe acute respiratory syndrome coronavirus 2 (SARS-CoV-2) nucleic acid, which comprises amplifying nucleic acid of the sample, or amplifying nucleic acid derived from nucleic acid of the sample, by an isothermal amplification reaction using a forward nucleic acid amplification primer and a reverse nucleic acid amplification primer, wherein each nucleic acid amplification primer hybridises specifically to nucleic acid sequence that is conserved in SARS-CoV-2 nucleic acid, but not in other human coronavirus nucleic acid, or the complement thereof, wherein the nucleic acid sequence that is conserved in SARS-CoV-2 nucleic acid is nucleic acid sequence that is conserved in the ORF1ab gene or the nucleocapsid gene of SARS-CoV-2, wherein:

the forward nucleic acid primer hybridizes specifically to nucleic acid sequence that is conserved in the ORF1ab gene of SARS-CoV-2, or the complement thereof, wherein the forward nucleic acid primer comprises a nucleic acid sequence of CTGTTGGTCAACAAGACGGCA (SEQ ID NO:1), or the complement thereof, or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, or the complement thereof, and the reverse nucleic acid primer hybridizes specifically to nucleic acid sequence that is conserved in the ORF1ab gene of SARS-CoV-2, or the complement thereof, wherein the reverse nucleic acid primer comprises a nucleic acid sequence CAATAGTCTGAACAACTGGTGT (SEQ ID NO:5), or the complement thereof, or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, or the complement thereof; and/or the forward nucleic acid primer hybridizes specifically to nucleic acid sequence that is conserved in the nucleocapsid gene of SARS-CoV-2, or the complement thereof, wherein the forward nucleic acid primer comprises a nucleic acid sequence of: TAGTTGATGACCCGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14, or the complement thereof and the reverse nucleic acid primer hybridizes specifically to nucleic acid sequence that is conserved in the nucleocapsid gene of SARS-CoV-2, or the complement thereof, wherein the reverse nucleic acid primer comprises a nucleic acid sequence CAACACGAACGTCATGATAC (SEQ ID NO:16), or the complement thereof, or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:16, or the complement thereof.

2. A method according to claim 1, wherein the forward and reverse nucleic acid amplification primers comprise respective nucleic acid sequences of SEQ ID NO:1 and SEQ ID NO:5, or nucleic acid sequences which have at least 85%, 86%, 87%, 88%, 89%, 90%, 91%, 92%, 93%, 94%, 95%, 96%, 97%, 98%, or 99% identity along their entire length with such sequences, optionally wherein the forward nucleic acid amplification primer comprises a nucleic acid sequence of SEQ ID NO:1, and the reverse nucleic acid amplification primer comprises a nucleic acid sequence of SEQ ID NO:5.

3. A method according to claim 1, wherein the forward nucleic acid primer comprises a nucleic acid sequence of: T AGTTGATGACCCGTGTCCT (SEQ ID NO:14), or the complement thereof, or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14, or the complement thereof, optionally wherein the forward nucleic acid amplification primer comprises a nucleic acid sequence of SEQ ID NO:14, and the reverse nucleic acid amplification primer comprises a nucleic acid sequence of SEQ ID NO:16.

4. A method according to any preceding claim, which further comprises reverse transcribing SARS-CoV-2 RNA of the sample, and amplifying a product of the reverse transcription by an isothermal amplification reaction using the forward and reverse nucleic acid amplification primers, optionally wherein the reverse nucleic acid primer further comprises a promoter sequence for a DNA-dependent RNA polymerase at its 5'-end, and reverse transcription is carried out using the reverse nucleic acid primer and/or which further comprises isolating nucleic acid of the sample before reverse transcribing SARS-CoV-2 RNA of the sample present in the isolated nucleic acid.

5. A method according to any preceding claim, which further comprises capturing a product of the isothermal amplification reaction by hybridising nucleic acid of the product to a nucleic acid capture probe, wherein the capture probe hybridises specifically to nucleic acid sequence that is conserved in SARS-CoV-2 nucleic acid, but not in other human coronavirus nucleic acid, or the complement thereof, optionally wherein:

the forward and reverse nucleic acid primers hybridise specifically to nucleic acid sequence that is conserved in the ORF1ab gene of SARS-CoV-2, or the complement thereof, and the capture probe hybridizes specifically to nucleic acid sequence that is conserved in the ORF1ab gene of SARS-CoV-2, or the complement thereof, optionally wherein the capture probe comprises a nucleic acid of sequence GAGGACAATCAGACAACTACT ATTC (SEQ ID NO:9), or the complement thereof, or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:9, or the complement thereof; and/or
the forward and reverse nucleic acid primers hybridise specifically to nucleic acid sequence that is conserved in the nucleocapsid gene of SARS-CoV-2, or the complement thereof, and the capture probe hybridizes specifically to nucleic acid sequence that is conserved in the nucleocapsid gene of SARS-CoV-2, or the complement thereof, optionally wherein the capture probe comprises a nucleic acid of sequence: CTGGTTCTAAATCACCCATTCA (SEQ ID NO:18), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:18, or the complement thereof.

6. A method according to any preceding claim, which further comprises detecting a product of the isothermal amplification reaction by hybridising the product to a nucleic acid detector probe, wherein the detector probe hybridises specifically to SARS-CoV-2 nucleic acid sequence that is conserved in SARS-CoV-2 nucleic acid, but not in other human coronavirus nucleic acid, or the complement thereof, optionally wherein:

the forward and reverse nucleic acid primers hybridise specifically to nucleic acid sequence that is conserved in the ORF1ab gene of SARS-CoV-2, or the complement thereof, and the detector probe hybridizes specifically to nucleic acid sequence that is conserved in the ORF1ab gene of SARS-CoV-2, or the complement thereof, optionally wherein the detector probe comprises a nucleic acid sequence of GAGGTTCAACCTCAATTAGAG

ATGG (SEQ ID NO:12), or the complement thereof, or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:12, or the complement thereof; or

the forward and reverse nucleic acid primers hybridise specifically to nucleic acid sequence that is conserved in the nucleocapsid gene of SARS-CoV-2, or the complement thereof, and the detector probe hybridizes specifically to nucleic acid sequence that is conserved in the nucleocapsid gene of SARS-CoV-2, or the complement thereof, optionally wherein the detector probe comprises a nucleic acid sequence of GAACCTAAATTGGGTAGTCTT GTAG (SEQ ID NO:19), or the complement thereof, or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:19, or the complement thereof;

optionally wherein the detector probe is labelled with a visually detectable label.

7. A method according to any preceding claim, which comprises amplifying with forward and reverse nucleic acid amplification primers, capture of amplification product with capture probe, and detection of amplification product with detector probe, wherein the amplification primers and capture and detector probes comprise respective nucleic acid sequences of SEQ ID NO:1 (forward primer), SEQ ID NO:5 (reverse primer), SEQ ID NO:9 (capture probe), and SEQ ID NO:12 (detector probe), or nucleic acid sequences which have at least 85%, 86%, 87%, 88%, 89%, 90%, 91%, 92%, 93%, 94%, 95%, 96%, 97%, 98%, or 99% identity along their entire length with such sequences.

8. A method according to any of claims 5 to 7, wherein capture and/or detection of the product of the isothermal amplification reaction is carried out by chromatographic dipstick assay.

9. A method according to any preceding claim, wherein the sample is a biological sample that has been obtained from a subject suspected of being infected with SARS-CoV-2, optionally wherein the sample is a nasopharyngeal or a throat swab sample obtained from a subject suspected of being infected with SARS-CoV-2, and/or which is an *in vitro* method.

10. A kit for determining whether a sample includes SARS-CoV-2 nucleic acid, which comprises:

a forward nucleic acid amplification primer and a reverse nucleic acid amplification primer, for amplifying a template nucleic acid by an isothermal amplification reaction, wherein each nucleic acid amplification primer hybridises specifically to nucleic acid sequence that is conserved in SARS-CoV-2 nucleic acid, but not in other human coronavirus nucleic acid, or the complement thereof;

a nucleic acid capture probe, wherein the capture probe hybridises specifically to nucleic acid sequence that is conserved in SARS-CoV-2 nucleic acid, but not in other human coronavirus nucleic acid, or the complement thereof; and/or

a nucleic acid detector probe, wherein the detector probe hybridises specifically to nucleic acid sequence that is conserved in SARS-CoV-2 nucleic acid, but not in other human coronavirus nucleic acid, or the complement thereof, optionally wherein the detector probe comprises a detectable label for labelling a product of the isothermal nucleic acid amplification, wherein:

the forward nucleic acid primer hybridizes specifically to nucleic acid sequence that is conserved in the ORF1ab gene of SARS-CoV-2, or the complement thereof, wherein the forward and reverse nucleic acid amplification primers comprise respective nucleic acid sequences of SEQ ID NO:1 (forward) and SEQ ID NO:5 (reverse), or nucleic acid sequences which have at least 85%, 86%, 87%, 88%, 89%, 90%, 91%, 92%, 93%, 94%, 95%, 96%, 97%, 98%, or 99% identity along their entire length with such sequences, or the complement thereof; and/or

the forward nucleic acid primer hybridizes specifically to nucleic acid sequence that is conserved in the nucleocapsid gene of SARS-CoV-2, or the complement thereof, wherein the forward nucleic acid primer comprises a nucleic acid sequence of: TAGTTGATGACCCGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14, or the complement thereof, and wherein the reverse nucleic acid primer hybridizes specifically to nucleic acid sequence that is conserved in the nucleocapsid gene of SARS-CoV-2, or the complement thereof, wherein the reverse nucleic acid primer comprises a nucleic acid sequence CAACACGAACGTCATGATAC (SEQ ID NO:16), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:16, or the complement thereof, optionally wherein the forward nucleic acid amplification primer comprises a nucleic acid sequence of SEQ ID NO:14, and the reverse nucleic acid amplification primer comprises a nucleic acid sequence of SEQ ID NO:16.

11. A kit according to claim 10, wherein:

the forward and reverse nucleic acid primers hybridise specifically to nucleic acid sequence that is conserved in the ORF1ab gene of SARS-CoV-2, or the complement thereof, and the capture probe hybridizes specifically to nucleic acid sequence that is conserved in the ORF1ab gene of SARS-CoV-2, or the complement thereof, optionally wherein the capture probe comprises a nucleic acid of sequence: GAGGACAATCAGACAACTACT ATTC (SEQ ID NO:9), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:9, or the complement thereof; and/or

the forward and reverse nucleic acid primers hybridise specifically to nucleic acid sequence that is conserved in the nucleocapsid gene of SARS-CoV-2, or the complement thereof, and the capture probe hybridizes specifically to nucleic acid sequence that is conserved in the nucleocapsid gene of SARS-CoV-2, or the complement thereof, optionally wherein the capture probe comprises a nucleic acid of sequence: CTGGTTCTAAATCACCCATTCA (SEQ ID NO:18), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:18, or the complement thereof.

12. A kit according to claim 10 or 11, wherein the forward and reverse nucleic acid primers hybridise specifically to nucleic acid sequence that is conserved in the ORF1ab gene of SARS-CoV-2, or the complement thereof, and the detector probe hybridizes specifically to nucleic acid sequence that is conserved in the ORF1ab gene of SARS-CoV-2, or the complement thereof, optionally wherein the detector probe comprises a nucleic acid sequence of: GAGGTTCAA CCTCAATTAGAGATGG (SEQ ID NO:12), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:12, or the complement thereof.

13. A kit according to any of claims 10 to 12, which comprises forward and reverse amplification primers, and capture and detector probes comprising respective nucleic acid sequences SEQ ID NO:1 (forward), SEQ ID NO:5 (reverse), SEQ ID NO:9 (capture probe), and SEQ ID NO:12 (detector probe), or nucleic acid sequences which have at least 85%, 86%, 87%, 88%, 89%, 90%, 91%, 92%, 93%, 94%, 95%, 96%, 97%, 98%, or 99% identity along their entire length with such sequences.

14. A kit according to any of claims 10 to 13, wherein the forward and reverse nucleic acid primers hybridise specifically to nucleic acid sequence that is conserved in the nucleocapsid gene of SARS-CoV-2, or the complement thereof, and the detector probe hybridizes specifically to nucleic acid sequence that is conserved in the nucleocapsid gene of SARS-CoV-2, or the complement thereof, optionally wherein the detector probe comprises a nucleic acid sequence of: GAACCTAAATTGGGTAGTCTTGTAG (SEQ ID NO:19), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO: 19, or the complement thereof.

15. A kit according to any of claims 10 to 14, which further comprises:

an RNA-dependent DNA polymerase, a DNA-dependent DNA polymerase, a DNA/RNA duplex-specific ribonuclease, and a DNA-dependent RNA polymerase; and/or

a swab stick for obtaining a nasopharyngeal or throat swab sample from a subject, optionally wherein the kit further comprises a first swab stick for obtaining a nasopharyngeal swab sample from a subject, and a second swab stick for obtaining a throat swab sample from a subject; and/or

a chromatographic test strip for capturing and detecting a product of the isothermal nucleic acid amplification; and/or

a lysis/binding buffer, an elution buffer, and optionally a wash buffer, for extracting nucleic acid from a biological sample obtained from the subject.

16. A set of primers for amplifying SARS-CoV-2 nucleic acid by an isothermal nucleic acid amplification reaction, which comprises a forward nucleic acid amplification primer and a reverse nucleic acid amplification primer, wherein each nucleic acid amplification primer hybridises specifically to nucleic acid sequence that is conserved in SARS-CoV-2 nucleic acid, but not in other human coronavirus nucleic acid, or the complement thereof, wherein the nucleic acid sequence that is conserved in SARS-CoV-2 nucleic acid is nucleic acid sequence that is conserved in the ORF1ab gene or the Nucleocapsid gene of SARS-CoV-2, or the complement thereof, wherein:

the forward nucleic acid primer hybridizes specifically to nucleic acid sequence that is conserved in the ORF1ab gene of SARS-CoV-2, or the complement thereof, wherein the forward nucleic acid primer comprises a nucleic acid sequence of: CTGTTGGTCAACAAGACGGCA (SEQ ID NO:1), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, or the complement thereof, and the reverse nucleic acid primer hybridizes specifically to nucleic acid sequence that is conserved in the ORF1ab gene of SARS-CoV-2, or the complement thereof, wherein the reverse nucleic acid primer comprises a nucleic acid sequence: CAATA GTCTGAACAACTGGTGT (SEQ ID NO:5), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, or the complement thereof; and/or

the forward nucleic acid primer hybridizes specifically to nucleic acid sequence that is conserved in the nucleocapsid gene of SARS-CoV-2, or the complement thereof, wherein the forward nucleic acid primer comprises a nucleic acid sequence of: TAGTTGATGACCCGTGTCCT (SEQ ID NO:14) or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14, or the complement thereof, and wherein the reverse nucleic acid primer hybridizes specifically to nucleic acid sequence that is conserved in the nucleocapsid gene of SARS-CoV-2, or the complement thereof, wherein the reverse nucleic acid primer comprises a nucleic acid sequence: CAACACGAACGTCATGATAC (SEQ ID NO:16), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:16, or the complement thereof;

optionally wherein the forward and/or the reverse nucleic acid primer is up to 50 nucleotides long.

17. A set of oligonucleotides for amplifying SARS-CoV-2 nucleic acid by an isothermal nucleic acid amplification reaction, and for capturing and/or detecting a product of the amplification reaction, which comprises:

a set of primers according to claim 16;

a nucleic acid capture probe, wherein the capture probe hybridises specifically to nucleic acid sequence that is conserved in SARS-CoV-2 nucleic acid, but not in other human coronavirus nucleic acid, or the complement thereof; and/or

a nucleic acid detector probe, wherein the detector probe hybridises specifically to nucleic acid sequence that is conserved in SARS-CoV-2 nucleic acid, but not in other human coronavirus nucleic acid, or the complement thereof, optionally wherein the detector probe comprises a detectable label for labelling a product of the isothermal nucleic acid amplification.

18. A set of oligonucleotides according to claim 17, wherein:

the forward and reverse nucleic acid primers hybridise specifically to nucleic acid sequence that is conserved in the ORF1ab gene of SARS-CoV-2, or the complement thereof, and the capture probe hybridizes specifically to nucleic acid sequence that is conserved in the ORF1ab gene of SARS-CoV-2, or the complement thereof, wherein the capture probe comprises a nucleic acid of sequence: GAGGACAATCAGACAACTACTATTC (SEQ ID NO:9), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:9, or the complement thereof; and/or the detector probe comprises a nucleic acid sequence of: GAGGTTCAACCTCA ATTAGAGATGG (SEQ ID NO:12), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:12, or the complement thereof; and/or

the forward and reverse nucleic acid primers hybridise specifically to nucleic acid sequence that is conserved in the nucleocapsid gene of SARS-CoV-2, or the complement thereof, and the capture probe hybridizes specifically to nucleic acid sequence that is conserved in the nucleocapsid gene of SARS-CoV-2, or the complement thereof, wherein the capture probe comprises a nucleic acid of sequence: CTGGTTCTAAATCACCCATTCA (SEQ ID NO:18), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:18, or the complement thereof; and/or the detector probe comprises a nucleic acid sequence of: GAACCTAAATTGGG TAGTCTTGTAG (SEQ ID NO:19), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO: 19, or the complement thereof;
optionally wherein the capture and/or detector probe is up to 50 nucleotides long.

19. A kit according to any of claims 10 to 15, which comprises a set of primers according to claim 16, or a set of oligonucleotides according to claim 17 or 18.

20. Use of a set of primers according to claim 16, or a set of oligonucleotides according to claim 17 or 18, in a method according to any of claims 1 to 9.

21. An oligonucleotide for use in a method according to claim 1, which comprises:

a nucleic acid sequence of: CTGTTGGTCAACAAGACGGCA (SEQ ID NO:1), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:1, or the complement thereof;
a nucleic acid sequence of: CAATAGTCTGAACAACTGGTGT (SEQ ID NO:5), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:5, or the complement thereof;
a nucleic acid sequence of: GAGGACAATCAGACAACTACTATTC (SEQ ID NO:9), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:9, or the complement thereof;
a nucleic acid sequence of: GAGGTTCAACCTCAATTAGAGATGG (SEQ ID NO:12), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:12, or the complement thereof;
a nucleic acid sequence of: TAGTTGATGACCCGTGTCCT (SEQ ID NO:14), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:14, or the complement thereof;
a nucleic acid sequence of: CAACACGAACGTCATGATAC (SEQ ID NO:16), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:16, or the complement thereof;
a nucleic acid sequence of: CTGGTTCTAAATCACCCATTCA (SEQ ID NO:18), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:18, or the complement thereof; or
a nucleic acid sequence of: GAACCTAAATTGGGTAGTCTTGTAG (SEQ ID NO:19), or a nucleic acid sequence that has at least 85%, 86%, 87%, 88%, 89%, 90%, 91%, 92%, 93%, 94%, 95%, 96%, 97%, 98%, or 99% identity along its entire length with a nucleic acid sequence of SEQ ID NO:19, or the complement thereof.

**Patentansprüche**

1. Verfahren zum Bestimmen, ob eine Probe Nukleinsäure des Schweren Akuten Respiratorischen Syndroms Coronavirus 2 (SARS-CoV-2) einschließt, die das Amplifizieren von Nukleinsäure der Probe oder das Amplifizieren von Nukleinsäure, die von Nukleinsäure der Probe abgeleitet ist, durch eine isotherme Amplifikationsreaktion unter Verwendung eines Vorwärts-Nukleinsäureamplifikationsprimers und eines Rückwärts-Nukleinsäureamplifikationsprimers umfasst, wobei jeder Nukleinsäureamplifikationsprimer spezifisch an eine Nukleinsäuresequenz hybridisiert, die in der SARS-CoV-2-Nukleinsäure konserviert ist, jedoch nicht in anderer menschlicher Coronavirus-Nukleinsäure oder dem Komplement davon, wobei die in der SARS-CoV-2-Nukleinsäure konservierte Nukleinsäuresequenz eine Nukleinsäuresequenz ist, die im ORF1ab-Gen oder im Nukleokapsid-Gen von SARS-CoV-2 konserviert ist, wobei:

der Vorwärts-Nukleinsäureprimer spezifisch an eine Nukleinsäuresequenz hybridisiert, die im ORF1ab-Gen von SARS-CoV-2 oder dem Komplement davon konserviert ist, wobei der Vorwärts-Nukleinsäureprimer eine Nukleinsäuresequenz von CTGTTGGTCAACAAGACGGCA (SEQ ID NO:1) oder das Komplement davon umfasst, oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz SEQ ID NO:1 oder dem Komplement davon aufweist, und der Rückwärts-Nukleinsäureprimer spezifisch an eine Nukleinsäuresequenz hybridisiert, die im ORF1ab-Gen von SARS-CoV-2 oder dem Komplement davon konserviert ist, wobei der Rückwärts-Nukleinsäureprimer eine Nukleinsäuresequenz CAATAGTCTGAACAACTGGTGT (SEQ ID NO:5) oder das Komplement davon umfasst, oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz SEQ ID NO:5 oder dem Komplement davon aufweist; und/oder der Vorwärts-Nukleinsäureprimer spezifisch an eine Nukleinsäuresequenz hybridisiert, die im Nukleokapsid-Gen von SARS-CoV-2 oder dem Komplement davon konserviert ist, wobei der Vorwärts-Nukleinsäureprimer folgende Nukleinsäuresequenz umfasst: TAGTTGATGACCCGTGTCCT (SEQ ID NO: 14) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:14 oder dem Komplement davon aufweist und der Rückwärts-Nukleinsäureprimer spezifisch an eine Nukleinsäuresequenz hybridisiert, die in dem Nukleokapsid-Gen von SARS-CoV-2 oder dem Komplement davon konserviert ist, wobei der Rückwärts-Nukleinsäureprimer eine Nukleinsäuresequenz CAACACGAACGTCATGATAC (SEQ ID NO:16) oder das Komplement davon umfasst, oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:16 oder dem Komplement davon aufweist.

2. Verfahren nach Anspruch 1, wobei der Vorwärts- und der Rückwärts-Nukleinsäureamplifikationsprimer entsprechende Nukleinsäuresequenzen von SEQ ID NO:1 und SEQ ID NO:5 oder Nukleinsäuresequenzen umfassen, die mindestens 85%, 86%, 87%, 88%, 89%, 90%, 91%, 92%, 93%, 94%, 95%, 96%, 97%, 98% oder 99% Identität über ihre gesamte Länge mit solchen Sequenzen aufweisen, wobei optional der Vorwärts-Nukleinsäureamplifikationsprimer eine Nukleinsäuresequenz von SEQ ID NO:1 umfasst und der Rückwärts-Nukleinsäureamplifikationsprimer eine Nukleinsäuresequenz von SEQ ID NO:5 umfasst.

3. Verfahren nach Anspruch 1, wobei der Vorwärts-Nukleinsäureprimer folgende Nukleinsäuresequenz umfasst: TAGTTGATGACCCGTGTCCT (SEQ ID NO:14) oder das Komplement davon, oder eine Nukleinsäuresequenz, die mindestens 85%, 86%, 87%, 88%, 89%, 90%, 91%, 92%, 93%, 94%, 95%, 96%, 97%, 98%, Identität über ihre gesamte Länge mit einer Nukleinsäuresequenz von SEQ ID NO:14 oder dem Komplement davon aufweist, optional wobei der Vorwärts-Nukleinsäureamplifikationsprimer eine Nukleinsäuresequenz von SEQ ID NO:14 umfasst und der Rückwärts-Nukleinsäureamplifikationsprimer eine Nukleinsäuresequenz von SEQ ID NO:16 umfasst.

4. Verfahren nach einem der vorstehenden Ansprüche, das ferner das reverse Transkribieren der SARS-CoV-2-RNA der Probe und das Amplifizieren eines Produkts der reversen Transkription durch eine isotherme Amplifikationsreaktion unter Verwendung der Vorwärts- und Rückwärts-Nukleinsäureamplifikationsprimer umfasst, optional wobei der reverse Nukleinsäureprimer ferner eine Promotorsequenz für eine DNA-abhängige RNA-Polymerase an seinem 5'-Ende umfasst und die reverse Transkription unter Verwendung des reversen Nukleinsäureprimers durchgeführt wird und/oder das ferner das Isolieren von Nukleinsäure der Probe vor dem reversen Transkribieren von SARS-CoV-2-RNA der Probe, die in der isolierten Nukleinsäure vorhanden ist, umfasst.

5. Verfahren nach einem der vorstehenden Ansprüche, das ferner das Einfangen eines Produkts der isothermen Amplifikationsreaktion durch Hybridisieren der Nukleinsäure des Produkts mit einer Nukleinsäure-Einfangsonde umfasst, wobei die Einfangsonde spezifisch an eine Nukleinsäuresequenz hybridisiert, die in der SARS-CoV-2-Nukleinsäure, jedoch nicht in anderer menschlicher Coronavirus-Nukleinsäure oder dem Komplement davon konserviert ist, wobei optional:

der Vorwärts- und der Rückwärts-Nukleinsäureprimer spezifisch an eine Nukleinsäuresequenz hybridisieren, die im ORF1ab-Gen von SARS-CoV-2 oder dem Komplement davon konserviert ist, und die Einfangsonde spezifisch an eine Nukleinsäuresequenz hybridisiert, die im ORF1ab-Gen von SARS-CoV-2 oder dem Komplement davon konserviert ist, optional wobei die Einfangsonde eine Nukleinsäure der Sequenz GAGGACAATCAGACAACTACTATTC (SEQ ID NO:9) oder das Komplement davon umfasst, oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:9 oder dem Komplement davon aufweist; und/oder der Vorwärts- und der Rückwärts-Nukleinsäureprimer spezifisch an eine Nukleinsäuresequenz hybridisieren, die im Nukleokapsid-Gen von SARS-CoV-2 oder dem Komplement davon konserviert ist, und die Einfangsonde spezifisch an eine Nukleinsäuresequenz hybridisiert, die im Nukleokapsid-Gen von SARS-CoV-2 oder dem Komplement davon konserviert ist, optional wobei die Einfangsonde eine Nukleinsäure mit der folgenden Sequenz umfasst: CTGGTTCTAAATCACCCATTCA (SEQ ID NO:18), oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:18 oder dem Komplement davon aufweist.

6. Verfahren nach einem der vorstehenden Ansprüche, das ferner das Erfassen eines Produkts der isothermen Amplifikationsreaktion durch Hybridisieren des Produkts an einer Nukleinsäure-Detektorsonde umfasst, wobei die Detektorsonde spezifisch an eine SARS-CoV-2-Nukleinsäuresequenz hybridisiert, die in der SARS-CoV-2-Nukleinsäure, jedoch nicht in anderer menschlicher Coronavirus-Nukleinsäure oder dem Komplement davon konserviert ist, wobei optional:

   der Vorwärts- und der Rückwärts-Nukleinsäureprimer spezifisch an eine Nukleinsäuresequenz hybridisieren, die im ORF1ab-Gen von SARS-CoV-2 oder dem Komplement davon konserviert ist, und die Detektorsonde spezifisch an eine Nukleinsäuresequenz hybridisiert, die im ORF1ab-Gen von SARS-CoV-2 oder dem Komplement davon konserviert ist, optional wobei die Detektorsonde eine Nukleinsäuresequenz GAGGTTCAAC CTCAATTAGAGATGG (SEQ ID NO:12) oder das Komplement davon umfasst, oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:12 oder dem Komplement davon aufweist; und/oder
   der Vorwärts- und der Rückwärts-Nukleinsäureprimer spezifisch an eine Nukleinsäuresequenz hybridisieren, die im Nukleokapsid-Gen von SARS-CoV-2 oder dem Komplement davon konserviert ist, und die Detektorsonde spezifisch an eine Nukleinsäuresequenz hybridisiert, die im Nukleokapsid-Gen von SARS-CoV-2 oder dem Komplement davon konserviert ist, optional wobei die Detektorsonde eine Nukleinsäuresequenz GAACCTA AATTGGGTAGTCTTGTAG (SEQ ID NO:19) oder das Komplement davon umfasst, oder eine Nukleinsäuresequenz, die mindestens 85%, 86%, 87%, 88%, 89%, 90%, 91%, 92%, 93%, 94 %, 95%, 96%, 97%, 98% oder 99% Identität über ihre gesamte Länge mit einer Nukleinsäuresequenz von SEQ ID NO:19 oder dem Komplement davon aufweist;
   optional wobei die Detektorsonde mit einem visuell erkennbaren Label markiert ist.

7. Verfahren nach einem der vorstehenden Ansprüche, das das Amplifizieren mit Vorwärts- und Rückwärts-Nukleinsäureamplifikationsprimern, das Einfangen des Amplifikationsprodukts mit einer Einfangsonde und das Erfassen eines Amplifikationsprodukts mit einer Detektorsonde umfasst, wobei die Amplifikationsprimer und die Einfang- und Detektorsonde entsprechende Nukleinsäuresequenzen von SEQ ID NO:1 (Vorwärtsprimer), SEQ ID NO:5 (Rückwärtsprimer), SEQ ID NO:9 (Einfangsonde) und SEQ ID NO:12 (Detektorsonde) umfassen oder Nukleinsäuresequenzen, die mindestens 85%, 86%, 87%, 88%, 89%, 90%, 91%, 92%, 93%, 94%, 95%, 96%, 97%, 98% oder 99% Identität über ihre gesamte Länge mit solchen Sequenzen aufweisen.

8. Verfahren nach einem der Ansprüche 5 bis 7, wobei das Einfangen und/oder Erfassen des Produkts der isothermen Amplifikationsreaktion durch einen chromatographischen Dipstick-Assay ausgeführt wird.

9. Verfahren nach einem der vorstehenden Ansprüche, wobei die Probe eine biologische Probe ist, die von einem Subjekt erlangt wurde, von dem man davon ausgeht, dass es mit SARS-CoV-2 infiziert ist, optional wobei die Probe eine Nasopharyngeal- oder Rachenabstrichprobe ist, die von einem Subjekt erlangt wurde, von dem man davon ausgeht, dass es mit SARS-CoV-2 infiziert ist, und/oder das ein *In Vitro*-Verfahren ist.

10. Kit zum Bestimmen, ob eine Probe SARS-CoV-2-Nukleinsäure einschließt, umfassend:

   einen Vorwärts-Nukleinsäureamplifikationsprimer und einen Rückwärts-Nukleinsäureamplifikationsprimer zum Amplifizieren einer Matrizen-Nukleinsäure durch eine isotherme Amplifikationsreaktion, wobei jeder Nukleinsäureamplifikationsprimer spezifisch an eine Nukleinsäuresequenz hybridisiert, die in SARS-CoV-2-Nukleinsäure konserviert ist, jedoch nicht in anderer menschlicher Coronavirus-Nukleinsäure oder dem Komplement davon;
   eine Nukleinsäure-Einfangsonde, wobei die Einfangsonde spezifisch an eine Nukleinsäuresequenz hybridisiert, die in SARS-CoV-2-Nukleinsäure konserviert ist, jedoch nicht in anderer menschlicher Coronavirus-Nukleinsäure oder dem Komplement davon; und/oder
   eine Nukleinsäure-Detektorsonde, wobei die Detektorsonde spezifisch an eine Nukleinsäuresequenz hybridisiert, die in SARS-CoV-2-Nukleinsäure konserviert ist, jedoch nicht in anderer menschlicher Coronavirus-Nukleinsäure oder dem Komplement davon, optional wobei die Detektorsonde ein erkennbares Label zum Markieren eines Produkts der isothermen Nukleinsäureamplifikation umfasst, wobei:

   der Vorwärts-Nukleinsäureprimer spezifisch an eine Nukleinsäuresequenz hybridisiert, die im ORF1ab-Gen von SARS-CoV-2 oder dem Komplement davon konserviert ist, wobei der Vorwärts- und der Rückwärts-Nukleinsäureamplifikationsprimer entsprechende Nukleinsäuresequenzen von SEQ ID NO:1 (Vorwärts) und SEQ ID NO:5 (Rückwärts) oder Nukleinsäuresequenzen umfassen, die mindestens 85%, 86%, 87%, 88%, 89%, 90%, 91%, 92%, 93%, 94%, 95%, 96%, 97%, 98% oder 99% Identität über ihre gesamte Länge mit solchen Sequenzen oder dem Komplement davon aufweisen; und/oder

der Vorwärts-Nukleinsäureprimer spezifisch an eine Nukleinsäuresequenz hybridisiert, die im Nukleokapsid-Gen von SARS-CoV-2 oder dem Komplement davon konserviert ist, wobei der Vorwärts-Nukleinsäureprimer folgende Nukleinsäuresequenz umfasst: TAGTTGATGACCCGTGTCCT (SEQ ID NO: 14) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:14 oder dem Komplement davon aufweist und wobei der Rückwärts-Nukleinsäureprimer spezifisch an einer Nukleinsäuresequenz hybridisiert, die in dem Nukleokapsid-Gen von SARS-CoV-2 oder dem Komplement davon konserviert ist, wobei der Rückwärts-Nukleinsäureprimer eine Nukleinsäuresequenz CAACACGA ACGTCATGATAC (SEQ ID NO:16) umfasst, oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:16 oder dem Komplement davon aufweist, optional wobei der Vorwärts-Nukleinsäureamplifikationsprimer eine Nukleinsäuresequenz von SEQ ID NO:14 umfasst und der Rückwärts-Nukleinsäureamplifikationsprimer eine Nukleinsäuresequenz von SEQ ID NO:16 umfasst.

11. Kit nach Anspruch 10, wobei:

der Vorwärts- und der Rückwärts-Nukleinsäureprimer spezifisch an eine Nukleinsäuresequenz hybridisieren, die im ORF1ab-Gen von SARS-CoV-2 oder dem Komplement davon konserviert ist, und die Einfangsonde spezifisch an eine Nukleinsäuresequenz hybridisiert, die im ORF1ab-Gen von SARS-CoV-2 oder dem Komplement davon konserviert ist, optional wobei die Einfangsonde eine Nukleinsäure mit der folgenden Sequenz umfasst: GAGGACAATCAGACAACTACTATTC (SEQ ID NO:9), oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:9 oder dem Komplement davon aufweist; und/oder
der Vorwärts- und der Rückwärts-Nukleinsäureprimer spezifisch an eine Nukleinsäuresequenz hybridisieren, die im Nukleokapsid-Gen von SARS-CoV-2 oder dem Komplement davon konserviert ist, und die Einfangsonde spezifisch an eine Nukleinsäuresequenz hybridisiert, die im Nukleokapsid-Gen von SARS-CoV-2 oder dem Komplement davon konserviert ist, optional wobei die Einfangsonde eine Nukleinsäure mit der folgenden Sequenz umfasst: CTGGTTCTAAATCACCCATTCA (SEQ ID NO:18), oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:18 oder dem Komplement davon aufweist.

12. Kit nach Anspruch 10 oder 11, wobei der Vorwärts- und der Rückwärts-Nukleinsäureprimer spezifisch an eine Nukleinsäuresequenz hybridisieren, die im ORF1ab-Gen von SARS-CoV-2 oder dem Komplement davon konserviert ist, und die Detektorsonde spezifisch an eine Nukleinsäuresequenz hybridisiert, die im ORF1ab-Gen von SARS-CoV-2 oder dem Komplement davon konserviert ist, optional wobei die Detektorsonde eine Nukleinsäure mit der folgenden Sequenz umfasst: GAGGTTCAACCTCAATTAGAGATGG (SEQ ID NO:12) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:12 oder dem Komplement davon aufweist.

13. Kit nach einem der Ansprüche 10 bis 12, das Vorwärts- und Rückwärts-Amplifikationsprimer umfasst, sowie Einfang- und Detektorsonden, die entsprechende Nukleinsäuresequenzen von SEQ ID NO:1 (Vorwärts), SEQ ID NO:5 (Rückwärts), SEQ ID NO:9 (Einfangsonde) und SEQ ID NO:12 (Detektorsonde) umfassen oder Nukleinsäuresequenzen, die mindestens 85%, 86%, 87%, 88%, 89%, 90%, 91%, 92%, 93%, 94%, 95%, 96%, 97%, 98% oder 99% Identität über ihre gesamte Länge mit solchen Sequenzen aufweisen.

14. Kit nach Anspruch 10 bis 13, wobei der Vorwärts- und der Rückwärts-Nukleinsäureprimer spezifisch an eine Nukleinsäuresequenz hybridisieren, die im Nukleokapsid-Gen von SARS-CoV-2 oder dem Komplement davon konserviert ist, und die Detektorsonde spezifisch an eine Nukleinsäuresequenz hybridisiert, die im Nukleokapsid-Gen von SARS-CoV-2 oder dem Komplement davon konserviert ist, optional wobei die Detektorsonde eine Nukleinsäure mit der folgenden Sequenz umfasst: GAACCTAAATTGGGTAGTCTTGTAG (SEQ ID NO:19) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO: 19 oder dem Komplement davon aufweist.

15. Kit nach einem der Ansprüche 10 bis 14, ferner umfassend:

eine RNA-abhängige DNA-Polymerase, eine DNA-abhängige DNA-Polymerase, eine DNA/RNA-Duplex-spezifische Ribonuklease und eine DNA-abhängige RNA-Polymerase; und/oder

Abstrichstäbchen zum Erlangen einer Nasopharyngeal- oder Rachenabstrichprobe von einem Subjekt, optional wobei das Kit ferner ein erstes Abstrichstäbchen zum Erlangen einer Nasopharyngeal-Abstrichprobe von einem Subjekt und ein zweites Abstrichstäbchen zum Erlangen einer Rachenabstrichprobe von einem Subjekt umfasst; und/oder

einen chromatographischen Teststreifen zum Einfangen und Erfassen eines Produkts der isothermen Nukleinsäureamplifikation; und/oder

einen Lyse-/Bindungs-Puffer, einen Elutionspuffer und optional einen Waschpuffer zum Extrahieren von Nukleinsäure aus einer biologischen Probe, die von dem Subjekt erlangt wurde.

16. Ein Satz von Primern zum Amplifizieren von SARS-CoV-2-Nukleinsäure durch eine isotherme Nukleinsäureamplifikationsreaktion, der einen Vorwärts-Nukleinsäureamplifikationsprimer und einen Rückwärts-Nukleinsäureamplifikationsprimer umfasst, wobei jeder Nukleinsäureamplifikationsprimer spezifisch an eine Nukleinsäuresequenz hybridisiert, die in der SARS-CoV-2-Nukleinsäure konserviert ist, jedoch nicht in anderer menschlicher Coronavirus-Nukleinsäure oder dem Komplement davon, wobei die in SARS-CoV-2-Nukleinsäure konservierte Nukleinsäuresequenz eine Nukleinsäuresequenz ist, die im ORF1ab-Gen oder im Nukleokapsid-Gen von SARS-CoV-2 oder dem Komplement davon konserviert ist, wobei:

der Vorwärts-Nukleinsäureprimer spezifisch an eine Nukleinsäuresequenz hybridisiert, die im ORF1ab-Gen von SARS-CoV-2 oder dem Komplement davon konserviert ist, wobei der Vorwärts-Nukleinsäureprimer folgende Nukleinsäuresequenz umfasst: CTGTTGGTCAACAAGACGGCA (SEQ ID NO:1) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:1 oder dem Komplement davon aufweist, und der reverse Nukleinsäureprimer spezifisch an eine Nukleinsäuresequenz hybridisiert, die im ORF1ab-Gen von SARS-CoV-2 oder dem Komplement davon konserviert ist, wobei der reverse Nukleinsäureprimer eine Nukleinsäuresequenz umfasst: CAATAGTCTGAACAACTGGTGT (SEQ ID NO:5) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:5 oder dem Komplement davon aufweist; und/oder

der Vorwärts-Nukleinsäureprimer spezifisch an eine Nukleinsäuresequenz hybridisiert, die im Nukleokapsid-Gen von SARS-CoV-2 oder dem Komplement davon konserviert ist, wobei der Vorwärts-Nukleinsäureprimer folgende Nukleinsäuresequenz umfasst: TAGTTGATGACCCGTGTCCT (SEQ ID NO: 14) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:14 oder dem Komplement davon aufweist, und wobei der reverse Nukleinsäureprimer spezifisch an eine Nukleinsäuresequenz hybridisiert, die im Nukleokapsid-Gen von SARS-CoV-2 oder dem Komplement davon konserviert ist, wobei der reverse Nukleinsäureprimer eine Nukleinsäuresequenz umfasst: CAACACGAACGTCATGATAC (SEQ ID NO:16) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:16 oder dem Komplement davon aufweist; optional wobei der Vorwärts- und/oder der Rückwärtsprimer bis zu 50 Nukleotide lang ist.

17. Satz von Oligonukleotiden zum Amplifizieren von SARS-CoV-2-Nukleinsäure durch eine isotherme Nukleinsäureamplifikationsreaktion und zum Einfangen und/oder Erfassen eines Produkts der Amplifikationsreaktion, der umfasst:

Satz von Primern nach Anspruch 16;

eine Nukleinsäure-Einfangsonde, wobei die Einfangsonde spezifisch an eine Nukleinsäuresequenz hybridisiert, die in SARS-CoV-2-Nukleinsäure konserviert ist, jedoch nicht in anderer menschlicher Coronavirus-Nukleinsäure oder dem Komplement davon; und/oder

eine Nukleinsäure-Detektorsonde, wobei die Detektorsonde spezifisch an eine Nukleinsäuresequenz hybridisiert, die in SARS-CoV-2-Nukleinsäure konserviert ist, jedoch nicht in anderer menschlicher Coronavirus-Nukleinsäure oder dem Komplement davon, optional wobei die Detektorsonde ein erkennbares Label zum

EP 4 127 246 B1

Markieren eines Produkts der isothermen Nukleinsäureamplifikation umfasst.

18. Satz von Oligonukleotiden nach Anspruch 17, wobei:

der Vorwärts- und der Rückwärts-Nukleinsäureprimer spezifisch an eine Nukleinsäuresequenz hybridisieren, die im ORF1ab-Gen von SARS-CoV-2 oder dem Komplement davon konserviert ist, und die Einfangsonde spezifisch an eine Nukleinsäuresequenz hybridisiert, die im ORF1ab-Gen von SARS-CoV-2 oder dem Komplement davon konserviert ist, wobei die Einfangsonde eine Nukleinsäure mit der folgenden Sequenz umfasst: GAG GACAATCAGACAACTACTATTC (SEQ ID NO:9) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:9 oder dem Komplement davon aufweist; und/oder die Detektorsonde folgende Nukleinsäuresequenz umfasst: GAGGTTCAACCTCAATTAGAGATGG (SEQ ID NO:12) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:12 oder dem Komplement davon aufweist; und/oder
der Vorwärts- und der Rückwärts-Nukleinsäureprimer spezifisch an eine Nukleinsäuresequenz hybridisieren, die im Nukleokapsid-Gen von SARS-CoV-2 oder dem Komplement davon konserviert ist, und die Einfangsonde spezifisch an eine Nukleinsäuresequenz hybridisiert, die im Nukleokapsid-Gen von SARS-CoV-2 oder dem Komplement davon konserviert ist, wobei die Einfangsonde eine Nukleinsäure mit der folgenden Sequenz umfasst: CTGGTTCTAAATCACCCATTCA (SEQ ID NO:18) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:18 oder dem Komplement davon aufweist; und/oder die Detektorsonde folgende Nukleinsäuresequenz umfasst: GAACCTAAATTGGGTAGTCTTGTAG (SEQ ID NO:19) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO: 19 oder dem Komplement davon aufweist;
optional wobei die Einfang- und/oder Detektorsonde bis zu 50 Nukleotide lang ist.

19. Kit nach einem der Ansprüche 10 bis 15, das einen Satz von Primern nach Anspruch 16 oder einen Satz von Oligonukleotiden nach Anspruch 17 oder 18 umfasst.

20. Verwenden eines Satzes von Primern nach Anspruch 16 oder eines Satzes von Oligonukleotiden nach Anspruch 17 oder 18, in einem Verfahren nach einem der Ansprüche 1 bis 9.

21. Oligonukleotid für die Verwendung in einem Verfahren nach Anspruch 1, umfassend:

eine Nukleinsäuresequenz von: CTGTTGGTCAACAAGACGGCA (SEQ ID NO:1) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:1 oder dem Komplement davon aufweist;
eine Nukleinsäuresequenz von: CAATAGTCTGAACAACTGGTGT (SEQ ID NO:5) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:5 oder dem Komplement davon aufweist;
eine Nukleinsäuresequenz von: GAGGACAATCAGACAACTACTATTC (SEQ ID NO:9) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:9 oder dem Komplement davon aufweist.
eine Nukleinsäuresequenz von: GAGGTTCAACCTCAATTAGAGATGG (SEQ ID NO:12) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:12 oder dem Komplement davon aufweist;
eine Nukleinsäuresequenz von: TAGTTGATGACCCGTGTCCT (SEQ ID NO:14) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:14 oder dem Komplement davon aufweist.
eine Nukleinsäuresequenz von: CAACACGAACGTCATGATAC (SEQ ID N0:16) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:16 oder dem Komplement davon aufweist;

eine Nukleinsäuresequenz von: CTGGTTCTAAATCACCCATTCA (SEQ ID NO:18) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:18 oder dem Komplement davon aufweist. oder

eine Nukleinsäuresequenz von: GAACCTAAATTGGGTAGTCTTGTAG (SEQ ID NO:19) oder eine Nukleinsäuresequenz, die mindestens 85%, 86%, 87%, 88%, 89%, 90%, 91%, 92%, 93%, 94%, 95%, 96%, 97%, 98% oder 99% Identität über ihre gesamte Länge mit einer Nukleinsäuresequenz von SEQ ID NO:19 oder dem Komplement davon aufweist.

## Revendications

1. Procédé destiné à déterminer si un échantillon comporte un acide nucléique du coronavirus 2 du syndrome respiratoire aigu sévère (SARS-CoV-2), qui comprend l'amplification de l'acide nucléique de l'échantillon, ou l'amplification de l'acide nucléique dérivé de l'acide nucléique de l'échantillon, par une réaction d'amplification isotherme à l'aide d'une amorce d'amplification d'acide nucléique sens et une amorce d'amplification d'acide nucléique antisens, dans lequel chaque amorce d'amplification d'acide nucléique s'hybride plus particulièrement à une séquence d'acide nucléique qui est conservée dans l'acide nucléique du SARS-CoV-2, mais pas dans un autre acide nucléique du coronavirus humain, ou son complément, dans lequel la séquence d'acide nucléique qui est conservée dans l'acide nucléique du SARS-CoV-2 est une séquence d'acide nucléique qui est conservée dans le gène ORF1ab ou le gène de nucléocapside du SARS-CoV-2, dans lequel :

   l'amorce d'acide nucléique sens s'hybride plus particulièrement à la séquence d'acide nucléique qui est conservée dans le gène ORF1ab du SARS-CoV-2, ou son complément, l'amorce d'acide nucléique sens comprenant une séquence d'acide nucléique de CTGTTGGTCAACAAGACGGCA (SEQ ID N° : 1), ou son complément, ou une séquence d'acide nucléique qui présente au moins 85 %, 86 %, 87 %, 88 %, 89 %, 90 %, 91 %, 92 %, 93 %, 94 %, identité de 95 %, 96 %, 97 %, 98 %, ou 99 % sur toute sa longueur avec une séquence d'acide nucléique de SEQ ID N° : 1, ou son complément, et l'amorce d'acide nucléique antisens s'hybride plus particulièrement à la séquence d'acide nucléique qui est conservée dans le gène ORF1ab du SARS-CoV-2, ou son complément, dans lequel l'amorce d'acide nucléique antisens comprend une séquence d'acide nucléique CAATAGTCTGAACAACTGGTGT (SEQ ID N° : 5), ou son complément, ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 5, ou son complément ; et/ou l'amorce d'acide nucléique sens s'hybride plus particulièrement à la séquence d'acide nucléique qui est conservée dans le gène de nucléocapside du SARS-CoV-2, ou son complément, dans lequel l'amorce d'acide nucléique sens comprend une séquence d'acide nucléique de :
   TAGTTGATGACCCGTGTCCT (SEQ ID N° : 14) ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 14, ou son complément et l'amorce d'acide nucléique antisens s'hybride plus particulièrement à la séquence d'acide nucléique qui est conservée dans le gène de nucléocapside du SARS-CoV-2, ou son complément, dans lequel l'amorce d'acide nucléique antisens comprend une séquence d'acide nucléique CAACACGAACGTCATGATAC (SEQ ID N° : 16), ou son complément, ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 16, ou son complément.

2. Procédé selon la revendication 1, dans lequel les amorces d'amplification d'acide nucléique sens et antisens comprennent des séquences d'acide nucléique respectives de SEQ ID N° : 1 et de SEQ ID N° : 5, ou des séquences d'acide nucléique qui présentent au moins 85 %, 86 %, 87 %, 88 %, 89 %, 90 %, 91 %, 92 %, 93 %, 94 %, 95 %, 96 %, 97 %, 98 %, ou 99 % d'identité sur toute leur longueur avec de telles séquences, éventuellement dans lequel l'amorce d'amplification d'acide nucléique sens comprend une séquence d'acide nucléique de SEQ ID N° : 1, et l'amorce d'amplification d'acide nucléique antisens comprend une séquence d'acide nucléique de SEQ ID N° : 5.

3. Procédé selon la revendication 1, dans lequel l'amorce d'acide nucléique sens comprend une séquence d'acide nucléique de : TAGTTGATGACCCGTGTCCT (SEQ ID N° : 14), ou son complément, ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 14, ou son complément, éventuellement dans lequel l'amorce d'amplification d'acide nucléique sens comprend une séquence d'acide nucléique de SEQ ID N° : 14, et l'amorce d'amplification d'acide nucléique antisens comprend une séquence d'acide nucléique de SEQ ID N° : 16.

**4.** Procédé selon une quelconque revendication précédente, qui comprend en outre la transcription inverse de l'ARN SARS-CoV-2 de l'échantillon, et l'amplification d'un produit de la transcription inverse par une réaction d'amplification isotherme à l'aide des amorces d'amplification d'acide nucléique sens et antisens, éventuellement dans lequel l'amorce d'acide nucléique sens antisens comprend en outre une séquence de promoteur destinée à une ARN polymérase dépendante de l'ADN à son extrémité 5', et la transcription inverse est effectuée à l'aide de l'amorce d'acide nucléique antisens et/ou qui comprend en outre l'isolement de l'acide nucléique de l'échantillon avant la transcription inverse de l'ARN SARS-CoV-2 de l'échantillon présent dans l'acide nucléique isolé.

**5.** Procédé selon une quelconque revendication précédente, qui comprend en outre la capture d'un produit de la réaction d'amplification isotherme par hybridation de l'acide nucléique du produit à une sonde de capture d'acide nucléique, dans lequel la sonde de capture s'hybride plus particulièrement à la séquence d'acide nucléique qui est conservée dans l'acide nucléique du SARS-CoV-2, mais pas dans un autre acide nucléique du coronavirus humain, ou son complément, éventuellement dans lequel :

les amorces d'acide nucléique sens et antisens s'hybrident plus particulièrement à la séquence d'acide nucléique qui est conservée dans le gène ORF1ab du SARS-CoV-2, ou son complément, et la sonde de capture s'hybride plus particulièrement à la séquence d'acide nucléique qui est conservée dans le gène ORF1ab du SARS-CoV-2, ou son complément, éventuellement dans lequel la sonde de capture comprend un acide nucléique de séquence GAGGACAATCAGACAACTACTATTC (SEQ ID N° : 9), ou son complément, ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 9, ou son complément ; et/ou
les amorces d'acide nucléique sens et antisens s'hybrident plus particulièrement à la séquence d'acide nucléique qui est conservée dans le gène de nucléocapside du SARS-CoV-2, ou son complément, et la sonde de capture s'hybride plus particulièrement à la séquence d'acide nucléique qui est conservée dans le gène de nucléocapside du SARS-CoV-2, ou son complément, éventuellement dans lequel la sonde de capture comprend un acide nucléique de séquence : CTGGTTCTAAATCACCCATTCA (SEQ ID N° : 18), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 18, ou son complément.

**6.** Procédé selon une quelconque revendication précédente, qui comprend en outre la détection d'un produit de la réaction d'amplification isotherme par hybidation du produit à une sonde de détection d'acide nucléique, dans lequel la sonde de détection s'hybride plus particulièrement à la séquence d'acide nucléique du SARS-CoV-2 qui est conservée dans l'acide nucléique du SARS-CoV-2, mais pas dans un autre acide nucléique du coronavirus humain, ou son complément, éventuellement dans lequel :

les amorces d'acide nucléique sens et antisens s'hybrident plus particulièrement à la séquence d'acide nucléique qui est conservée dans le gène ORF1ab du SARS-CoV-2, ou son complément, et la sonde de détection s'hybride plus particulièrement à la séquence d'acide nucléique qui est conservée dans le gène ORF1ab du SARS-CoV-2, ou son complément, éventuellement dans lequel la sonde de détection comprend une séquence d'acide nucléique de GAGGTTCAACCTCAATTAGAGATGG (SEQ ID N° : 12), ou son complément, ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 12, ou son complément ; ou
les amorces d'acide nucléique sens et antisens s'hybrident plus particulièrement à la séquence d'acide nucléique qui est conservée dans le gène de nucléocapside du SARS-CoV-2, ou son complément, et la sonde de détection s'hybride plus particulièrement à la séquence d'acide nucléique qui est conservée dans le gène de nucléocapside du SARS-CoV-2, ou son complément, éventuellement dans lequel la sonde de détection comprend une séquence d'acide nucléique de GAACCTAAATTGGGTAGTCTTGTAG (SEQ ID N° : 19), ou son complément, ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 19, ou son complément ;
éventuellement, dans lequel la sonde de détection est marquée avec un marqueur visuellement détectable.

**7.** Procédé selon une quelconque revendication précédente, qui comprend l'amplification avec des amorces d'amplification d'acide nucléique sens et antisens, la capture du produit d'amplification avec une sonde de capture, et la détection du produit d'amplification avec une sonde de détection, dans lequel les amorces d'amplification et les sondes de capture et de détection comprennent des séquences d'acide nucléique respectives de SEQ ID N° : 1 (amorce sens), SEQ ID N° : 5 (amorce antisens), SEQ ID N° : 9 (sonde de capture) et SEQ ID N° : 12 (sonde de détection), ou des séquences d'acides nucléiques qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute leur longueur avec de telles séquences.

**8.** Procédé selon l'une quelconque des revendications 5 à 7, dans lequel la capture et/ou la détection du produit de la réaction d'amplification isotherme sont effectuées par dosage chromatographique sur bandelette réactive.

**9.** Procédé selon une quelconque revendication précédente, dans lequel l'échantillon est un échantillon biologique qui a été obtenu à partir d'un sujet suspecté d'être infecté par le SARS-CoV-2, éventuellement dans lequel l'échantillon est un échantillon nasopharyngé ou un échantillon de prélèvement de gorge obtenu à partir d'un sujet suspecté d'être infecté par le SARS-CoV-2, et/ou qui est un procédé *in vitro.*

**10.** Kit destiné à déterminer si un échantillon comporte un acide nucléique du SARS-CoV-2, qui comprend :

une amorce d'amplification d'acide nucléique sens et une amorce d'amplification d'acide nucléique antisens, destinées à amplifier un acide nucléique matrice par une réaction d'amplification isotherme, dans lequel chaque amorce d'amplification d'acide nucléique s'hybride plus particulièrement à une séquence d'acide nucléique qui est conservée dans l'acide nucléique du SARS-CoV-2, mais pas dans un autre acide nucléique du coronavirus humain, ou son complément ;
une sonde de capture d'acide nucléique, dans lequel la sonde de capture s'hybride plus particulièrement à une séquence d'acide nucléique qui est conservée dans l'acide nucléique du SARS-CoV-2, mais pas dans un autre acide nucléique du coronavirus humain, ou son complément ; et/ou
une sonde de détection d'acide nucléique, dans lequel la sonde de détection s'hybride plus particulièrement à une séquence d'acide nucléique qui est conservée dans l'acide nucléique du SARS-CoV-2, mais pas dans un autre acide nucléique du coronavirus humain, ou son complément, éventuellement dans lequel la sonde de détection comprend un marqueur détectable destiné à marquer un produit de l'amplification isotherme d'acide nucléique, dans lequel :

l'amorce d'acide nucléique sens s'hybride plus particulièrement à la séquence d'acide nucléique qui est conservée dans le gène ORF1ab du SARS-CoV-2, ou son complément, dans lequel les amorces d'amplification d'acide nucléique sens et antisens comprennent des séquences d'acide nucléique respectives de SEQ ID N° : 1 (sens) et SEQ ID N° : 5 (antisens), ou des séquences d'acide nucléique qui présentent au moins 85 %, 86 %, 87 %, 88 %, 89 %, 90 %, 91 %, 92 %, 93 %, 94 %, 95 %, 96 %, 97 %, 98 %, ou 99 % d'identité sur toute leur longueur avec de telles séquences, ou leur complément ; et/ou
l'amorce d'acide nucléique sens s'hybride plus particulièrement à la séquence d'acide nucléique qui est conservée dans le gène de nucléocapside du SARS-CoV-2, ou son complément, dans lequel l'amorce d'acide nucléique sens comprend une séquence d'acide nucléique de : TAGTTGATGACCCGTGTCCT (SEQ ID N° : 14) ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 14, ou son complément, et dans lequel l'amorce d'acide nucléique antisens s'hybride plus particulièrement à une séquence d'acide nucléique qui est conservé dans le gène de nucléocapside du SARS-CoV-2, ou son complément, dans lequel l'amorce d'acide nucléique antisens comprend une séquence d'acide nucléique CAACACGAACGTCATGATAC (SEQ ID N° : 16), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 16, ou son complément, éventuellement dans lequel l'amorce d'amplification d'acide nucléique sens comprend une séquence d'acide nucléique de SEQ ID N° : 14, et l'amorce d'amplification d'acide nucléique antisens comprend une séquence d'acide nucléique de SEQ ID N° : 16.

**11.** Kit selon la revendication 10, dans lequel :

les amorces d'acide nucléique sens et antisens s'hybrident plus particulièrement à la séquence d'acide nucléique qui est conservée dans le gène ORF1ab du SARS-CoV-2, ou son complément, et la sonde de capture s'hybride plus particulièrement à la séquence d'acide nucléique qui est conservée dans le gène ORF1ab du SARS-CoV-2, ou son complément, éventuellement dans lequel la sonde de capture comprend un acide nucléique de séquence : GAGGACAATCAGACAACTACTATTC (SEQ ID N° : 9), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 9, ou son complément ; et/ou

les amorces d'acide nucléique sens et antisens s'hybrident plus particulièrement à la séquence d'acide nucléique qui est conservée dans le gène de nucléocapside du SARS-CoV-2, ou son complément, et la sonde de capture s'hybride plus particulièrement à la séquence d'acide nucléique qui est conservée dans le gène de nucléocapside du SARS-CoV-2, ou son complément, éventuellement dans lequel la sonde de capture comprend un acide nucléique de séquence : CTGGTTCTAAATCACCCATTCA (SEQ ID N° : 18), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 18, ou son complément.

12. Kit selon la revendication 10 ou 11, dans lequel les amorces d'acide nucléique sens et antisens s'hybrident plus particulièrement à la séquence d'acide nucléique qui est conservée dans le gène ORF1ab du SARS-CoV-2, ou son complément, et la sonde de détection s'hybride plus particulièrement à la séquence d'acide nucléique qui est conservée dans le gène ORF1ab du SARS-CoV-2, ou son complément, éventuellement dans lequel la sonde de détection comprend une séquence d'acide nucléique de : GAGGTTCAACCTCAATTAGAGATGG (SEQ ID N° : 12), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 12, ou son complément.

13. Kit selon l'une quelconque des revendications 10 à 12, qui comprend des amorces d'amplification sens et antisens, et des sondes de capture et de détection comprenant des séquences d'acide nucléique respectives SEQ ID N° : 1 (sens), SEQ ID N° : 5 (antisens), SEQ ID N° : 9 (sonde de capture), et SEQ ID N° : 12 (sonde de détection), ou des séquences d'acide nucléique qui présentent au moins 85 %, 86 %, 87 %, 88 %, 89 %, 90 %, 91 %, 92 %, 93 %, 94 %, 95 %, 96 %, 97 %, 98 %, ou 99 % d'identité sur toute leur longueur avec de telles séquences.

14. Kit selon l'une quelconque des revendications 10 à 13, dans lequel les amorces d'acide nucléique sens et antisens s'hybrident plus particulièrement à la séquence d'acide nucléique qui est conservée dans le gène de nucléocapside du SARS-CoV-2, ou son complément, et la sonde de détection s'hybride plus particulièrement à la séquence d'acide nucléique qui est conservée dans le gène de nucléocapside du SARS-CoV-2, ou son complément, éventuellement dans lequel la sonde de détection comprend une séquence d'acide nucléique de : GAACCTAAATTGGGTAGTCT TGTAG (SEQ ID N° : 19), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 19, ou son complément.

15. Kit selon l'une quelconque des revendications 10 à 14, qui comprend en outre :

une ADN polymérase dépendante de l'ARN, une ADN polymérase dépendante de l'ADN, une ribonucléase spécifique de duplex ADN/ARN et une ARN polymérase dépendante de l'ADN ; et/ou
écouvillon destiné à obtenir un échantillon de prélèvement nasopharyngé ou de prélèvement de gorge provenant d'un sujet, dans lequel le kit comprenant éventuellement en outre un premier écouvillon destiné à obtenir un échantillon de prélèvement nasopharyngé provenant d'un sujet, et un second écouvillon destiné à obtenir un échantillon de prélèvement de gorge provenant d'un sujet ; et/ou
une bandelette de test chromatographique destinée à capturer et à détecter un produit de l'amplification isotherme d'acide nucléique ; et/ou
une solution tampon de lyse/liaison, une solution tampon d'élution et éventuellement une solution tampon de lavage, destinées à extraire un acide nucléique d'un échantillon biologique obtenu provenant du sujet.

16. Ensemble d'amorces destiné à amplifier l'acide nucléique du SARS-CoV-2 par une réaction d'amplification isotherme d'acide nucléique, qui comprend une amorce d'amplification d'acide nucléique sens et une amorce d'amplification d'acide nucléique antisens, dans lequel chaque amorce d'amplification d'acide nucléique s'hybride plus particulièrement à une séquence d'acide nucléique qui est conservée dans l'acide nucléique du SARS-CoV-2, mais pas dans un autre acide nucléique du coronavirus humain, ou son complément, dans lequel la séquence d'acide nucléique qui est conservée dans l'acide nucléique du SARS-CoV-2 est une séquence d'acide nucléique qui est conservée dans le gène ORF1ab ou le gène de nucléocapside du SARS-CoV-2, ou son complément, dans lequel :

l'amorce d'acide nucléique sens s'hybride plus particulièrement à la séquence d'acide nucléique qui est conservée dans le gène ORF1ab du SARS-CoV-2, ou son complément, dans lequel l'amorce d'acide nucléique sens comprend une séquence d'acide nucléique de : CTGTTGGTCAACAAGACGGCA (SEQ ID N° : 1), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 1, ou son complément, et l'amorce d'acide nucléique antisens s'hybride plus particulièrement à la séquence d'acide nucléique qui est conservée dans le gène ORF1ab du SARS-CoV-2, ou son complément, dans lequel l'amorce d'acide nucléique antisens comprend une séquence d'acide nucléique : CAATAGTCTGAACAACTG GTGT (SEQ ID N° : 5), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 5, ou son complément ; et/ou

l'amorce d'acide nucléique sens s'hybride plus particulièrement à la séquence d'acide nucléique qui est conservée dans le gène de nucléocapside du SARS-CoV-2, ou son complément, dans lequel l'amorce d'acide nucléique sens comprend une séquence d'acide nucléique de : TAGTTGATGACCCGTGTCCT (SEQ ID N° : 14), ou son complément, ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 14, ou son complément, et dans lequel l'amorce d'amplification d'acide nucléique antisens s'hybride plus particulièrement à une séquence d'acide nucléique qui est conservé dans le gène de nucléocapside du SARS-CoV-2, ou son complément, et dans lequel l'amorce d'amplification d'acide nucléique antisens comprend une séquence d'acide nucléique : CAACACGAACGTCATGATAC (SEQ ID N° : 16), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 16, ou son complément ;

éventuellement, dans lequel l'amorce d'acide nucléique sens et/ou antisens présente une longueur allant jusqu'à 50 nucléotides.

17. Ensemble d'oligonucléotides destiné à amplifier l'acide nucléique du SARS-CoV-2 par une réaction d'amplification d'acide nucléique isotherme, et destiné à capturer et/ou à détecter un produit de la réaction d'amplification, qui comprend :

un ensemble d'amorces selon la revendication 16 ;
une sonde de capture d'acide nucléique, dans lequel la sonde de capture s'hybride plus particulièrement à une séquence d'acide nucléique qui est conservée dans l'acide nucléique du SARS-CoV-2, mais pas dans un autre acide nucléique du coronavirus humain, ou son complément ; et/ou
une sonde de détection d'acide nucléique, dans lequel la sonde de détection s'hybride plus particulièrement à une séquence d'acide nucléique qui est conservée dans l'acide nucléique du SARS-CoV-2, mais pas dans un autre acide nucléique du coronavirus humain, ou son complément, éventuellement dans lequel la sonde de détection comprend un marqueur détectable destiné à marquer un produit de l'amplification d'acide nucléique isotherme.

18. Ensemble d'oligonucléotides selon la revendication 17, dans lequel :

les amorces d'acide nucléique sens et antisens s'hybrident plus particulièrement à la séquence d'acide nucléique qui est conservée dans le gène ORF1ab du SARS-CoV-2, ou son complément, et la sonde de capture s'hybride plus particulièrement à la séquence d'acide nucléique qui est conservée dans le gène ORF1ab du SARS-CoV-2, ou son complément, dans lequel la sonde de capture comprend un acide nucléique de séquence : GAGGAC AATCAGACAACTACTATTC (SEQ ID N° : 9), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 9, ou son complément ; et/ou la sonde de détection comprend une séquence d'acide nucléique de : GAGGTTCAACCTCAATTAGAGATGG (SEQ ID N° : 12), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 12, ou son complément ; et/ou
les amorces d'acide nucléique sens et antisens s'hybrident plus particulièrement à la séquence d'acide nucléique qui est conservée dans le gène de nucléocapside du SARS-CoV-2, ou son complément, et la sonde de capture s'hybride plus particulièrement à la séquence d'acide nucléique qui est conservée dans le gène de nucléocapside du SARS-CoV-2, ou son complément, dans lequel la sonde de capture comprend un acide nucléique de séquence : CTGGTTCTAAATCACCCATTCA (SEQ ID N° : 18), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 18, ou son complément ; et/ou la sonde de détection comprend une séquence d'acide nucléique de : GAACCTAAATTGGGTAGTCTTGTAG (SEQ ID N° : 19), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 19, ou son complément ;

éventuellement, dans lequel la sonde de capture et/ou de détection présente une longueur maximale de 50 nucléotides.

19. Kit selon l'une quelconque des revendications 10 à 15, qui comprend un ensemble d'amorces selon la revendication 16, ou un ensemble d'oligonucléotides selon la revendication 17 ou 18.

20. Utilisation d'un ensemble d'amorces selon la revendication 16, ou d'un ensemble d'oligonucléotides selon la revendication 17 ou 18, dans un procédé selon l'une quelconque des revendications 1 à 9.

21. Oligonucléotide destiné à être utilisé dans un procédé selon la revendication 1, qui comprend :

une séquence d'acide nucléique de : CTGTTGGTCAACAAGACGGCA (SEQ ID N° : 1), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 1, ou son complément ;

une séquence d'acide nucléique de : CAATAGTCTGAACAACTGGTGT (SEQ ID N° : 5), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 5, ou son complément ;

une séquence d'acide nucléique de : GAGGACAATCAGACAACTACTATTC (SEQ ID N° : 9), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 9, ou son complément ;

une séquence d'acide nucléique de : GAGGTTCAACCTCAATTAGAGATGG (SEQ ID N° : 12), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 12, ou son complément ;

une séquence d'acide nucléique de : TAGTTGATGACCCGTGTCCT (SEQ ID N° : 14), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 14, ou son complément ;

une séquence d'acide nucléique de : CAACACGAACGTCATGATAC (SEQ ID N° : 16), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 16, ou son complément ;

une séquence d'acide nucléique de : CTGGTTCTAAATCACCCATTCA (SEQ ID N° : 18), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 18, ou son complément ; ou

une séquence d'acide nucléique de : GAACCTAAATTGGGTAGTCTTGTAG (SEQ ID N° : 19), ou une séquence d'acide nucléique qui présente au moins 85 %, 86 %, 87 %, 88 %, 89 %, 90 %, 91 %, 92 %, 93 %, 94 %, 95 %, 96 %, 97 %, 98 %, ou 99 % d'identité sur toute sa longueur avec une séquence d'acide nucléique de SEQ ID N° : 19, ou son complément.

**Figure 1**

**Figure 2A**

CLUSTAL O(1.2.4) multiple sequence alignment

```
hCoV_229E_AF304460                          ------------ACATGCCAATT-------------------------------GCAGACCCTACA    23
SARS_CoV_GZ02_                              --------AAGAAGAAGAGGAAGACTGGCTGGATGATACTACTGAGCAATCAGAGATTGAG    53
SARS_CoV_ZS-B_                              --------AAGAAGAAGAGGAAGACTGGCTGGATGATACTACTGAGCAATCAGAGATTGAG    53
Bat_SARS-like_CoV_Rs4231_                   ----------AAGAAGAAGAGGACTGGCTTGGTGATGCTACTGAATTATCGGAGCATGAA    50
hCoV-HKU1_MH940245.1                        ACTGGTGACAATGACGATGAAGATGTTGTTACTGGTGACAATGACGATGAAGATGTTGTT    60
MERS_CoV_KJ477103.2                         ------------------AGGAGTGTTCTGAA-----------GTAGAGGCTTCAGATTTA    32
hCoV_OC43_KU131570                          ---------------------------ATGATGGTCCAAGTGTGGAGACATCTGATTCA    32
hCoV-NL63_AY567487                          ---------------------ACACTTGTTTT-------------------GGTGTTAGTAAA    23
2019-nCoV_MN988713_Atlanta_USA             --------AAGAGCAAGAAGAAGATTGGTTAGATGATGATAGTCAACAAACTGTTGGTCAA    53
2019-nCoV_MN988669.1_WuhanUniversity_China  --------AAGAGCAAGAAGAAGATTGGTTAGATGATGATAGTCAACAAACTGTTGGTCAA    53
2019-nCoV_MN908947.3_PublicHealth_China     --------AAGAGCAAGAAGAAGATTGGTTAGATGATGATAGTCAACAAACTGTTGGTCAA    53

2019-nCoV-ARP1.1F                           -----------------------------------------------------CTGTTGGTCAA    11
2019-nCoV-ARP1.2F                           ------------------------------------------GTCAACAAACTGTTGGTCAA    20
2019-nCoV-ARP1.3F                           ------------------------------------------GTCAACAAACTGTTGGTCAA    20
2019-nCoV-AR-CP1.1                          ------------------------------------------------------------     0
2019-nCoV-AR-CP1.2                          ------------------------------------------------------------     0
2019-nCoV-AR-DP1.2                          ------------------------------------------------------------     0
2019-nCoV-AR-DP1.1                          ------------------------------------------------------------     0
2019-nCoV-ARP1.1R                           ------------------------------------------------------------     0
2019-nCoV-ARP1.2R                           ------------------------------------------------------------     0
```

```
hCoV_229E_AF304460               CATTTTGACATTGAA------------------------------------GAAGTTGAA    47
SARS_CoV_GZ02_                   C----------------------------------------------------------    54
SARS_CoV_ZS-B_                   C----------------------------------------------------------    54
Bat_SARS-like_CoV_Rs4231_        C----------------------------------------------------------    51
hCoV-HKU1_MH940245.1             ACTGGTGACAATGACGATGAAGATGTTGTTACTGGTGACAATGACGATGAAGAGATTGTT   120
MERS_CoV_KJ477103.2              GAAGAA-----------GGTGAATCAG--------------------------AGTGCA    54
hCoV_OC43_KU131570               CAAGTTGAAGAAGAT-----------------G-------------TAGAAATGTCGGAT    62
hCoV-NL63_AY567487               CCTAATGCCATTGATGTTGAACATTTAGAGCT--------------TAAAGAAACTGTT    68
2019-nCoV_MN988713_Atlanta_USA   CAAGACGGCAGTGAGGACAATCAGACAACTACTA-----------TTCAAACAATTGTT   101
2019-nCoV_MN988669.1_WuhanUniversity_China  CAAGACGGCAGTGAGGACAATCAGACAACTACTA-----------TTCAAACAATTGTT   101
2019-nCoV_MN908947.3_PublicHealth_China  CAAGACGGCAGTGAGGACAATCAGACAACTACTA-----------TTCAAACAATTGTT   101

2019-nCoV-ARP1.1F                CAAGACGGCA-------------------------------------------------    21
2019-nCoV-ARP1.2F                ----------------------------------------------------------    20
2019-nCoV-ARP1.3F                CA--------------------------------------------------------    22
2019-nCoV-AR-CP1.1               -----------GGCAGTGAGGACAATCAGCAACTAC-----------------------    25
2019-nCoV-AR-CP1.2               ------------GAGGACAATCAGACAACTACTA-----------TTC-----------    25
2019-nCoV-AR-DP1.2               ----------------------------------------------------------     0
2019-nCoV-AR-DP1.1               ------------------------------------------CAAACAATTGTT        12
2019-nCoV-ARP1.1R                ----------------------------------------------------------     0
2019-nCoV-ARP1.2R                ----------------------------------------------------------     0
```

```
hCoV_229E_AF304460                           CTTTTAGATGCAGAG------TTTGTAGAACCAGGCTGTGGTGGTATTTTGGCAGT-AAT   100
SARS_CoV_GZ02_                               ---------------------CAGAACCAGAACCTACACCTGAAGA--------------ACCAGTTAAT    89
SARS_CoV_ZS-B_                               -----------------CAGAACCAGAACCTACACCTGAAGA---------ACCAGTTAAT    89
Bat_SARS-like_CoV_Rs4231_                    -----------------CAGAACCAGAACTAACACCTGAAGA---------ACCAGTTAAC    86
hCoV-HKU1_MH940245.1                         ACTGGTGACAATGATGACCAAATTGTTGTTACTGGTGATGATG---TAGATGATATTGAA   177
MERS_CoV_KJ477103.2                          TTTCTGAGACTTCAACT--------------GAACAAGTTGACG---TTTCTCATGAGACT    98
hCoV_OC43_KU131570                           TTTGTTGATCTTGAA-----------------TCTGTGATTCAGGATTATGAAAATGTT   104
hCoV-NL63_AY567487                           TTTGTTGAACCTAAG-----------------GATGGTGGTCAATTTTTTGTTTCTGGT   110
2019-nCoV_MN988713_Atlanta_USA               GAGGTTCAACCTCAATTAGAGATGGAACTTACACCAGTTGTTCAGACTATTGAAGTGAAT   161
2019-nCoV_MN988669.1_WuhanUniversity_China   GAGGTTCAACCTCAATTAGAGATGGAACTTACACCAGTTGTTCAGACTATTGAAGTGAAT   161
2019-nCoV_MN908947.3_PublicHealth_China      GAGGTTCAACCTCAATTAGAGATGGAACTTACACCAGTTGTTCAGACTATTGAAGTGAAT   161

2019-nCoV-ARP1.1F                            ------------------------------------------------------------    21
2019-nCoV-ARP1.2F                            ------------------------------------------------------------    20
2019-nCoV-ARP1.3F                            ------------------------------------------------------------    22
2019-nCoV-AR-CP1.1                           ------------------------------------------------------------    25
2019-nCoV-AR-CP1.2                           ------------------------------------------------------------    25
2019-nCoV-AR-DP1.2                           GAGGTTCAACCTCAATTAGAGATGG-----------------------------------    25
2019-nCoV-AR-DP1.1                           GAGGTTCAACCTC----------------------------------------------    25
2019-nCoV-ARP1.1R                            -------------------------ACACCAGTTGTTCAGACTATTG-------    22
2019-nCoV-ARP1.2R                            ---------------------AGAGATGGAACTTACACCAG-------------    20
```

EP 4 127 246 B1

```
hCoV_229E_AF304460                              AGATGAGCACGTCTTTTATAAGAAGG   126
SARS_CoV_GZ02_                                  CAGTTTACTGGTTATTTAAAACTTAC   115
SARS_CoV_ZS-B_                                  CAGTTTACTGGTTATTTAAAACTTAC   115
Bat_SARS-like_CoV_Rs4231_                       CAGTTTACTGGTTATTTAAAACTTAC   112
hCoV-HKU1_MH940245.1                            AGTGTCTATGATTTTG-----------  193
MERS_CoV_KJ477103.2                             TCT----------------------    101
hCoV_OC43_KU131570                              TGTTTTGA------------------   112
hCoV-NL63_AY567487                              GATTATCTTTGGTATGT---------   127
2019-nCoV_MN988713_Atlanta_USA                  AGTTTTAGTGGTTATTTAAAACTTAC   187
2019-nCoV_MN988669.1_WuhanUniversity_China      AGTTTTAGTGGTTATTTAAAACTTAC   187
2019-nCoV_MN908947.3_PublicHealth_China         AGTTTTAGTGGTTATTTAAAACTTAC   187

2019-nCoV-ARP1.1F                               --------------------------    21
2019-nCoV-ARP1.2F                               --------------------------    20
2019-nCoV-ARP1.3F                               --------------------------    22
2019-nCoV-AR-CP1.1                              --------------------------    25
2019-nCoV-AR-CP1.2                              --------------------------    25
2019-nCoV-AR-DP1.2                              --------------------------    25
2019-nCoV-AR-DP1.1                              --------------------------    25
2019-nCoV-ARP1.1R                               --------------------------    22
2019-nCoV-ARP1.2R                               --------------------------    20
```

**Figure 3A**

```
hCoV_OC43_KU131570                        AGAACCGGCAGTTTTTGGAGTTTCAACCCAGAAACAAACAACTTGATGTGTATAGATATG   60
hCoV-HKU1_MH940245.1                      AGAACTGGCAGTTGGTGGAGTTTTAACCCAGAGACTAATAATCTTATGTGTATTGATATG   60
hCoV-NL63_AY567487                        CGTGTTAAAACTTTTTGGGCTTTTAATCCTGAAACTAATGCAATCATCTCTCTCCAGGTT   60
hCoV_229E_AF304460                        CGTGCTCGAACTTTTTGGGCATGGAATCCTGAGGTTAATGCAATCACT---GTCACAAC   56
MERS_CoV_KJ477103.2                       AGAACTGGATCATGGTGGTCATTCAATCCTGAGACTAATTGCCTTTTGAACGTTCCATTT   60
Bat_SARS-like_CoV_Rs4231_                 ------------GTGTATTGCATGCATAAAGAATGCAGTA----------TACAAGAA   36
SARS_CoV_ZS-B_                            ------------------------------------------------------------   0
SARS_CoV_GZ02_                            ----------------GCAGTTGCATACGCACTGTAGTA------------CAGCGC   29
2019-nCoV_MN988713_Atlanta_USA            ----------------GCTGCATTTCACCAAGAATGTAGTT----------TACAGTCA   33
2019-nCoV_MN988669.1_WuhanUniversity_China ----------------GCTGCATTTCACCAAGAATGTAGTT----------TACAGTCA   33
2019-nCoV_MN908947.3_PublicHealth_China   ----------------GCTGCATTTCACCAAGAATGTAGTT----------TACAGTCA   33

SA-nCoV-F2.3                              ------------------------------------------------------------   0
SA-nCoV-R2.3                              ------------------------------------------------------------   0
SA-nCoV-R2.5                              ------------------------------------------------------------   0
SA-nCoV-R2.4                              ------------------------------------------------------------   0
SA-nCoV-DP2.4                             ------------------------------------------------------------   0
SA-nCoV-DP2.3                             ------------------------------------------------------------   0
SA-nCoV-CP2.3                             ------------------------------------------------------------   0
```

EP 4 127 246 B1

**Figure 3**B (continued)

```
hCoV_OC43_KU131570            AAAGGAACAATGTTT-GTTAGGCCAATAATTGAGGACTACCATACC----CTGACGGTCA    115
hCoV-HKU1_MH940245.1          AAAGGTAAGATGTAT-GTTAGGCCAGTTATTGAGGACTATCATACA----TTAACGGCTA    115
hCoV-NL63_AY567487            TACGGACATAATTAT----TACTTACCGGTGATGGCTGCACCTACAGGT-GTTACATTAA    115
hCoV_229E_AF304460            CGTGTTGGGACAGACATACTATCAACCCATTCAACAAGCTCCAACAGG-------CATTA    109
MERS_CoV_KJ477103.2           GGTGGTACAACTGTC-GTACGTCCACTCGTAGAGGACTCT---------ACCAGTGTAA    109
Bat_SARS-like_CoV_Rs4231_     TGTTGTGAAAATCAA-CCATTCCAACCTGAAGACCCATGTCCAATACATTATTATTCGGA   95
SARS_CoV_ZS-B_                ------------------------------------------------------GCAGTTGC    8
SARS_CoV_GZ02_                TGTGCATCTAATAAA-CCTCATGTGCTTGAAGATCCTTGTCCTACTGGTTACCAACCTGA   88
2019-nCoV_MN988713_Atlanta_USA        TGTACTCAACATCAA-CCATATGTAGTTGATGACCCGTGTCCTATTCACTTCTATTCTAA   92
2019-nCoV_MN988669.1_WuhanUniversity_China  TGTACTCAACATCAA-CCATATGTAGTTGATGACCCGTGTCCTATTCACTTCTATTCTAA   92
2019-nCoV_MN908947.3_PublicHealth_China     TGTACTCAACATCAA-CCATATGTAGTTGATGACCCGTGTCCTATTCACTTCTATTCTAA   92

SA-nCoV-F2.3                  -----------------------TAGTTGATGACCCGTGTCCT----------------   20
SA-nCoV-R2.3                  ------------------------------------------------------------    0
SA-nCoV-R2.5                  ------------------------------------------------------------    0
SA-nCoV-R2.4                  ------------------------------------------------------------    0
SA-nCoV-DP2.4                 ------------------------------------------------------------    0
SA-nCoV-DP2.3                 ------------------------------------------------------------    0
SA-nCoV-CP2.3                 ------------------------------------------------------------    0
```

```
hCoV_OC43_KU131570             CAATAATACGCGG-------TCATCTTTACATTCAAGGTATAAAACTAGGTAC---TGGC     165
hCoV-HKU1_MH940245.1           CTGTTATCCGTGG-------TCATCTTTATATACAGGGTGTTAAACTTGGCAC---TGGT     165
hCoV-NL63_AY567487             CACTTCTTAGTGG-------TGTACTTCTTGTTGATGGCCATAAGATTGCTACTCGTGTT     168
hCoV_229E_AF304460             CTGTG-ACCTTGCTGAGCGGCGTGCTTTACGTTGACGGACATAGATTGGCTTCAGGTGTT     168
MERS_CoV_KJ477103.2            CTGCTGTTGTAAC-------CAATGGCCACCTCAAAATGGCTGGCATGCATTTCGGTGCT     162
Bat_SARS-like_CoV_Rs4231_      CTGGTTTGTAAAAATTGGA---CCTCGCAAGTCTGCTCGCCTAGTACAACTTTGTGCTGG     152
SARS_CoV_ZS-B_                 ATAC--GCACTGTAGTACAGCGCTGTGCATCTAATATAGCACTGCTTGGCTTTGTGCTCT      66
SARS_CoV_GZ02_                 ATGGAATATAAGGTACAACACTAGGGGTAATACTTATAGCACTGCTTGGCTTTGTGCTCT     148
2019-nCoV_MN988713_Atlanta_USA ATGGTATATTAGAGTAGGAGCTAGAAAATCAGCACCTTTAATTGAAT---TGTGCSTGGA     149
2019-nCoV_MN988669.1_WuhanUniversity_China ATGGTATATTAGAGTAGGAGCTAGAAAATCAGCACCTTTAATTGAAT---TGTGCSTGGA     149
2019-nCoV_MN908947.3_PublicHealth_China    ATGGTATATTAGAGTAGGAGCTAGAAAATCAGCACCTTTAATTGAAT---TGTGCSTGGA     149

SA-nCoV-F2.3                   ------------------------------------------------------------      20
SA-nCoV-R2.3                   ------------------------------------------------------------       0
SA-nCoV-R2.5                   ------------------------------------------------------------       0
SA-nCoV-R2.4                   ------------------------------------------------------------       0
SA-nCoV-DP2.4                  ------------------------------------------------------------       0
SA-nCoV-DP2.3                  ------------------------------------------------------------       0
SA-nCoV-CP2.3                  ------------------------------------------------------------       0
```

EP 4 127 246 B1

**Figure 3D (continued)**

```
hCoV_OC43_KU131570                          TATTCTTTGGCAGAGATTTGCCAGCTTATATGACTGTTGCTAAGGTCACATACCTGTGCACA    225
hCoV-HKU1_MH940245.1                        TACACGCTTGCCGATTTGCCTGTTTAGTTGCTAGCTAAGGTGCAAGTCCTCTGTACT         225
hCoV-NL63_AY567487                          CA---AGTGGGTCAGTTGCCTAAATATGTAATAGTTGCTACGCCTAGTACCACAATTGTT      225
hCoV_229E_AF304460                          CAGGTT--CATAACCTACCTGAATACATGACAGTTGCCGTGCCGAGCACTACTATAATT       225
MERS_CoV_KJ477103.2                         TGTGACTACGACAGACTTCCTAATGAAGTCACCGTGGCCAAACCCAATGTGCTGATTGCT      222
Bat_SARS-like_CoV_Rs4231_                   TGAATATGGACACA--------------------GAGTTCCAATACAT                  180
SARS_CoV_ZS-B_                              AGGAAA------------------------GGTTTTACCT                          82
SARS_CoV_GZ02_                              AGGAAA------------------------GGTTTTACCT                          164
2019-nCoV_MN988713_Atlanta_USA              TGAGGCTGGTTCTA--------------------AATCACCCATTCAG                  177
2019-nCoV_MN988669.1_WuhanUniversity_China  TGAGGCTGGTTCTA--------------------AATCACCCATTCAG                  177
2019-nCoV_MN908947.3_PublicHealth_China     TGAGGCTGGTTCTA--------------------AATCACCCATTCAG                  177

SA-nCoV-F2.3                                --------------------------------------------                      20
SA-nCoV-R2.3                                --------------------------------------------                      0
SA-nCoV-R2.5                                --------------------------------------------                      0
SA-nCoV-R2.4                                --------------------------------------------                      0
SA-nCoV-DP2.4                               --------------------------------------------                      0
SA-nCoV-DP2.3                               --------------------------------------------                      0
SA-nCoV-CP2.3                               ----CTGGTTCTA--------------------AATCACCCATTCA-                    22
```

**Figure 3E (continued)**

```
hCoV_OC43_KU131570                              TATAAGCGTGGTTTTC---TTGACAAGATAAGCGATACTAGTGGTTTTGCTGTTTATGTT    282
hCoV-HKU1_MH940245.1                            TATAAACGTGCCTTTT---TAGATAAGTTAGATGTTAATAGTGGTTTTGCTGTTTTTGTT    282
hCoV-NL63_AY567487                              TGTGACCGTGTTGGTCGCTCTGTTAATGAAACAAGCCAGACTGGTTGGGCATTCTACGTC    285
hCoV_229E_AF304460                              TATAGTAGAGTCGGAAGGTCCGTAAATTCACAAAATAGCACAGGCTGGGTTTTCTACGTA    285
MERS_CoV_KJ477103.2                             TTAAAAATGGTGAAGCGGCAAAGC--TACGGA-ACTAATTCCGGCGTTGCCATTTACCAT    279
Bat_SARS-like_CoV_Rs4231_                       TATGAAATGT-TTGGCAATTATAC--TATTTCATGT---GAACCACTTGAAATAAATTGT    234
SARS_CoV_ZS-B_                                  TTTCATAGAT-GGCACACTATGGT--TCAAACATGCACACCTAATGTTACTATCAACTGT    139
SARS_CoV_GZ02_                                  TTTCATAGAT-GGCACACTATGGT--TCAAACATGCACACCTAATGTTACTATCAACTGT    221
2019-nCoV_MN988713_Atlanta_USA                  TACA---TCG-ATATCGGTAATTA--TACAGTTTCCTGTTYACCTTTTACAATTAATTGC    231
2019-nCoV_MN988669.1_WuhanUniversity_China      TACA---TCG-ATATCGGTAATTA--TACAGTTTCCTGTTYACCTTTTACAATTAATTGC    231
2019-nCoV_MN908947.3_PublicHealth_China         TACA---TCG-ATATCGGTAATTA--TACAGTTTCCTGTTYACCTTTTACAATTAATTGC    231

SA-nCoV-F2.3                                     ------------------------------------------------------------     20
SA-nCoV-R2.3                                     ------------------------------------------------------------      0
SA-nCoV-R2.5                                     ------------------------------------------------------------      0
SA-nCoV-R2.4                                     ------------------------------------------------------------      0
SA-nCoV-DP2.4                                    ------------------------------------------------------------      0
SA-nCoV-DP2.3                                    ------------------------------------------------------------      0
SA-nCoV-CP2.3                                    ------------------------------------------------------------     22
```

**Figure 3**F (continued)

```
hCoV_OC43_KU131570            AAGTCCAAAGTCGGTAATTACCGACTGCCATCAACCCAAAAGGGTTC------AGGCATG    336
hCoV-HKU1_MH940245.1          AAGTCTAAAGTTGGTAACTATCGTTTACCTTCTAGTAAA---TCTAG------TGGTATG    333
hCoV-NL63_AY567487            CGTGCTAAACATGGTGATTTTTCTGGTGTTGCCTCTCAGGAGGGTGT------TTTGTCA    339
hCoV_229E_AF304460            CGAGTAAAACACGGTGATTTTTCTGCAGTGAGCT----CTCCCATGAGCAACATGACAGA    341
MERS_CoV_KJ477103.2           AGATATAAGGCAGGTAATTACAGGAGTCCGCCTATTACGGCGGATATTGAACTTGCATTG    339
Bat_SARS-like_CoV_Rs4231_     CAAAACCCACCAGTTGGAAGTCTCATTGTACGTTGTTCATATGATGTTGACTTTATGGAG    294
SARS_CoV_ZS-B_                CAAGATCCAGCTGGTGGTGCGCTTATAGCTAGGTGTTGGTACCTTCATGAAGGTCACCAA    199
SARS_CoV_GZ02_                CAAGATCCAGCTGGTGGTGCGCTTATAGCTAGGTGTTGGTACCTTCATGAAGGTCACCAA    281
2019-nCoV_MN988713_Atlanta_USA        CAGGAACCTAAATTGGGTAGTCTTGTAGTGCGTTGTTCGTTCTATGAAGACTTTTAGAG    291
2019-nCoV_MN988669.1_WuhanUniversity_China   CAGGAACCTAAATTGGGTAGTCTTGTAGTGCGTTGTTCGTTCTATGAAGACTTTTAGAG    291
2019-nCoV_MN908947.3_PublicHealth_China      CAGGAACCTAAATTGGGTAGTCTTGTAGTGCGTTGTTCGTTCTATGAAGACTTTTAGAG    291

SA-nCoV-F2.3                  ------------------------------------------------------------    20
SA-nCoV-R2.3                  ------------------------------------------------------------    0
SA-nCoV-R2.5                  -----------------------GTGCGTTGTTCGTTCTATG--------------    19
SA-nCoV-R2.4                  ---------------------------------------------------------G    1
SA-nCoV-DP2.4                 --GGAACCTAAATTGGGTAGTCTTG-----------------------------------    23
SA-nCoV-DP2.3                 ---GAACCTAAATTGGGTAGTCTTGTAG--------------------------------    25
SA-nCoV-CP2.3                 ------------------------------------------------------------    22
```

EP 4 127 246 B1

```
hCoV_OC43_KU131570              GACA---CCGCATTGTTGAGAAATAATATCTAAATTTTAA----------------GGAT     377
hCoV-HKU1_MH940245.1            GATA---CTGCCTTGTTGAGAGCTTAAATCTAAACTATTA--------------GGAT       374
hCoV-NL63_AY567487              GAAA---GAGAGAAGTTGCTTCATTTAATCTAAACTAAACAAAATGGCTA---GTGTAAA     393
hCoV_229E_AF304460              AAAC---GAAAGATTGCT--TCATTTTTTCTAAACTGAACGAAAAGATGGCTACAGTCAA     396
MERS_CoV_KJ477103.2             CTT----CGAGCTTAGGC---------TCTTTAGTAAG----AGTATCT------TAAT       375
Bat_SARS-like_CoV_Rs4231_       TATCA--CGACGTTCGTGTTGTTCTAGATTTCATCTAAACGAACAAACTA------AAAT   346
SARS_CoV_ZS-B_                  ACTGCTGCATTTAGAGACGTACTTGTTGTTTTAAATAAACGAACAAATTA------AAAT   253
SARS_CoV_GZ02_                  ACTGCTGCATTTAGAGACGTATTTGTTGTTTTAAATAAACGAACAAATTA------AAAT   335
2019-nCoV_MN988713_Atlanta_USA  TATCA--TGACGTTCGTGTTGTTTTAGATTTCATCTAAACGAACAAACTA------AAAT   343
2019-nCoV_MN988669.1_WuhanUniversity_China  TATCA--TGACGTTCGTGTTGTTTTAGATTTCATCTAAACGAACAAACTA------AAAT   343
2019-nCoV_MN908947.3_PublicHealth_China     TATCA--TGACGTTCGTGTTGTTTTAGATTTCATCTAAACGAACAAACTA------AAAT   343

SA-nCoV-F2.3                    ------------------------------------------------------------    20
SA-nCoV-R2.3                    ----------------------------------------------------------AT     2
SA-nCoV-R2.5                    ---------------------------------------------------------        19
SA-nCoV-R2.4                    TATCA--TGACGTTCGTGTTG---------------------------------------    20
SA-nCoV-DP2.4                   ------------------------------------------------------------    23
SA-nCoV-DP2.3                   ------------------------------------------------------------    25
SA-nCoV-CP2.3                   ---------------------------------------------------------        22
```

EP 4 127 246 B1

```
hCoV_OC43_KU131570              GTCTTTTACTCCTGGTAAGCAATCCAGTAGTAGAGCGTCCTCTGGAAATCGTTCTGGTAA    437
hCoV-HKU1_MH940245.1            GTCTTATACTCCCGGTCATCATGCTGGAAGTAGAAGCTCCTCTGGAAATCGTTCAGGAAT    434
hCoV-NL63_AY567487              TTGGGCCGATGA--CAGAGCTGCTAGGAAGAAATTTC--CTC---------------CT    433
hCoV_229E_AF304460              ATGGGCTGATGCATCTGAACCACAACGTGGTCGTCAG--GGTA-------------GA    439
MERS_CoV_KJ477103.2             TGATTTTAACGAATCTCAATTTCATTGTTATGGCATC--CCCTGCTGCAC--CTCGTGCT    431
Bat_SARS-like_CoV_Rs4231_       GTCTGATAATGGACCCCAACCAAATCAGCGTAGTGCC--CCCCGCATTACATTTGGTGGA    404
SARS_CoV_ZS-B_                  GTCTGATAATGGACCCCAATCAAACCAACGTAGTGCC--CCCCGCATTACATTTGGTGGA    311
SARS_CoV_GZ02_                  GTCTGATAATGGACCCCAATCAAACCAACGTAGTGCC--CCCCGCATTACATTTGGTGGA    393
2019-nCoV_MN988713_Atlanta_USA  GTCTGATAATGGACCCCAAAAT---CAGCGAAATGCA--CCCCGCATTACGTTTGGTGGA    398
2019-nCoV_MN988669.1_WuhanUniversity_China  GTCTGATAATGGACCCCAAAAT---CAGCGAAATGCA--CCCCGCATTACGTTTGGTGGA    398
2019-nCoV_MN908947.3_PublicHealth_China  GTCTGATAATGGACCCCAAAAT---CAGCGAAATGCA--CCCCGCATTACGTTTGGTGGA    398

SA-nCoV-F2.3                    ------------------------------------------------------------    20
SA-nCoV-R2.3                    GTCTGATAATGGACCCCA------------------------------------------    20
SA-nCoV-R2.5                    ------------------------------------------------------------    19
SA-nCoV-R2.4                    ------------------------------------------------------------    20
SA-nCoV-DP2.4                   ------------------------------------------------------------    23
SA-nCoV-DP2.3                   ------------------------------------------------------------    25
SA-nCoV-CP2.3                   ------------------------------------------------------------    22
```

EP 4 127 246 B1

```
hCoV_OC43_KU131570                              TGGCATCCTTAAGTG      452
hCoV-HKU1_MH940245.1                            CCTCAAGAA------      443
hCoV-NL63_AY567487                              CCTTC----------      438
hCoV_229E_AF304460                              ATACCTTATTC----      450
MERS_CoV_KJ477103.2                             GTTTC----------      436
Bat_SARS-like_CoV_Rs4231_                       CCCACAGATTC----      415
SARS_CoV_ZS-B_                                  CCCACAGATTC----      322
SARS_CoV_GZ02_                                  CCCACAGATTC----      404
2019-nCoV_MN988713_Atlanta_USA                  CCCTCAGATTC----      409
2019-nCoV_MN988669.1_WuhanUniversity_China      CCCTCAGATTC----      409
2019-nCoV_MN908947.3_PublicHealth_China         CCCTCAGATTC----      409

SA-nCoV-F2.3                                    --------------       20
SA-nCoV-R2.3                                    --------------       20
SA-nCoV-R2.5                                    --------------       19
SA-nCoV-R2.4                                    --------------       20
SA-nCoV-DP2.4                                   --------------       23
SA-nCoV-DP2.3                                   --------------       25
SA-nCoV-CP2.3                                   --------------       22
```

# Figure 4

EP 4 127 246 B1

# Figure 5

SENSITIVITY

SPECIFICITY

Internal Control
Covid-2019 ORF1ab
Covid-2019 NP

Internal Control

1000 copies/test    100 copies/test    10 copies/test    0 copies/test

SARS    MERS

All >100,000 copies/test

## REFERENCES CITED IN THE DESCRIPTION

### Patent documents cited in the description

- WO 2008090340 A
- WO 2010015835 A
- WO 0248164 A
- WO 2014140640 A

### Non-patent literature cited in the description

- **LAMB et al.** *The Lancet*, 2020, 1-19
- **MEINKOTH** ; **WAHL**. *Anal. Biochem.*, 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **CHAN** ; **FOX**. *Rev. Med. Microbiol.*, 1999, vol. 10, 185-196
- **GUATELLI et al.** *Proc. Natl. Acad. Sci.*, 1990, vol. 87, 1874-1878
- **COMPTON**. *Nature*, 1991, vol. 350, 91-92
- **LEE et al.** *Journal of Infectious Diseases*, 2010, vol. 201 (S1), S65-S71
- **BOOM et al.** *Journal of Clinical Microbiology*, 1990, vol. 28 (3), 495-503
- **HOURFAR et al.** *Clinical Chemistry*, 2005, vol. 51 (7), 1217-1222
- **CACACE et al.** *Q Rev Biophys*, 1997, vol. 30, 241-77
- **CAMPANELLA et al.** *BMC Bioinformatics*, 2003, vol. 4, 29
- **NEEDLEMAN** ; **WUNSCH**. *J. Mol. Biol.*, 1970, vol. 48, 443-453
- **ALTSCHUL et al.** *J. Mol. Biol.*, 1990, vol. 215, 403-410, http://www.ebi.ac.uk/fasta
- **LARKIN et al.** *Bioinformatics*, 2007, vol. 23, 2947-2948, http://www.ebi.ac.uk/tools/clustalw2
- **KRUSKAL**. Time warps, string edits and macromolecules: the theory and practice of sequence comparison. Addison Wesley, 1983, 1-44
- **CORMAN et al.** Detection of 2019 novel coronavirus (2019-nCoV) by real-time RT-PCR. *Euro Surveill.*, 2020, vol. 25 (3)